(19) Europäisches Patentamt
European Patent Office
Office européen des brevets

(11) EP 4 421 077 A1

(12) EUROPEAN PATENT APPLICATION
published in accordance with Art. 153(4) EPC

(43) Date of publication:
**28.08.2024 Bulletin 2024/35**

(21) Application number: **22884103.7**

(22) Date of filing: **21.10.2022**

(51) International Patent Classification (IPC):
***C07D 487/04*** (2006.01) ***A61K 31/5025*** (2006.01)
***A61K 31/4439*** (2006.01) ***A61K 31/422*** (2006.01)
***C07D 413/14*** (2006.01) ***C07D 513/04*** (2006.01)
***C07D 413/12*** (2006.01) ***C07D 417/12*** (2006.01)
***C07D 417/14*** (2006.01) ***C07D 471/04*** (2006.01)

(52) Cooperative Patent Classification (CPC):
**A61K 31/422; A61K 31/4439; A61K 31/5025; C07D 413/12; C07D 413/14; C07D 417/12; C07D 417/14; C07D 471/04; C07D 487/04; C07D 513/04**

(86) International application number:
**PCT/KR2022/016188**

(87) International publication number:
**WO 2023/068881 (27.04.2023 Gazette 2023/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **22.10.2021 KR 20210141877**
**04.02.2022 KR 20220014856**

(71) Applicant: **Voronoi Inc.**
**Incheon 21984 (KR)**

(72) Inventors:
- **JO, Seohyun**
  **Incheon 21984 (KR)**
- **MA, Dahoon**
  **Incheon 21984 (KR)**
- **PARK, Yeseul**
  **Incheon 21984 (KR)**
- **SIM, Gyuseok**
  **Incheon 21984 (KR)**
- **SON, Jungbeom**
  **Incheon 21984 (KR)**
- **KIM, Namdoo**
  **Incheon 21984 (KR)**
- **KIM, Sunghwan**
  **Incheon 21984 (KR)**

(74) Representative: **Plasseraud IP**
**104 Rue de Richelieu**
**CS92104**
**75080 Paris Cedex 02 (FR)**

(54) **ARYL OR HETEROARYL DERIVATIVE, AND PHARMACEUTICAL COMPOSITION COMPRISING SAME AS ACTIVE INGREDIENT FOR PREVENTION OR TREATMENT OF KINASE-RELATED DISEASES**

(57) The present invention relates to an aryl or heteroaryl derivative and a pharmaceutical composition for the treatment of kinase-related diseases comprising same as an active ingredient. An aryl or heteroaryl derivative provided according to an aspect of the present invention exhibits excellent inhibitory activity against kinases, especially receptor-interacting serine/threonine-protein kinase 1 (RIPK1), and thus can be used as a therapeutic agent for RIPK1-related diseases.

EP 4 421 077 A1

## Description

[Technical Field]

The present invention relates to an aryl or heteroaryl derivative, and a pharmaceutical composition for preventing or treating a kinase-related disease, which includes the aryl or heteroaryl derivative as an active ingredient.

[Background Art]

Protein kinases are enzymes that catalyze a phosphorylation reaction that transfers a $\gamma$-phosphate group of ATP to the hydroxy groups of tyrosine, serine, and threonine in proteins, and are responsible for cell metabolism, gene expression, cell growth, differentiation, and cell division and play an important role in cell signaling (Non-Patent Document 1, Thomas A. Hamilton, in Encyclopedia of Immunology (Second Edition), 1998, 2028-2033). Protein kinases are classified into tyrosine protein kinases and serine/threonine kinases and more than 90 types are tyrosine kinases.

Protein kinases are molecular switches that must actively regulate the transition between active and inactive states in cells. When the transition between the active and inactive states is abnormally regulated, intracellular signaling is excessively activated, leading to uncontrolled cell division and proliferation. In addition, the abnormal activation of a protein kinase due to genetic mutation, amplification, and overexpression is associated with the development and progression of various tumors and plays a critical role in the development of various diseases such as inflammatory diseases, degenerative brain diseases, autoimmune diseases, and cancer. Examples of the related kinases include ABL1, ABL2, BRAF, CDK11, CDK8, CDKL2, CIT, CSF1R, DDR1, DDR2, FLT3, KIT, LOK, LTK, MUSK, PAK3, PDGFRA, PDGFRB, RAF1, and RIPK1. Receptor-interacting serine/threonine-protein kinase 1 (RIPK1), which is one example of a protein kinase, is a multifunctional signal transducer that is involved in mediating NF-$\kappa$B activation, apoptosis, and/or necroptosis.

Particularly, RIPK1 activity is known to be critically involved in mediating necroptosis, which is a caspase-independent pathway of necrotic cell death (Non-Patent Document 2, Holler et al., Nat Immunol 2000; 1: 489-495; Non-Patent Document 3, Degterev et al., Nat Chem Biol 2008; 4: 313-321).

Accordingly, if RIPK1 activity can be effectively inhibited, cell protection is possible under conditions for inducing cell death caused by tumor necrosis factor-alpha (TNF-$\alpha$), thereby blocking necroptosis.

It has been reported that RIPK1-mediated necroptosis is associated with various diseases such as inflammatory diseases, degenerative brain diseases, autoimmune diseases, and cancer.

First, since it has been confirmed that RIP3 knockout mice designed to completely block RIPK1-mediated programmed necrosis have protective effects in inflammatory bowel diseases (including ulcerative colitis and Crohn's disease) (Non-Patent Document 4, (2011) Nature 477, 330-334), psoriasis (Non-Patent Document 5, (2011) Immunity 35, 572-582), retinal detachment-induced photoreceptor necrosis (Non-Patent Document 6, (2010) PNAS 107, 21695-21700), retinitis pigmentosa (Non-Patent Document 7, (2012) Proc. Natl. Acad. Sci., 109:36, 14598-14603), cerulein-induced acute pancreatitis (Non-Patent Document 8, (2009) Cell 137, 1100-1111), and sepsis/systemic inflammatory response syndrome (SIRS) (Non-Patent Document 9, (2011) Immunity 35, 908-918), if a specific compound can block RIPK1-mediated necroptosis, it is possible to develop the corresponding compound as a treatment for an inflammatory disease.

In addition, since it is known that RIPK1 mediates microglial responses in Alzheimer's disease (Non-Patent Document 10, PNAS October 10, 2017. 114 (41) E8788-E8797), if a specific compound can target RIPK1 to effectively inhibit its activity, it is possible to develop the corresponding compound as a treatment for degenerative brain diseases (i.e., degenerative neurological diseases) such as Alzheimer's disease, Down syndrome, Parkinson's disease, Lou Gehrig's disease, dementia, Huntington's disease, multiple sclerosis, amyotrophic lateral sclerosis, palsy, stroke, and mild cognitive impairment.

Further, it is known that RIPK1 regulates the production of tumor necrosis factor-alpha (TNF-$\alpha$), and TNF-$\alpha$ is a pro-inflammatory cytokine that is involved in mediating cell death and inflammation in various diseases, including rheumatoid arthritis and cancer (Non-Patent Document 11, Cell Death and Disease (2012) 3, e320). Therefore, if a specific compound can target RIPK1 to effectively inhibit activity, it is possible to develop the corresponding compound as a treatment for rheumatoid arthritis, polymyalgia rheumatica, ankylosing spondylitis, an autoimmune disease such as motor neurone disease, and cancer.

In addition, it has been disclosed that RIPK1 induces macrophage-mediated adaptive immune tolerance in pancreatic cancer (Non-Patent Document 12, Cancer Cell 34, 757-774, November 12, 2018). More specifically, it has been shown that RIPK1 inhibition in TAMs results in cytotoxic T cell activation and T helper cell differentiation toward a mixed Th1/Th17 phenotype, resulting in tumor immunity in mice and organotypic models of human PDA.

Therefore, since targeting RIPK1 acts synergistically with PD-1 and inducible co-stimulator-based immunotherapies, it can be seen that RIPK1 is a checkpoint kinase that governs tumor immunity. That is, since it is possible to develop a compound that can effectively inhibit the activity of a protein kinase, including RIPK1, as a treatment for various

diseases such as inflammatory diseases, degenerative brain diseases, autoimmune diseases, and cancer, there is a need for the development of a protein kinase inhibitor with a novel structure.

[Disclosure]

[Technical Problem]

The present invention is directed to providing an aryl or heteroaryl derivative with a novel structure, an isomer thereof, a solvate thereof, a hydrate thereof, or a pharmaceutically acceptable salt thereof, which exhibits excellent inhibitory activity against various kinases and thus has a therapeutic effect on kinase-related diseases.

[Technical Solution]

To achieve the above purpose, one aspect of the present invention provides a compound represented by Chemical Formula 1, an isomer thereof, a solvate thereof, a hydrate thereof, or a pharmaceutically acceptable salt thereof:

[Chemical Formula 1]

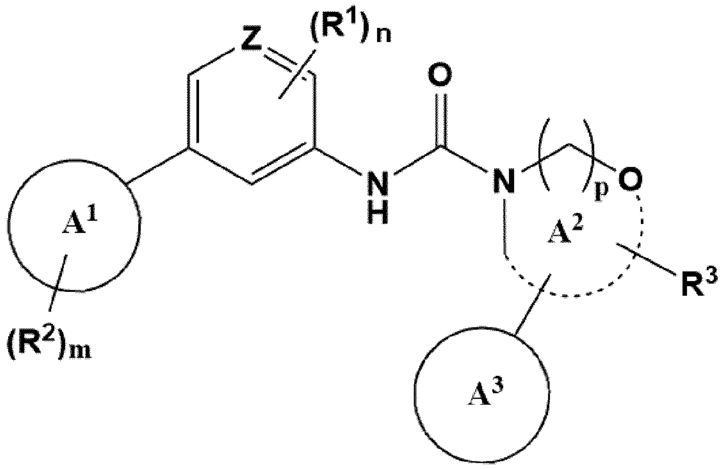

In Chemical Formula 1,

Z is N or CH,
ring $A^1$ is 5- to 12-membered heteroaryl,
ring $A^2$ is 5- to 10-membered heterocycloalkyl, 5- to 10-membered heterocycloalkyl-aryl bicyclic ring, or 5- to 10-membered heterocycloalkyl-heteroaryl bicyclic ring, wherein p is an integer from 0 to 2,
ring $A^3$ is 4- to 12-membered aryl or 4- to 12-membered heteroaryl, and is unsubstituted or substituted with one or more substituents $R^4$ selected from halogen, cyano, $C_{1-8}$ alkoxy, $C_{1-8}$ alkyl, $C_{1-8}$ haloalkyl, and $C_{1-8}$ alkylamino,
$R^1$ is hydrogen, halogen, $C_{1-8}$ alkyl, $C_{1-8}$ alkoxy, cyano, $C_{3-8}$ cycloalkyl, or $C_{3-8}$ heterocycloalkyl, and n is an integer from 1 to 3,
$R^2$ is halogen, $C_{1-8}$ alkyl, $C_{1-8}$ alkoxy, amine, cyano, $C_{1-8}$ haloalkyl, or $C_{2-8}$ alkynyl that is unsubstituted or substituted with a hydroxyl group,

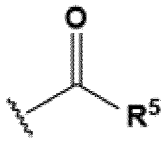,
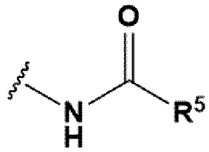,

or

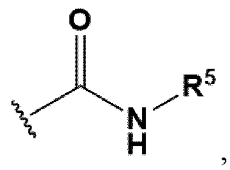

and m is an integer from 0 to 4,
$R^5$ is $C_{1-8}$ alkyl, $-C_{1-8}$ alkyl-$C_{1-8}$ alkoxy, $-C_{1-8}$ alkyl-hydroxy, or $C_{3-8}$ cycloalkyl, where the $C_{3-8}$ cycloalkyl of $R^5$ is unsubstituted or substituted with $C_{1-8}$ alkylamino or halogen, and
$R^3$ is hydrogen or $C_{1-8}$ alkyl.

Another aspect of the present invention provides a method of preparing a compound of Chemical Formula 1, which includes preparing a compound of Chemical Formula 3 from a compound of Chemical Formula 2 (Step 1); and preparing a compound of Chemical Formula 1 from the compound of Chemical Formula 3 (Step 2).

[Chemical Formula 2]

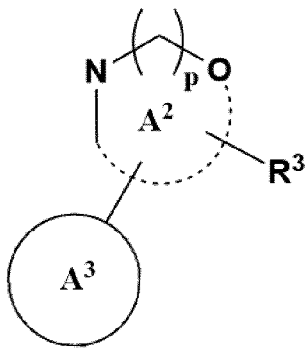

[Chemical Formula 3]

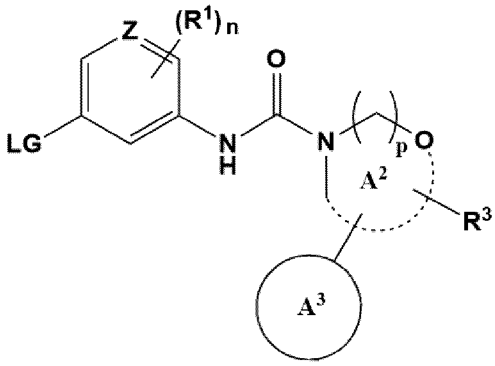

[Chemical Formula 1]

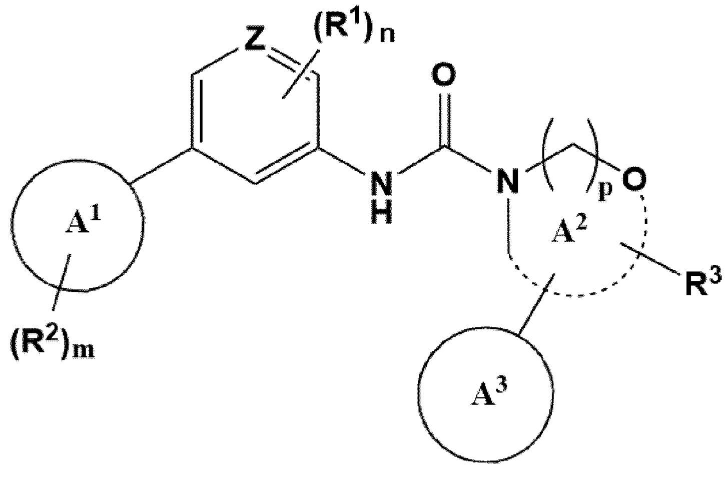

In the above Formulas, LG is a leaving group, and Z, rings $A^1$ to $A^3$, $R^1$ to $R^3$, m, n, and p may be the same as defined in this specification.

Still another aspect of the present invention provides a pharmaceutical composition for preventing or treating one or more of kinase-related diseases, which includes the above compound, an isomer thereof, a solvate thereof, a hydrate thereof, or a pharmaceutically acceptable salt thereof as an active ingredient.

[Advantageous Effects]

A compound provided in one aspect of the present invention, an isomer thereof, a solvate thereof, a hydrate thereof, or a pharmaceutically acceptable salt thereof exhibits excellent inhibitory activity against kinases, especially RIPK1, and thus has an excellent inhibitory effect on cell necrosis. Therefore, it can be effectively used as a treatment for the kinase-related diseases.

[Modes of the Invention]

Hereinafter, the present invention will be described in detail.

Meanwhile, embodiments of the present invention may be modified in various different forms, and the scope of the present invention is not limited to embodiments to be described below. In addition, the embodiments of the present invention are provided to more completely explain the present invention to those of ordinary skill in the art. Further, when a certain part "includes" a certain component, it means that, unless specifically stated otherwise, another component may be further included, rather than excluded.

Hereinafter, the present invention will be described in detail with reference to the following embodiments.

Embodiments of the present invention may be modified in a variety of different forms, and the scope of the present invention is not limited to embodiments to be explained below. In addition, the embodiments of the present invention are provided to more completely explain the present invention to those of ordinary skill in the art.

Further, when a certain part "includes" a certain component, it means that, unless particularly stated otherwise, another component may be further included, rather than excluded.

In the structural formulas of this specification, the symbol "-" and "=", which combine atoms and/or groups, may refer to a single bond and a double bond, respectively. These symbols may be omitted, and may be displayed, if necessary, for example, when specifying a bonding atom or a bonding position.

In this specification, "linking" between atoms may represent not only the case of directly linking atoms but also the case of indirectly linking atoms via another atom and/or group. Here, the other atom and/or group may be, but are not limited to, oxygen, sulfur, a $C_{1-8}$ alkylamino, or a $C_{1-8}$ alkylene, and the atom and/or group may be substituted or unsubstituted.

In this specification, "substituted or unsubstituted" may mean that, unless otherwise specified, one or more hydrogen atoms are substituted with another atom or substituent, or not substituted. The substituent may be, but is not limited to, one or more selected from the group consisting of halogen (chloro (Cl), iodo (I), bromo (Br), or fluoro (F)), $C_{1\sim10}$ alkyl, $C_{2\sim10}$ alkenyl, $C_{2\sim10}$ alkynyl, hydroxyl, $C_{1\sim10}$ alkoxy, amino, nitro, thiol, thioether, imine, cyano, phosphine, carboxy, carbamoyl, acetal, thiocarbonyl, sulfonyl, sulfonamide, ketone, aldehyde, ester, acetyl, amide, oxygen (=O),

haloalkyl (e.g., trifluoromethyl), substituted aminoacyl and amino alkyl, monocyclic or fused or unfused polycyclic carbon ring cycloalkyl (e.g., cyclopropyl, cyclobutyl, cyclopentyl, or cyclohexyl), or monocyclic or fused or unfused polycyclic heterocycloalkyl (e.g., pyrrolidinyl, piperidinyl, piperazinyl, morpholinyl, or thiazinyl), cyclic or heterocyclic, monocyclic, or fused or unfused polycyclic aryl (e.g., phenyl, naphthyl, pyrrolyl, indolyl, furanyl, thienyl, imidazolyl, oxazolyl, isoxazolyl, thiazolyl, triazolyl, tetrazolyl, pyrazolyl, pyridinyl, quinolinyl, isoquinolinyl, acridinyl, pyrazinyl, pyridizinyl, pyrimidinyl, benzimidazolyl, benzothienyl, or benzofuranyl), amino (primary, secondary or tertiary), aryl, aryloxy, and aryl-alkyl. In addition, each of the exemplified substituents may be unsubstituted or substituted with a substituent selected from the above substituent group.

In this specification, "halogen" may be F, Cl, Br, or I.

In this specification, "alkyl" may refer to, unless otherwise stated, a saturated hydrocarbon of a straight-chain or branched chain noncyclic alkyl; a cyclic alkyl; or one formed by the combination of the both above-mentioned alkyls. In addition, "Ci-s alkyl" may refer to alkyl containing 1 to 8 carbon atoms. The acyclic alkyl may include, for example, but is not limited to, methyl, ethyl, N-propyl, N-butyl, isopropyl, sec-butyl, isobutyl, tert-butyl, isopentyl, or 2-methylpentyl. The cyclic alkyl may include, for example, but is not limited to, cyclopropyl, cyclobutyl, cyclopentyl, or cyclohexyl. The alkyl, which is a combination of acyclic and cyclic alkyl, may include, for example, but is not limited to, methylcyclopropyl, cyclopropylmethyl, ethylcyclopropyl, or cyclopropylethyl.

In this specification, "cycloalkyl" may refer to, particularly, a cyclic alkyl among alkyls, where the alkyl is as defined above.

In this specification, "heterocycloalkyl" may refer to a ring containing 1 to 5 hetero atoms selected from N, O, and S as ring-forming atoms, and may be saturated or partially unsaturated. Unless otherwise specified, a heterocycloalkyl may be monocyclic, or polycyclic such as a spiro ring, a bridged ring, or a fused ring. In addition, "3- to 10-membered heterocycloalkyl" may refer to a heterocycloalkyl that include 3 to 10 ring-forming atoms, including pyrrolidine, piperidine, N-methylpiperidine, imidazolidine, pyrazolidine, imidazolidinone, piperidine, pyrimidine-2,4(1H,3H)-dione, 1,4-dioxane, morpholine, thiomorpholine, piperazine, pyran, pyridone, 3-pyrroline, thiopyran, pyrone, tetrahydrofuran, tetrahydrothiophene, 2-azaspiro[3.3]heptane, isoxazolidine, benzo[d]isoxazolidine, oxazinane, 5-oxa-6-azaspiro[2.4]heptane, (1R,5S)-3-azabicyclo[3.2.1]octane, (1s,4s)-2-azabicyclo[2.2.2]octane, or (1R,4R)-2-oxa-5-azabicyclo[2.2.2]octane, but the present invention is not limited thereto.

In this specification, "bicyclic ring" may refer to two rings that are fused while having one or more atoms in common. Specifically, it may be formed by a common single or double bond (polycyclic bicyclic ring), by sharing three or more arranged atoms (bridged bicyclic ring), or through a common single atom (spiro bicyclic ring). A bicyclic ring may be a saturated, partially unsaturated, unsaturated, or aromatic ring, and may include a hetero atom. Moreover, the ring may also include a ring in which hydrogen has been removed.

In this specification, "alkoxy" may refer to an alkyl ether group, i.e., -(O-alkyl), where the alkyl is as defined above. In addition, "Ci-s alkoxy" may refer to alkoxy containing $C_{1-8}$ alkyl, i.e., -(O-$C_{1-8}$ alkyl).

In this specification, "alkylamino" may refer to -(NR'R"), where R' and R" may each be independently selected from the group consisting of hydrogen and $C_{1-8}$ alkyl, and the selected R' and R" may each be independently substituted or unsubstituted. In addition, "$C_{1-8}$ alkylamino" may refer to $C_{1-8}$ alkyl-containing amino, that is, -N-H($C_{1-8}$ alkyl) or -N-($C_{1-8}$ alkyl)$_2$, and may be, but is not limited to, dimethylamino, diethylamino, methylethylamino, methylpropylamino, or ethylpropylamino.

In this specification, "haloalkyl" may refer to -RX (X is one or more halogens (F, Cl, Br, or I)). In other words, "haloalkyl" may be an alkyl form substituted with one or more halogens. For example, "$C_{1-8}$ haloalkyl" may be, but is not limited to, trifluoromethyl or difluoromethyl.

In this specification, "haloalkoxy" may refer to -(O-RX) (X is one or more halogens (F, Cl, Br, or I)). In other words, "haloalkoxy" may be an alkoxy form substituted with one or more halogens. For example, "$C_{1-8}$ haloalkoxy" may be, but is not limited to, trifluoromethoxy or difluoromethoxy.

In this specification, "aryl" may refer to an aromatic ring in which one hydrogen is removed from an aromatic hydrocarbon ring, and may be a monocyclic ring or a polycyclic ring. "$C_{3-12}$ aryl" may refer to an aryl including 3 to 12 ring-forming atoms, and may be, for example, but is not limited to, phenyl, benzyl, naphthyl, anthracenyl, phenanthryl, biphenyl, or terphenyl.

In this specification, "heteroaryl" may refer to an aromatic ring containing one or more hetero atoms of N, O, and S as ring-forming atoms, and may be a monocyclic ring or a polycyclic ring. In addition, "$C_{3-12}$ heteroaryl" may refer to heteroaryl including 3 to 12 ring-forming atoms, and may be, for example, but is not limited to, thienyl, thiophenyl, furyl, pyrrolyl, pyrazolyl, imidazolyl, thiazolyl, oxazolyl, isothiazolyl, oxadiazolyl, triazoyl, pyridinyl, bipyridinyl, pyrimidinyl, triazinyl, acridyl, pyridazinyl, pyrazinyl, quinolinyl, or quinazoline.

In this specification, "hydroxy" may refer to -OH.

In this specification, "alkenyl" may refer to, unless otherwise stated, a linear or branched, acyclic or cyclic hydrocarbon with one or more double bonds.

In this specification, "cyano" may refer to -(CN).

In this specification, "alkynyl" may refer to, unless otherwise stated, a linear or branched, acyclic or cyclic hydrocarbon with one or more triple bonds. For example, "$C_{2-4}$ alkynyl" may refer to alkynyl that has 2 to 4 carbon atoms and one or more triple bonds. Specifically, the alkynyl may include, but is not limited to, ethynyl, propyn-1-yl, propyn-2-yl, or methylbutyn-1-yl.

In this specification, "hydrate" may refer to a compound of the present invention that includes a stoichiometric or non-stoichiometric amount of water bound by a non-covalent intermolecular force, or a salt thereof. A hydrate of the compound represented by Chemical Formula 1 of the present invention may include a stoichiometric or non-stoichiometric amount of water bound by a non-covalent intermolecular force. The hydrate may contain 1 eq or more, and preferably, 1 eq to 5 eq of water. Such a hydrate may be prepared by crystallizing the compound represented by Chemical Formula 1 of the present invention, an isomer thereof, or a pharmaceutically acceptable salt thereof from water or a water-containing solvent.

In this specification, "solvate" may refer to a compound of the present invention, which includes a stoichiometric or non-stoichiometric amount of solvent bound by a non-covalent intermolecular force, or a salt thereof. Exemplary solvents are solvents that are volatile, non-toxic, and/or suitable for administration to humans.

In this specification, "isomer" may refer to a compound of the present invention, which has the same chemical or molecular formula, but is structurally or sterically different, or a salt thereof. These isomers include structural isomers such as tautomers, R or S isomers having an asymmetric carbon center, isomers such as geometric isomers (trans, cis), and optical isomers (enantiomers). All of these isomers and mixtures thereof are also included within the scope of the present invention.

The present invention provides a compound of Chemical Formula 1 below, an isomer thereof, a solvate thereof, a hydrate thereof, or a pharmaceutically acceptable salt thereof:

[Chemical Formula 1]

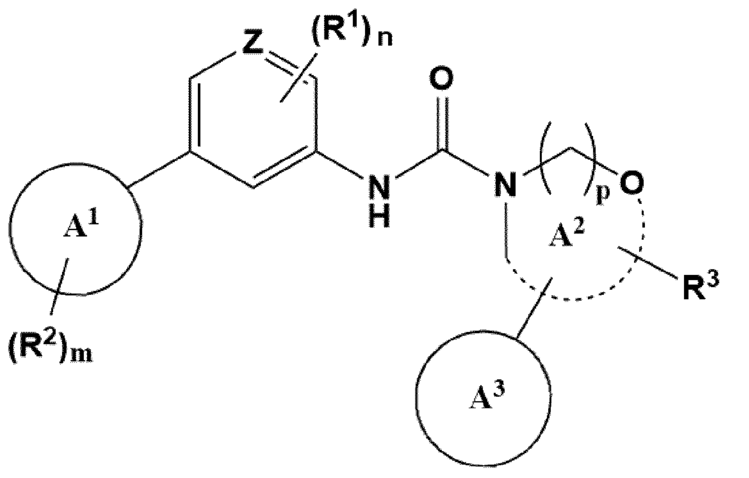

In Chemical Formula 1,

Z is N or CH,
ring $A^1$ is 5- to 12-membered heteroaryl,
ring $A^2$ is 5- to 10-membered heterocycloalkyl, 5- to 10-membered heterocycloalkyl-aryl bicyclic ring, or 5- to 10-membered heterocycloalkyl-heteroaryl bicyclic ring, wherein p is an integer from 0 to 2,
ring $A^3$ is 4- to 12-membered aryl or 4- to 12-membered heteroaryl, and is unsubstituted or substituted with one or more substituents $R^4$ selected from halogen, cyano, $C_{1-8}$ alkoxy, $C_{1-8}$ alkyl, $C_{1-8}$ haloalkyl, and $C_{1-8}$ alkylamino,
$R^1$ is hydrogen, halogen, $C_{1-8}$ alkyl, $C_{1-8}$ alkoxy, cyano, $C_{3-8}$ cycloalkyl, or $C_{3-8}$ heterocycloalkyl, and n is an integer from 1 to 3,
$R^2$ is halogen, $C_{1-8}$ alkyl, $C_{1-8}$ alkoxy, amine, cyano, $C_{1-8}$ haloalkyl, $C_{2-8}$ alkynyl which is unsubstituted or substituted with a hydroxyl group,

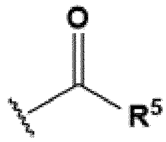, 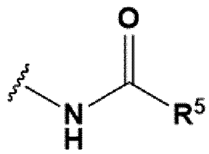,

or

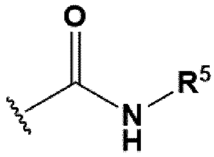,

and m is an integer from 0 to 4,
$R^5$ is $C_{1-8}$ alkyl, -$C_{1-8}$ alkyl-$C_{1-8}$ alkoxy, -$C_{1-8}$ alkyl-hydroxy, or $C_{3-8}$ cycloalkyl, where the $C_{3-8}$ cycloalkyl of $R^5$ is unsubstituted or substituted with $C_{1-8}$ alkylamino or halogen, and
$R^3$ is hydrogen or $C_{1-8}$ alkyl.

According to an exemplary embodiment of the present invention, in the compound of Chemical Formula 1,

Z is N or CH,
ring $A^1$ is 8- to 10-membered heteroaryl that includes one or more hetero atoms selected from the group consisting of N, O, and S,
ring $A^2$ is 5- to 8-membered heterocycloalkyl or 8- to 10-membered heterocycloalkyl-aryl bicyclic ring, where p is an integer from 0 to 2,
ring $A^3$ is 5- to 10-membered aryl or 4- to 8-membered heteroaryl including one or more hetero atoms selected from N, O, and S, and is unsubstituted or substituted with one or more substituents $R^4$ selected from halogen, cyano, $C_{1-6}$ alkoxy, $C_{1-6}$ alkyl, $C_{1-6}$ haloalkyl, and $C_{1-6}$ alkylamino,
$R^1$ is hydrogen, halogen, $C_{1-6}$ alkyl, $C_{1-6}$ alkoxy, cyano, or $C_{3-6}$ cycloalkyl, and n is an integer from 1 to 3,
$R^2$ is halogen, $C_{1-6}$ alkyl, $C_{1-6}$ alkoxy, amine, cyano, $C_{1-6}$ haloalkyl, or $C_{2-6}$ alkynyl that is unsubstituted or substituted with a hydroxyl group,

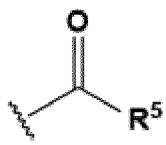, 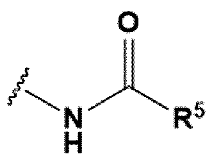,

or

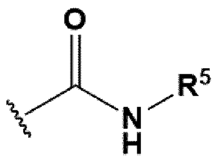,

and m is an integer from 0 to 4,
$R^5$ is $C_{1-6}$ alkyl, -$C_{1-6}$ alkyl-$C_{1-6}$ alkoxy, -$C_{1-6}$ alkyl-hydroxy, or $C_{3-6}$ cycloalkyl, where the $C_{3-6}$ cycloalkyl of $R^5$ is unsubstituted or substituted with $C_{1-6}$ alkylamino or halogen, and
$R^3$ is hydrogen or $C_{1-6}$ alkyl.

According to an exemplary embodiment of the present invention, in the compound of Chemical Formula 1, ring $A^1$ is 5- to 12-membered heteroaryl and may include one or more of hetero atoms selected from the group consisting

of N, O, and S.

According to an exemplary embodiment of the present invention, ring $A^1$ is 8- to 10-membered heteroaryl that includes one or more hetero atoms selected from the group consisting of N, O, and S, and more specifically, ring $A^1$ may include two or more of these hetero atoms.

More specifically, ring $A^1$ may be imidazo[1,2-b]pyridazine, benzo[d]oxazole, benzo[d][1,2,3]triazole, benzo[d]thiazole, indazole, thiazolo[5,4-b]pyridine, triazolo[1,5-a]pyridine, pyrrolo[2,1-f][1,2,4]triazine, triazolo[4,3-b]pyridazine, imidazo[1,2-a]pyridine, thieno[3,2-d]pyrimidine, pyrazolo[1,5-a]pyridine, thiazolo[4,5-c]pyridine, imidazo[4,5-c]pyridine, or imidazo[4,5-b]pyridine.

According to an exemplary embodiment of the present invention, ring $A^1$ may be

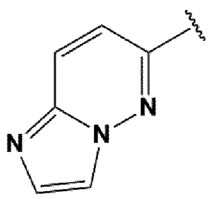 , 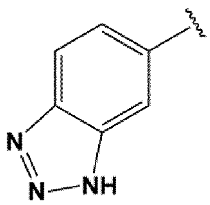 , 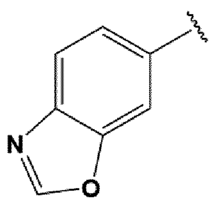 , 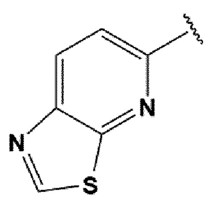 , 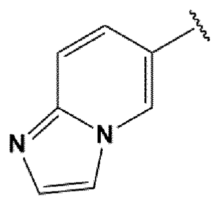 ,

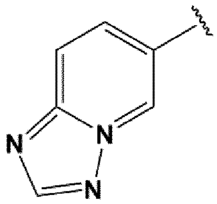 , 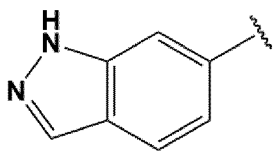 , 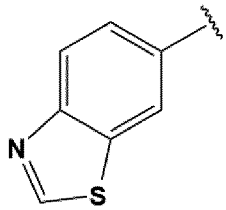 , 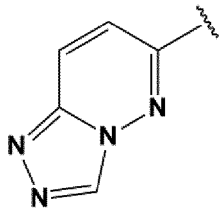 ,

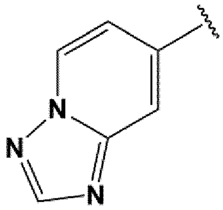 , 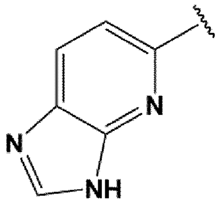 , 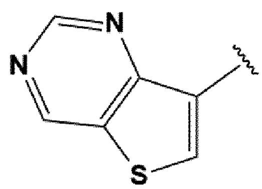 , 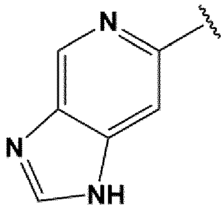 ,

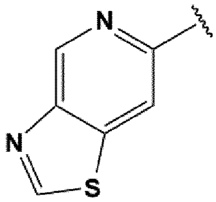 , 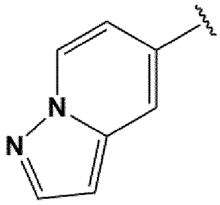 ,

or

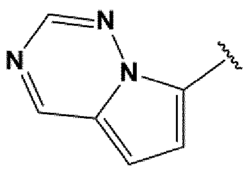 .

According to an exemplary embodiment of the present invention, in the compound of Chemical Formula 1, ring $A^2$ is 5- to 10-membered heterocycloalkyl, 5- to 10-membered heterocycloalkyl-aryl bicyclic ring, or 5- to 10-membered heterocycloalkyl-heteroaryl bicyclic ring, where p may be an integer from 0 to 2.

Specifically, ring $A^2$ may be 5- to 8-membered heterocycloalkyl including hetero atoms of N and O or an 8- to

10-membered heterocycloalkyl-aryl bicyclic ring including hetero atoms of N and O.

More specifically, ring $A^2$ may be isoxazolidine, morpholine, benzo[d]isoxazolidine, oxa-azaspiro[2.4]heptane, or oxazinane.

According to an exemplary embodiment of the present invention, in the compound of Chemical Formula 1, ring $A^3$ is 4- to 12-membered aryl or 4- to 12-membered heteroaryl, and may be unsubstituted or substituted with one or more substituents $R^4$ selected from halogen, cyano, $C_{1-8}$ alkoxy, $C_{1-8}$ alkyl, $C_{1-8}$ haloalkyl, and $C_{1-8}$ alkylamino.

Specifically, ring $A^3$ may be 5- to 10-membered aryl or 4- to 8-membered heteroaryl, more specifically, ring $A^3$ may be 5- to 10-membered aryl or 4- to 8-membered heteroaryl including one or more hetero atoms selected from N, O, and S, and most specifically, ring $A^3$ may be phenyl, pyridinyl, naphthalenyl, thiazolyl, pyrazolyl, or furanyl.

In the present invention, ring $A^3$ may be unsubstituted or substituted with one or more substituents $R^4$ selected from halogen, cyano, $C_{1-8}$ alkoxy, $C_{1-8}$ alkyl, $C_{1-8}$ haloalkyl, and $C_{1-8}$ alkylamino.

Specifically, $R^4$ may be selected from halogen, cyano, $C_{1-6}$ alkoxy, $C_{1-6}$ alkyl, $C_{1-6}$ haloalkyl, and $C_{1-6}$ alkylamino, and more specifically, $R^4$ may be selected from halogen, cyano, methoxy, methyl, trifluoromethyl, and dimethylamine.

According to an exemplary embodiment of the present invention, in the compound of Chemical Formula 1, $R^1$ is hydrogen, halogen, $C_{1-8}$ alkyl, $C_{1-8}$ alkoxy, cyano, $C_{3-8}$ cycloalkyl, or $C_{3-8}$ heterocycloalkyl, and n may be an integer from 1 to 3.

More specifically, $R^1$ may be hydrogen, halogen, $C_{1-6}$ alkyl, $C_{1-6}$ alkoxy, cyano, $C_{3-6}$ cycloalkyl, or $C_{3-6}$ heterocycloalkyl, and most specifically, $R^1$ may be hydrogen, halogen, $C_{1-3}$ alkyl, $C_{1-3}$ alkoxy, cyano, or $C_{3-6}$ cycloalkyl.

According to an exemplary embodiment of the present invention, when Z is N, $R^1$ may be $C_{1-8}$ alkyl or $C_{1-8}$ alkoxy.

According to an exemplary embodiment of the present invention, in the compound of Chemical Formula 1,

$R^2$ is halogen, $C_{1-8}$ alkyl, $C_{1-8}$ alkoxy, amine, cyano, $C_{1-8}$ haloalkyl, or $C_{2-8}$ alkynyl that is unsubstituted or substituted with a hydroxyl group,

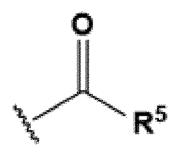,
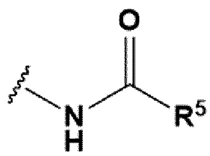,

or

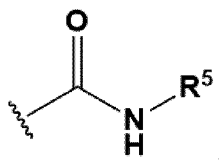,

and m is an integer from 0 to 4, and

$R^5$ is $C_{1-8}$ alkyl, $-C_{1-8}$ alkyl-$C_{1-8}$ alkoxy, $-C_{1-8}$ alkyl-hydroxy, or $C_{3-8}$ cycloalkyl, where the $C_{3-8}$ cycloalkyl of $R^5$ is unsubstituted or substituted with $C_{1-8}$ alkylamino or halogen.

According to an exemplary embodiment of the present invention, $R^2$ may be halogen, $C_{1-6}$ alkyl, $C_{1-6}$ alkoxy, amine, cyano, $C_{1-6}$ haloalkyl, or $C_{2-6}$ alkynyl that is unsubstituted or substituted with a hydroxyl group,

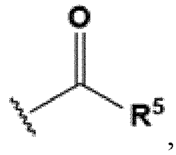,
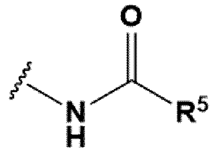,

or

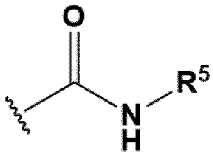,

and more specifically, $R^2$ may be $C_{1-3}$ alkyl, amine, cyano, $C_{1-3}$ haloalkyl, ethynyl, hydroxymethylbutynyl,

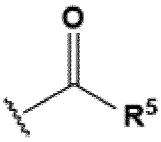, 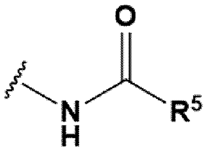, or 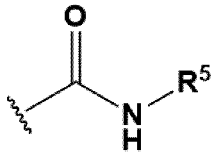.

Here, m may be an integer from 0 to 3, and more specifically, an integer from 0 to 2. When m is 0, ring $A^1$ means an unsubstituted ring.

According to an exemplary embodiment of the present invention, $R^5$ is $C_{1-6}$ alkyl, $-C_{1-6}$ alkyl-$C_{1-6}$ alkoxy, $-C_{1-6}$ alkyl-hydroxy, or $C_{3-6}$ cycloalkyl, where the $C_{3-6}$ cycloalkyl of $R^5$ may be unsubstituted or substituted with $C_{1-6}$ alkylamino or halogen.

According to an exemplary embodiment of the present invention,

when $R^2$ is

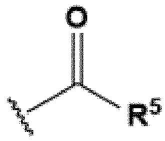,

$R^5$ is $C_{3-8}$ cycloalkyl that is unsubstituted or substituted with halogen,

when $R^2$ is

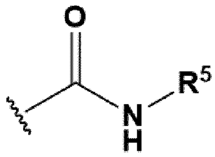,

$R^5$ is $C_{1-8}$ alkyl or $C_{3-8}$ cycloalkyl that is unsubstituted or substituted with $C_{1-8}$ alkylamino,

when $R^2$ is

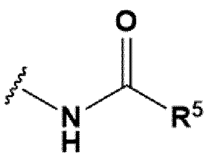,

$R^5$ may be $C_{3-8}$ cycloalkyl that is unsubstituted or substituted with $C_{1-8}$ alkyl, $-C_{1-8}$ alkyl-$C_{1-8}$ alkoxy, $-C_{1-8}$ alkyl-hydroxy, or halogen.

According to an exemplary embodiment of the present invention, in the compound of Chemical Formula 1, $R^3$ may be hydrogen or $C_{1-8}$ alkyl, and specifically, $R^3$ may be hydrogen or $C_{1-3}$ alkyl.

Examples of the compound of Chemical Formula 1 according to the present invention may include Compounds 1 to 158 listed in [Table 2] in the following examples, or free bases (when shown as pharmaceutically acceptable salts in Table 2), isomers thereof, hydrates thereof, solvates thereof, or pharmaceutically acceptable salts thereof.

The compound represented by Chemical Formula 1 of the present invention may be used in the form of a pharmaceutically acceptable salt thereof. Particularly, the pharmaceutically acceptable salt may be an acid addition salt formed by a free acid. Here, acid addition salts may be obtained from inorganic acids such as hydrochloric acid, nitric acid, phosphoric acid, sulfuric acid, hydrobromic acid, hydroiodic acid, nitrous acid, and phosphorous acid; non-toxic organic acids such as aliphatic mono- and di-carboxylates, phenyl-substituted alkanoates, hydroxy alkanoates, and alkanedioates, aromatic acids, and aliphatic and aromatic sulfonic acids; and organic acids such as trifluoroacetic acid, acetate, benzoic acid, citric acid, lactic acid, maleic acid, gluconic acid, methanesulfonic acid, 4-toluenesulfonic acid, tartaric acid, and fumaric acid. The types of such pharmaceutically acceptable salts may include sulfate, pyrosulfate, bisulfate, sulfite, bisulfite, nitrate, phosphate, monohydrogen phosphate, dihydrogen phosphate, metaphosphate, pyrophosphate chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexane-1,6-dioate, benzoate, chlorobenzoate, methylbenzoate, dinitro benzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, β-hydroxyburyrate, glycolate, maleate, tartrate, methanesulfonate, propanesulfonate, naphthalene-1-sulfonate, naphthalene-2-sulfonate, and mandelate. The acid addition salt may be prepared by a conventional method, for example, by dissolving a derivative of Chemical Formula 1 in an organic solvent such as methanol, ethanol, acetone, methylene chloride, or acetonitrile, adding an organic acid or inorganic acid, and filtering and drying the resulting precipitate, or prepared by distilling the solvent and excess acid under reduced pressure, dehydrating the resulting product and crystallizing the dehydrated product in an organic solvent. In addition, the pharmaceutically acceptable salt may be a salt or metal salt obtained using a base. As an example of the metal salt, an alkali metal or alkaline earth metal salt may be obtained by dissolving the compound in an excess alkali metal hydroxide or alkaline earth metal hydroxide solution, filtering an undissolved compound salt, and evaporating and dehydrating the filtrate. As an alkali metal salt, a sodium, potassium, or calcium salt may be pharmaceutically acceptable. In addition, corresponding salts may be obtained by reacting an alkali metal or alkaline earth metal salt with a suitable silver salt (e.g., silver nitrate).

Further, the present invention may include not only the compound represented by Chemical Formula 1 and a pharmaceutically acceptable salt thereof, but also an isomer thereof, especially a stereoisomer or enantiomer, or a hydrate and//or solvate that can be prepared therefrom.

The present invention may also provide a method of preparing the compound of Chemical Formula 1.

The method of preparing the compound of Chemical Formula 1 may include

preparing a compound of Chemical Formula 3 from a compound of Chemical Formula 2 (Step 1); and
preparing the compound of Chemical Formula 1 from the compound of Chemical Formula 3 (Step 2).

[Chemical Formula 2]

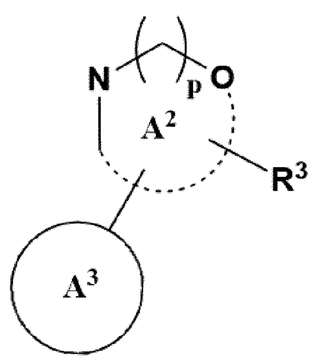

[Chemical Formula 3]

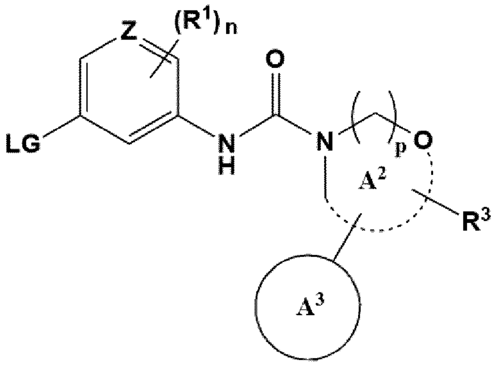

[Chemical Formula 1]

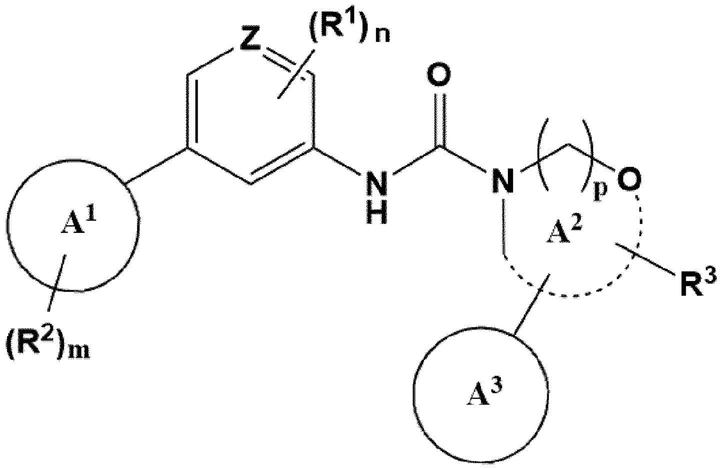

In the above Formulas, LG is a leaving group, and Z, rings $A^1$ to $A^3$, $R^1$ to $R^3$, m, n, and p may each be independently the same as defined in this specification. The leaving group may be a functional group such as a halogen, sulfonic acid ester, boronic ester, boronic acid, or alkoxy, and it is not limited as long as it is a functional group capable of producing the compound of Chemical Formula 1 by being detached from the compound of Chemical Formula 3.

In the method of preparing the compound of Chemical Formula 1 of the present invention, Step 1 may be a step of preparing the compound of Chemical Formula 3 by reacting the compound of Chemical Formula 2 with

[Chemical Formula 4]

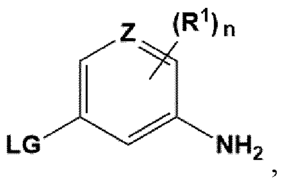

and LG-C(=O)-LG. Here, the leaving group (LG) is not limited as long as it is a functional group that can produce the compound of Chemical Formula 3 by being detached through the reaction of the compound of Chemical Formula 2 and the compound of Chemical Formula 4, and is preferably alkoxy or halogen.

In the method of preparing the compound of Chemical Formula 1 of the present invention, Step 2 may include substituting the LG group of the compound of Chemical Formula 3 with boronic ester or dioxaborolane, and reacting the resulting product with $(R^2)_m$-$A^1$-LG (LG may be halogen). Step 2 may be to link ring $A^1$ with a

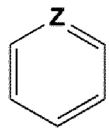

group through the Suzuki coupling reaction.

The present invention may also provide a pharmaceutical composition for preventing or treating one or more kinase-related diseases, which includes the compound, an isomer thereof, a solvate thereof, a hydrate thereof, or a pharmaceutically acceptable salt thereof as an active ingredient.

The compound of Chemical Formula 1 of the present invention may exhibit excellent inhibitory activity against various kinases, particularly, receptor-interacting serine/threonine-protein kinase 1 (RIPK1). In addition, the compound of Chemical Formula 1 may effectively inhibit cell necrosis, particularly, cell necrosis caused by necroptosis.

In the present invention, the disease may be a disease/disorder that may be at least partially regulated by programmed necrosis, apoptosis, or production of an inflammatory cytokine, particularly, inflammatory bowel disease (including Crohn's disease and ulcerative colitis), psoriasis, retinal detachment (and degeneration), retinitis pigmentosa, macular degeneration, pancreatitis, atopic dermatitis, arthritis (including rheumatoid arthritis, spondyloarthritis, gout, juvenile idiopathic arthritis (systemic juvenile idiopathic arthritis (SoJIA)), and psoriatic arthritis), systemic lupus erythematosus (SLE), Sjogren's syndrome, systemic scleroderma, anti-phospholipid syndrome (APS), vasculitis, osteoarthritis, a liver injury/disease (non-alcoholic steatohepatitis, alcoholic steatohepatitis, autoimmune hepatitis, autoimmune hepatobiliary disease, primary sclerosing cholangitis (PSC), acetaminophen toxicity, or hepatotoxicity), kidney injury/damage (nephritis, kidney transplant, surgery, administration of a nephrotoxic drug, such as cisplatin, or acute kidney injury (AKI)), celiac disease, autoimmune idiopathic thrombocytopenic purpura (autoimmune ITP), transplant rejection (rejection of a transplanted organ, tissue, or cells), ischemia-reperfusion injury of parenchymal organs, sepsis, systemic inflammatory response syndrome (SIRS), cerebrovascular accident (CVA or stroke), myocardial infarction (MI), atherosclerosis, Huntington's disease, Alzheimer's disease, Parkinson's disease, amyotrophic lateral sclerosis (ALS), neonatal hypoxic brain injury, ischemic brain injury, traumatic brain injury, allergic disease (including asthma and atopic dermatitis), burns, multiple sclerosis, type I diabetes, Wegener's granulomatosis, pulmonary sarcoidosis, Behcet's disease, interleukin-1 converting enzyme (ICE, also known as caspase-1)-associated fever syndrome, chronic obstructive pulmonary disease (COPD), cigarette smoke-induced injury, cystic fibrosis, tumor necrosis factor receptor-associated periodic syndrome (TRAPS), neoplastic tumors, periodontitis, NEMO mutation (mutation in NF-kappa-B essential regulator gene (also known as IKK gamma or IKKG)), particularly, NEMO-deficient syndrome, HOIL-1 deficiency (also known as RBCK1), heme-oxidized IRP2 ubiquitin ligase-1 deficiency), linear ubiquitin chain assembly complex (LUBAC)-deficient syndrome, blood and parenchymal malignancies, bacterial and viral infections (e.g., influenza, staphylococcus, and mycobacterium (tuberculosis)), and a lysosomal storage disease (particularly, Gaucher disease, GM2 gangliosidosis, alpha-mannosidosis, aspartylglucosaminuria, cholesteryl ester storage disease, chronic hexosaminidase A deficiency, cystinosis, Danon disease, Fabry disease, Farber disease, fucosidosis, galactosialidosis, GM1 gangliosidosis, mucolipidosis, infantile sialic acid storage disease, juvenile hexosaminidase A deficiency, Krabbe disease, lysosomal acid lipid deficiency, metachromatic leukodystrophy, mucopolysaccharidosis, multiple sulfatase deficiency, Niemann-Pick disease, neuronal ceroid lipofuscinose, Pompe disease, pyknodysostosis, Sandhoff disease, Shindler disease, sialic acid storage disease, Tay-Sachs' disease, or Wolman's disease), Stevens-Johnson syndrome, glaucoma, spinal cord injury, mesothelioma, melanoma, or acute liver failure, but the present invention is not limited thereto.

More specifically, the disease may be selected from the group consisting of inflammatory bowel disease, Crohn's disease, ulcerative colitis, psoriasis, retinal detachment, retinitis pigmentosa, macular degeneration, pancreatitis, atopic dermatitis, rheumatoid arthritis, spondyloarthritis, gout, Sjogren's syndrome, systemic scleroderma, anti-phospholipid syndrome, vasculitis, osteoarthritis, non-alcoholic steatohepatitis, alcoholic steatohepatitis, primary sclerosing cholangitis, nephritis, celiac disease, transplant rejection, sepsis, systemic inflammatory response syndrome, myocardial infarction, Huntington's disease, Alzheimer's disease, Parkinson's disease, allergic disease, asthma, atopic dermatitis, multiple sclerosis, type I diabetes, Wegener's granulomatosis, pulmonary sarcoidosis, Behcet's disease, motor neurone disease, chronic obstructive pulmonary disease, and periodontitis, but the present invention is not limited thereto.

The pharmaceutical composition of the present invention may inhibit the expression or activity of RIPK1 kinase.

The pharmaceutical composition of the present invention may include a pharmaceutically acceptable carrier. The pharmaceutically acceptable carrier may include diluents or excipients such as a filler, a thickening agent, a binder, a wetting agent, a disintegrant, and a surfactant, and the composition of the present invention may be formulated with them.

The pharmaceutical composition of the present invention may be used in clinical administration and prepared to be administered in a variety of oral and parenteral formulations.

Solid preparations for oral administration may include tablets, pills, powders, granules, and capsules, and such solid preparations may be prepared by mixing at least one excipient, such as starch, calcium carbonate, sucrose, lactose,

or gelatin with one or more compounds. Aside from simple excipients, lubricants such as magnesium stearate, talc, etc. may also be used.

In addition, as liquid preparations for oral administration, a suspension, a liquid for internal use, an emulsion, or a syrup may be used, and a generally-used simple diluent such as water or liquid paraffin, as well as various types of excipients, for example, a wetting agent, a sweetener, a fragrance and a preservative may be included.

As preparations for parenteral administration, a sterile aqueous solution, a non-aqueous solvent, a suspension, or an emulsion may be used, and as the non-aqueous solvent or suspension, propylene glycol, polyethylene glycol, a vegetable oil such as olive oil, or an injectable ester such as ethyl oleate may be used.

In addition, parenteral administration may be performed by subcutaneous injection, intravenous injection, intramuscular injection, or intrathoracic injection. Here, for preparation into formulations for parenteral administration, the pharmaceutical composition may be prepared in a solution or suspension by mixing a compound represented by Chemical Formula 1 or a pharmaceutically acceptable salt thereof with a stabilizer or buffer in water, and prepared in unit-dose ampoules or vials. The composition may be sterilized and/or contain preservatives, stabilizers, wetting agents or emulsion accelerators, auxiliary materials such as salts and/or buffers for regulating osmotic pressure, and other therapeutically useful substances through mixing, granulating or coating according to a conventional method.

The present invention provides a method of preventing or treating a kinase-related disease, which includes administering the compound, an isomer thereof, a solvate thereof, a hydrate thereof, a pharmaceutically acceptable salt thereof, or a pharmaceutical composition including the same to a subject in need thereof at a pharmaceutically effective amount. The subject may be a mammal, including a human.

The term "pharmaceutically effective amount" used herein refers to an amount of the compound represented by Chemical Formula 1 that is effective in treatment or prevention of the kinase-related disease. Specifically, the "pharmaceutically effective amount" used herein refers to an amount sufficient for treating a disease at a reasonable benefit/risk ratio applicable for medical treatment, and an effective dosage may be determined by factors including a type of individual, severity, age, gender, a type of disease, drug activity, sensitivity to a drug, administration time, an administration route, an excretion rate, the duration of treatment, and concurrently used drugs, and other factors well known in the medical field. The pharmaceutical composition of the present invention may be administered separately or in combination with other therapeutic agents, and may be sequentially or simultaneously administered with a conventional therapeutic agent, or administered in a single or multiple dose(s). In consideration of all of the above-mentioned parameters, it is important to achieve the maximum effect with the minimum dose without side effects, and such a dose may be easily determined by one of ordinary skill in the art. The dosage of the pharmaceutical composition of the present invention may be determined by an expert depending on various factors such as a patient's condition, age, gender, and complications. Since the active ingredient of the pharmaceutical composition of the present invention has excellent safety, the pharmaceutical composition of the present invention may be used at a dose exceeding the determined dosage.

The present invention provides a use of the compound of Chemical Formula 1, an isomer thereof, a solvate thereof, a hydrate thereof, or a pharmaceutically acceptable salt thereof for preparing a medicament used in the prevention or treatment of a kinase-related disease.

In the use and treatment method of the present invention, the compound of Chemical Formula 1, an isomer thereof, a solvate thereof, a hydrate thereof, a pharmaceutically acceptable salt thereof, and a kinase-related disease may be applied as described above.

Hereinafter, the present invention will be described in detail with reference to examples and experimental examples.

However, the following examples and experimental examples are merely provided to exemplify the present invention, and the contents of the present invention are not limited to the following examples.

## Examples

Compounds synthesized in Examples of the present invention were purified or subjected to structural analysis under the following UPLC conditions: Medium pressure liquid chromatography (MPLC) for purification; For MPLC, CombiFlash Rf +UV (TELEEDYNE ISCO) was used.

HPLC conditions for analysis (ACQUITY UPLC H-Class Core System)

For the Waters UPLC system (ACQUITY UPLC PDA Detector), equipment equipped with the Waters mass QDA detector was used. The column used was Waters ACQUITY UPLC®BEH C18 (1.7 μm, 2.1X50 mm), and a column temperature was 30 °C.

Mobile phase A was water containing 0.1% formic acid, and mobile phase B was acetonitrile containing 0.1% formic acid.

Gradient conditions (10-100% B for 3 min; flow rate = 0.6 mL/min)

Prep-150 LC System for purification (Preparative-Liquid chromatography UV spectrometry)

For the Waters Prep 150 LC system (2545 Quaternary gradient module, 2998 Photodiode Array Detector, Fraction collector III), equipment manufactured by Waters was used. The column used was Waters XTERRA®Prep RP18 OBD™(10 μm, 30X300 mm), and a column temperature was room temperature.

Gradient conditions (3-100% B for 120 min; flow rate = 40 mL/min)

Commercial reagents used were used without further purification. In the present invention, room temperature is approximately 1 to 35 °C. For concentration under reduced pressure or distillation for removing a solvent, a rotary evaporator was used.

**<Preparation Example 1> Preparation of (S)-3-phenylisoxazolidine**

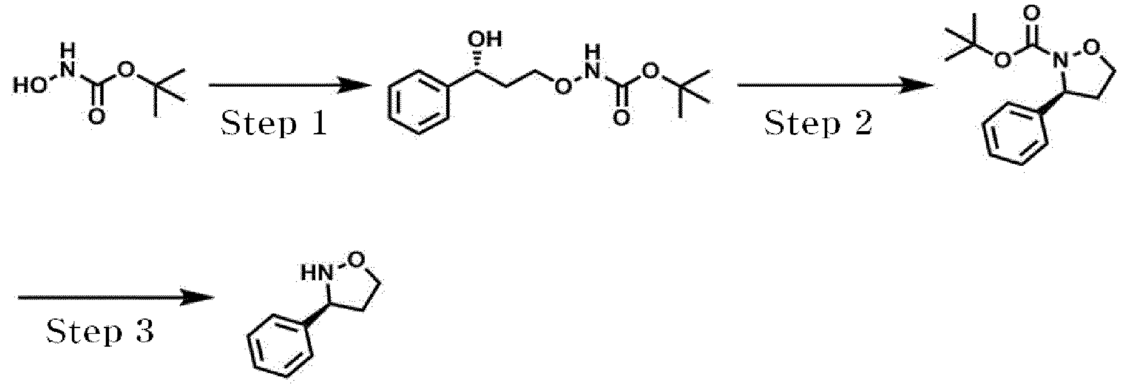


Step 1: Preparation of tert-butyl (R)-(3-hydroxy-3-phenylpropoxy)carbamate

After dissolving tert-butyl hydroxycarbamate (7.8 g, 58.6 mmol) in dimethylformamide (DMF, 140 mL), NaH (2.58 g, 64.5 mmol) was added at 0 °C and reacted for 30 minutes. Then, (R)-3-chloro-1-phenylpropan-1-ol (5 g, 29.3 mmol) dissolved in DMF (10 mL) was slowly added dropwise for 10 minutes at 0 °C and stirred at room temperature for 72 hours. The reaction was terminated by adding an aqueous ammonium chloride solution to the reaction mixture, the resulting mixture was extracted with ethyl acetate (EA) and brine, and then organic layers were combined. The organic layer was dried over sodium sulfate, concentrated under reduced pressure, and purified by MPLC (ethyl acetate/hexane, EA/Hex), thereby obtaining the target compound (2.8 g, 68%).

MS (m/z): 150.17 $[M+1]^+$

$^1H$ NMR (400 MHz, $CDCl_3$) $\delta$ = 7.43 - 7.39 (m, 2H), 7.38 - 7.32 (m, 2H), 7.27 - 7.24 (m, 1H), 5.05 - 4.97 (m, 1H), 4.15 - 4.08 (m, 1H), 4.07 - 4.00 (m, 1H), 2.10 - 1.93 (m, 2H), 1.54 - 1.48 (m, 9H)

Step 2: Preparation of tert-butyl (S)-3-phenylisoxazolidine-2-carboxylate

Tert-butyl (R)-(3-hydroxy-3-phenylpropoxy)carbamate (2.55 g, 9.54 mmol) obtained in Step 1 of Preparation Example 1 and triethylamine (TEA, 3.13 mL, 22.44 mmol) were dissolved in dichloromethane (DCM, 250 mL) and cooled to 0 °C. Then, methanesulfonyl chloride (1 mL, 13 mmol) was added dropwise and stirred at 0 °C for 2 hours. The reaction mixture was extracted with brine and DCM, and then organic layers were combined. The organic layer was dried over sodium sulfate, concentrated under reduced pressure to obtain the target compound, and then the obtained compound was used in the next reaction without purification.

MS (m/z): 194.13 $[M+1]^+$

$^1H$ NMR (400 MHz, $CDCl_3$) $\delta$ = 7.29 - 7.23 (m, 4H), 7.18 - 7.17 (m, 1H), 5.12 - 5.11 (m, 1H), 4.10 - 4.03 (m, 1H), 3.82- 3.80 (m, 1H), 2.75 - 2.65 (m, 1H), 2.29 - 2.15 (m, 1H), 1.37 (s, 9H)

Step 3: Preparation of (S)-3-phenylisoxazolidine

Tert-butyl (S)-3-phenylisoxazolidine-2-carboxylate (2.3 g) obtained in Step 2 of Preparation Example 1 was dissolved in DCM (90 mL), and trifluoroacetic acid (TFA, 14 mL) was added and reacted at room temperature for 1 hour. The reaction mixture was neutralized with an NaHCOs aqueous solution, and then organic layers were combined. The

organic layer was dried over sodium sulfate, concentrated under reduced pressure, and purified by MPLC (tetrahydrofuran/hexane, THF/Hex), thereby obtaining the target compound (1.3 g, 94%).

MS (m/z): 150.08 $[M+1]^+$

$^1H$ NMR (400 MHz, DMSO-$d_6$) δ = 7.59 - 7.52 (m, 2H), 7.50 - 7.39 (m, 3H), 5.01 - 4.93 (m, 1H), 2.93 - 2.82 (m, 1H), 2.62 - 2.53 (m, 2H)

**<Preparation Example 2> Preparation of (S)-3-(3-fluorophenyl)isoxazolidine**

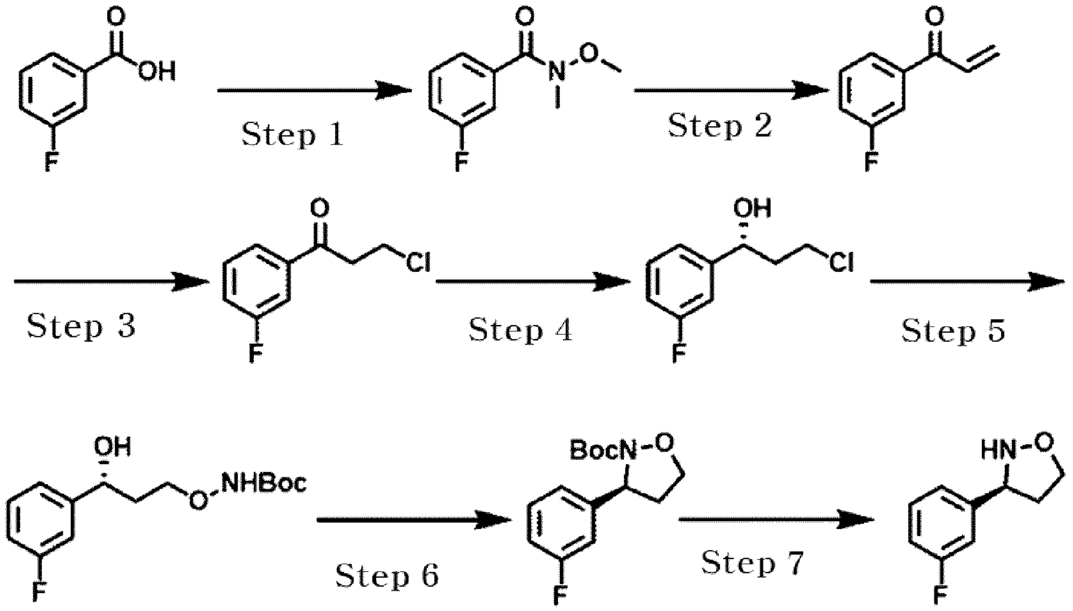


Step 1: Preparation of 3-fluoro-N-methoxy-N-methylbenzamide

After dissolving 3-fluorobenzoic acid (90 g, 642.35 mmol, 1 eq) in pyridine (150 mL), N,O-dimethylhydroxylamine hydrochloride (75.19 g, 770.81 mmol, 1.2 eq) was added. Afterward, 1-ethyl-3-(3-dimethylaminopropyl)carbodiimide (EDCI, 147.77 g, 770.81 mmol, 1.2 eq) was added at 15 °C. The reaction mixture was stirred at 50 °C for 30 minutes. As a result of TLC analysis (petroleum ether (PE):EA = 3:1), all starting materials disappeared and a new spot with low polarity was detected. The pyridine solvent was removed by concentration under reduced pressure, and an organic layer was extracted using DCM (500 mL), 2N HCl (500 mL), and brine (200 mL). The organic layer was dried over sodium sulfate and concentrated under reduced pressure, thereby obtaining the target compound as a yellow oil (110 g, yield: 93.5%).

Step 2: Preparation of 1-(3-fluorophenyl)prop-2-en-1-one

After dissolving the 3-fluoro-N-methoxy-N-methylbenzamide (110 g, 600.50 mmol, 1 eq) obtained in Step 1 of Preparation Example 2 in THF (1 L), at 0 °C, bromo(vinyl)magnesium (1 M, 630.53 mL, 1.05 eq) was added dropwise at -78 °C. Afterward, the reaction mixture was stirred at 0 °C for 30 minutes. As a result of TLC analysis (PE:EA=4:1), all starting materials disappeared and a new spot with low polarity was detected. After terminating the reaction by adding an HCl aqueous solution (4N, 500 mL), an organic layer was extracted using methyl tert-butyl ether (MTBE, 2000 mL) and brine (500 mL). The organic layer was dried over sodium sulfate and concentrated under reduced pressure. The concentrated compound was purified by MPLC (PE/EA=30/1), thereby obtaining the target compound as a yellow oil (80 g, 532.80 mmol, yield : 88.73%).

Step 3: Preparation of 3-chloro-1-(3-fluorophenyl)propane-1-one

After dissolving the 1-(3-fluorophenyl)prop-2-en-1-one (71 g, 472.86 mmol, 1.0 eq) obtained in Step 2 of Preparation Example 2 in DCM (71 mL), HCl in 1,4-dioxane (4 M, 295.54 mL, 2.5 eq) was added at 0 °C. Afterward, the reaction mixture was stirred at 15 °C for 1.5 hours. As a result of TLC analysis (PE:EA=10:1), all starting materials

disappeared, and the target compound was detected. The reaction mixture was concentrated under reduced pressure, an organic layer was extracted by adding DCM (450 mL) and water (200 mL * 5), dried over sodium sulfate, and concentrated under reduced pressure, thereby obtaining the target compound as a yellow solid (73 g, yield: 82.7%).

$^1H$ NMR (400MHz, $CDCl_3$) $\delta$ = 7.78 - 7.72 (m, 1H), 7.69 - 7.60 (m, 1H), 7.53 - 7.44 (m, 1H), 7.37 - 7.24 (m, 1H), 3.93 (t, J=6.8 Hz, 2H), 3.46 (t, J=6.8 Hz, 2H)

Step 4: Preparation of (R)-3-chloro-1-(3-fluorophenyl)propane-1-ol

After dissolving (S)-1-methyl-3,3-diphenyltetrahydro-1H,3H-pyrrolo[1,2-c][1,3,2]oxazaborol (8.61 g, 31.08 mmol, 9.27 mL, 0.1 eq) in THF (380 mL), $BH_3$.THF (1 M, 186.48 mL, 0.6 eq) was added dropwise under a nitrogen stream at 0 °C. The reaction mixture was stirred at 0 °C for 30 minutes. Afterward, after diluting the reaction mixture in THF (390 mL), the 3-chloro-1-(3-fluorophenyl)propan-1-one (70 g, 375.11 mmol, 1 eq) obtained in Step 3 of Preparation Example 2 was added dropwise at 0 °C. The reaction mixture was stirred at 0 °C for 30 minutes. As a result of TLC analysis (PE:EA=5:1), all starting materials disappeared and a target compound spot was detected. After terminating the reaction by adding methanol (MeOH, 100 mL) at 0 °C, the solvent was concentrated under reduced pressure. The organic layer of the concentrated compound was extracted using DCM (100 mL*3) and an $NH_4Cl$ solution (300 mL). The organic layer was dried over sodium sulfate and concentrated under reduced pressure. The concentrated compound was purified by MPLC (PE:EA=50:1 to 5:1), thereby obtaining the target compound as colorless oil (55 g, yield: 92%).

MS (m/z): 135.2 $[M-56+H]^+$

$^1H$ NMR (400 MHz, $CDCl_3$) $\delta$ = 7.35 - 7.28 (m, 1H), 7.19 - 7.04 (m, 2H), 7.01 - 6.91 (m, 1H), 5.00 - 4.88 (m, 1H), 3.76 - 3.70 (m, 1H), 3.60 - 3.51 (m, 1H), 2.24 - 2.13 (m, 1H), 2.12 - 1.98 (m, 2H)

Step 5: Preparation of tert-butyl (R)-(3-(3-fluorophenyl)-3-hydroxypropoxy)carbamate

After dissolving tert-butyl hydroxycarbamate (40.76 g, 306.16 mmol, 1.05 eq) in DMF (400 mL), NaH (12.83 g, 320.74 mmol, 60% purity, 1.1 eq) was added under a nitrogen stream at 0 °C. The reaction mixture was stirred at 10 °C for 1 hour, the (R)-3-chloro-1-(3-fluorophenyl)propan-1-ol (55 g, 291.58 mmol, 1 eq) obtained in Step 4 of Preparation Example 2, which had been diluted in DMF (150 mL), was added dropwise at 0 °C, and stirred at 10 °C for 16 hours. As a result of TLC analysis (PE:EA=2:1), all starting materials disappeared and the target compound was detected. After terminating the reaction by adding water (800 mL), the resulting solid was filtered. The obtained solid was treated with EA (800 mL), water (100 mL), and brine (100 mL) to extract an organic layer. The organic layer was dried over sodium sulfate and concentrated under reduced pressure, thereby obtaining the target compound as a light yellow solid (72 g, yield: 65%).

MS (m/z): 167.9 $[M-118+H]^+$

$^1H$ NMR (400 MHz, $CDCl_3$) $\delta$ = 7.17 - 7.06 (m, 1H), 7.01 - 6.93 (m, 2H), 6.80 - 6.73 (m, 1H), 4.89 - 4.79 (m, 1H), 3.95 - 3.89 (m, 1H), 3.88 - 3.82 (m, 1H), 1.89 - 1.81 (m, 1H), 1.80 - 1.69 (m, 1H), 1.32 (s, 9H)

Step 6: Preparation of tert-butyl (S)-3-(3-fluorophenyl)isoxazolidine-2-carboxylate

After dissolving the tert-butyl (R)-(3-(3-fluorophenyl)-3-hydroxypropoxy)carbamate (72 g, 252.36 mmol, 1 eq) obtained in Step 5 of Preparation Example 2 and TEA (76.61 g, 757.07 mmol, 105.38 mL, 3 eq) in DCM (700 mL), anhydrous methanesulfonic acid (65.94 g, 378.53 mmol, 1.5 eq) was slowly added at 0 °C. The reaction mixture was stirred at 20 °C for 12 hours. As a result of TLC analysis (PE:EA=3:1), all starting materials disappeared and a new spot was detected. After terminating the reaction by adding water (1000 mL), an organic layer was extracted using DCM (200 mL * 3). The organic layer was dried over sodium sulfate and concentrated under reduced pressure. The concentrated compound was purified by MPLC (PE:EA=50:1 to 5:1) to extract 35 g of the target compound with an ee value of 57.7%. The target compound was purified through SFC (column: DAICEL CHIRALPAK AD-H (250 mm*30 mm,5 $\mu$m); mobile phase: [Neu-MeOH]; B%: 15 %, 2.9 min;760 min), thereby obtaining the target compound as a light yellow solid (25 g, yield: 36.2%) and tert-butyl (R)-3-(3-fluorophenyl)isoxazolidine-2-carboxylate (6.5 g, yield: 8.7%).

MS (m/z): 212.2 $[M-56+H]^+$

$^1H$ NMR (400 MHz, $CDCl_3$) $\delta$ = 7.34 - 7.27 (m, 1H), 7.17 - 7.05 (m, 2H), 6.99 - 6.91 (m, 1H), 5.20 (dd, J=5.6, 8.8 Hz, 1H), 4.22 - 4.14 (m, 1H), 3.94 - 3.84 (m, 1H), 2.84 - 2.73 (m, 1H), 2.34 - 2.22 (m, 1H), 1.47 (s, 9H)

Step 7: Preparation of (S)-3-(3-fluorophenyl)isoxazolidine

After dissolving the tert-butyl (S)-3-(3-fluorophenyl)isoxazolidine-2-carboxylate (25 g, 93.53 mmol, 1 eq) obtained in Step 6 of Preparation Example 2 in EA (50 mL), HCl in EA (4M, 250 mL) was added at 0 °C. Afterward, the reaction mixture was stirred at 10 °C for 1 hour. As a result of LC-MS analysis, all starting materials disappeared and

96.97% of the target compound was detected. The target compound (19.12 g, yield: 93%) was obtained as a white solid by concentration under reduced pressure for obtaining a solid.

MS (m/z): 168.3 $[M+H]^+$

$^1H$ NMR (400 MHz, DMSO-$d_6$) $\delta$ = 12.51 (br s, 1H), 7.55 - 7.44 (m, 2H), 7.44 - 7.37 (m, 1H), 7.32 - 7.22 (m, 1H), 5.03 (t, J=8.0 Hz, 1H), 4.54 - 4.43 (m, 1H), 4.34 - 4.24 (m, 1H), 2.95 - 2.83 (m, 1H), 2.64 - 2.52 (m, 1H)

The compounds of Preparation Examples 3 to 33 were prepared in a similar manner to Preparation Examples 1 and 2, and the names, chemical structure formulas, and LC-MS analysis results for the compounds of Preparation Examples 3 to 33 are summarized in Table 1.

[Table 1]

| Preparation Example | Structural Formula | Name of Compound | LC-MS $[M+H]^+$ |
|---|---|---|---|
| 3 | 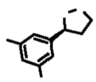 | (S)-3-(3,5-difluorophenyl)isoxazolidine | 186.1 |
| 4 | 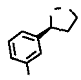 | (S)-3-(3-chlorophenyl)isoxazolidine | 184.1 |
| 5 | 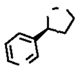 | (S)-3-(pyridin-3-yl)isoxazolidine | 151.1 |
| 6 | 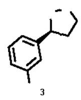 | (S)-3-(3-(trifluoromethyl)phenyl)isoxazolidine | 218.1 |
| 7 | 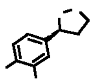 | (S)-3-(3-chloro-4-fluorophenyl)isoxazolidine | 202.0 |
| 8 | 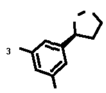 | (S)-3-(3-fluoro-5-(trifluoromethyl)phenyl) isoxazolidine | 236.1 |
| 9 | 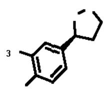 | (S)-3-(4-chloro-3-(trifluoromethyl)phenyl) isoxazolidine | 252.0 |
| 10 | 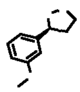 | (S)-3-(3-methoxyphenyl)isoxazolidine | 180.1 |
| 11 | 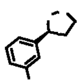 | (S)-3-(isoxazolidine-3-yl)benzonitrile | 175.1 |
| 12 | 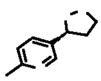 | (S)-3-(6-methylpyridin-3-yl)isoxazolidine | 165.1 |
| 13 | 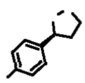 | (S)-3-(4-fluorophenyl)isoxazolidine | 168.1 |
| 14 | 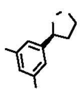 | (S)-3-(3-chloro-5-fluorophenyl)isoxazolidine | 202.0 |
| 15 | 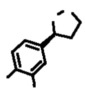 | (S)-3-(3,4-difluorophenyl)isoxazolidine | 186.1 |

(continued)

| Preparation Example | Structural Formula | Name of Compound | LC-MS [M+H]+ |
|---|---|---|---|
| 16 | 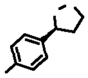 | (S)-3-(4-chlorophenyl)isoxazolidine | 184.1 |
| 17 | 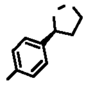 | (S)-4-(isoxazolidine-3-yl)benzonitrile | 175.1 |
| 18 | 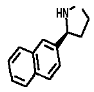 | (S)-3-(naphthalen-2-yl)isoxazolidine | 200.1 |
| 19 | 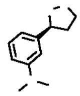 | (S)-3-(isoxazolidine-3-yl)-N,N-dimethylaniline | 193.1 |
| 20 | 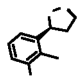 | (S)-3-(3-chloro-2-methylphenyl)isoxazolidine | 198.1 |
| 21 | 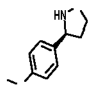 | (S)-3-(4-methoxyphenyl)isoxazolidine | 180.1 |
| 22 | 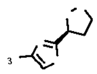 | (S)-3-(4-(trifluoromethyl)thiazol-2-yl)isoxazolidine | 225.0 |
| 23 | 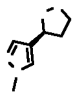 | (S)-3-(1-methyl-1H-pyrazol-4-yl)isoxazolidine | 154.1 |
| 24 | 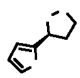 | (S)-3-(furan-2-yl)isoxazolidine | 140.1 |
| 25 | 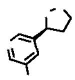 | (S)-3-(5-fluoropyridin-3-yl)isoxazolidine | 169.1 |
| 26 | 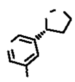 | (R)-3-(5-fluoropyridin-3-yl)isoxazolidine | 169.1 |
| 27 | 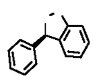 | (R)-3-phenyl-2,3-dihydrobenzo[d]isoxazole | 198.1 |
| 28 | 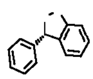 | (S)-3-phenyl-2,3-dihydrobenzo[d]isoxazole | 198.1 |
| 29 | 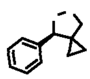 | (R)-7-phenyl-5-oxa-6-azaspiro[2.4]heptane | 176.1 |
| 30 | 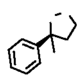 | (S)-3-methyl-3-phenylisoxazolidine | 164.1 |
| 31 | 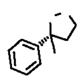 | (R)-3-methyl-3-phenylisoxazolidine | 164.1 |

(continued)

| Preparation Example | Structural Formula | Name of Compound | LC-MS [M+H]+ |
|---|---|---|---|
| 32 | 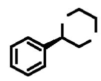 | (R)-3 -phenylmorpholine | 164.1 |
| 33 | 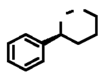 | (S)-3-phenyl-1,2-oxazinane | 164.1 |

**<Example 1> Preparation of (S)-N-(3-(2-acetamidoimidazo[1,2-b]pyrimidazin-6-yl)phenyl)-3-phenylisoxazolidine-2-carboxamide**

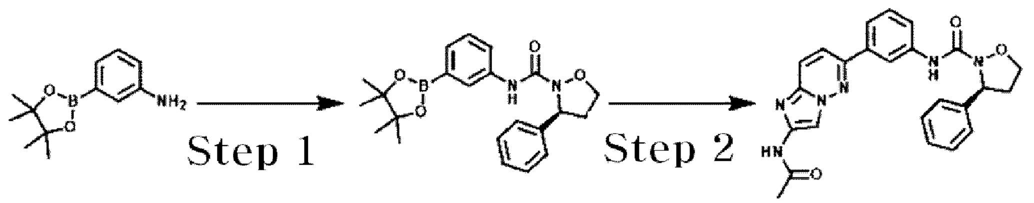


Step 1: Preparation of (S)-3-phenyl-N-(3-(4,4,5,5-tetramethyl-1,3,2-dioxaborolan-2-yl)phenyl)isoxazolidine-2-carboxamide

After dissolving 3-(4,4,5,5-tetramethyl-1,3,2-dioxaborolan-2-yl)aniline (3 g) and TEA (1.39 g, 1 eq) in DCM (70 mL), phenyl chloroformate (2.36 g, 1.1 eq) was added at 0 °C. The reaction mixture was stirred at 0 °C for 1 hour. Afterward, the (S)-3-phenylisoxazolidine obtained in Preparation Example 1 (2.45 g, 1.2 eq) and TEA (1.39 g, 1 eq) were added. After terminating the reaction by stirring at room temperature for 2 hours and adding MeOH (10 mL), an organic layer was extracted with water (200 mL*2), brine (200 mL), and DCM (200 mL*2). The organic layer was dried over sodium sulfate, concentrated under reduced pressure and purified by MPLC (EA/Hex), thereby obtaining the target compound (5.1g, yield: 94%).
MS (m/z): 395.2 [M+H]+

Step 2: Preparation of (S)-N-(3-(2-acetamidoimidazo[1,2-b]pyridazin-6-yl)phenyl)-3-phenylisoxazolidine-2-carboxamide

N-(6-iodoimidazo[1,2-b]pyridazin-2-yl)acetamide (66 mg), the (S)-3-phenyl-N-(3-(4,4,5,5-tetramethyl-1,3,2-dioxaborolan-2-yl)phenyl)isoxazolidine-2-carboxamide (95 mg, 1.1 eq) obtained in Step 1 of Example 1, KOAc (42 mg, 2 eq), and Pd(dtbpf)$Cl_2$ (14 mg, 0.1 eq) were dissolved in dioxane (0.6 mL) and water (0.15 mL) and stirred at 90 °C for 1 hour. The reaction mixture was filtered through diatomaceous earth and concentrated under reduced pressure. The target compound (32 mg, yield: 33%) was obtained through purification using the Prep-150 LC System.

**<Example 10> Preparation of (S)-N-(5-(2-(cyclopropanecarboxamido)imidazo [1,2-b] pyridazin-6-yl)-2-methylpyridin-3-yl)-3-phenylisoxazolidine-2-carboxamide**

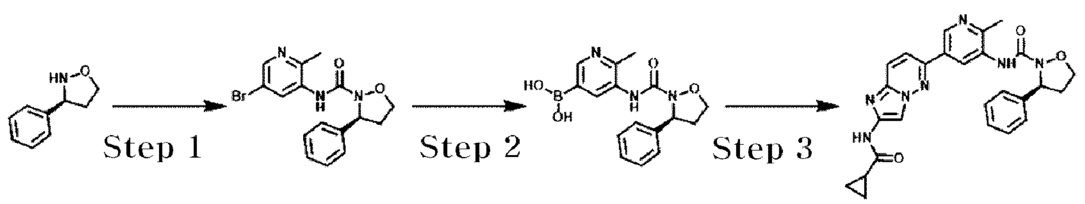

Step 1: Preparation of (S)-N-(5-bromo-2-methylpyridin-3-yl)-3-phenylisoxazolidine-2-carboxamide

After dissolving 5-bromo-2-methylpyridine-3-amine (6 g) and pyridine (1.39 g, 1 eq) in acetonitrile (ACN) (70 mL), phenyl chloroformate (5.27g, 1.05 eq) was added at 0 °C. The reaction mixture was stirred at 0 °C for 1 hour. Afterward, the (S)-3-phenylisoxazolidine (5.03 g, 1.05 eq) obtained in Preparation Example 1 and TEA (3.57 g, 1.1 eq) were added. After terminating the reaction by stirring at room temperature for 6 hours and adding MeOH (20 mL), an organic layer was extracted using water (300 mL*2), brine (300 mL), and DCM (400 mL*2). The organic layer was dried over sodium sulfate, concentrated under reduced pressure, and purified by MPLC (EA/Hex), thereby obtaining the target compound (10.9 g, yield: 94%).

MS (m/z): 362.0 $[M+H]^+$

Step 2: Preparation of (S)-(6-methyl-5-(3-phenylisoxazolidin-2-carboxamido)pyridin-3-yl)boronic acid

The (S)-N-(5-bromo-2-methylpyridin-3-yl)-3-phenylisoxazolidine-2-carboxamide (1 g) obtained in Step 1 of Example 10, bis(pinacolato)diborone (0.84 g 1.2 eq), potassium trimethylacetate (0.78 g, 2 eq), and bis(triphenylphosphine)palladium (II) chloride (0.2 g, 0.1 eq) were dissolved in 2-methyl-tetrahydrofuran (2-MeTHF, 15 mL) and stirred at 90 °C for 2 hours. The reaction mixture was filtered through diatomaceous earth and concentrated under reduced pressure, and then the resulting product was used in the next reaction without purification.

MS (m/z): 328.1 $[M+H]^+$

Step 3: Preparation of (S)-N-(5-(2-(cyclopropanecarboxamido)imidazo[1,2-b]pyridazin-6-yl)-2-methylpyridin-3-yl)-3-phenylisoxazolidine-2-carboxamide

N-(6-iodoimidazo[1,2-b]pyridazin-2-yl)cyclopropanecarboxamide (25 mg), the (S)-(6-methyl-5-(3-phenylisoxazolidin-2-carboxamido)pyridin-3-yl)boronic acid (75 mg, 3 eq) obtained in Step 2 of Example 10, sodium carbonate (16 mg, 2 eq), and tetrakis(triphenylphosphine)palladium (0) (9 mg, 0.1 eq) were dissolved in 1,4-dioxane (0.3 mL) and water (0.1 mL) and stirred at 90 °C for 2 hours. The reaction mixture was filtered through diatomaceous earth and concentrated under reduced pressure. The target compound (10 mg, yield: 26%) was obtained through purification using the Prep-150 LC System.

Examples 1 to 158 were prepared in a similar manner to Examples 1 and 10 using Preparation Examples 1 to 33, and the names, chemical structure formulas, and NMR and LC-MS analysis results for the compounds of Examples 1 to 158 are summarized in Table 2 below.

[Table 2]

| Example | Structure | Name of Compound | $^1H$ NMR | LC-MS $[M+H]^+$ |
|---|---|---|---|---|
| 1 | 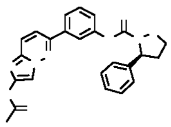 | (S)-N-(3-(2-acetamidoimidazo[1,2-b]pyridazin-6-yl) phenyl)-3-phenylisoxazolidine-2-caiboxamide | $^1H$ NMR (400 MHz, DMSO-$d_6$) $\delta$ = 10.92 (s, 1H), 9.60 (s, 1H), 8.37 (t, *J* = 1.9 Hz, 1H), 8.28 (d, *J* = 0.7 Hz, 1H), 8.07 (dd, *J* = 9.4, 0.7 Hz, 1H), 7.82 (ddd, *J* = 8.2, 2.2, 1.0 Hz, 1H), 7.72 - 7.65 (m, 2H), 7.46 (t, *J* = 8.0 Hz, 1H), 7.43 - 7.35 (m, 4H), 7.28 (tt, *J* = 6.2, 1.7 Hz, 1H), 5.44 (dd, *J* = 8.6, 5.9 Hz, 1H), 428 (td, *J* = 8.1, 3.1 Hz, 1H), 3.92 (ddd, *J* = 9.5, 8.1, 7.0 Hz, 1H), 2.87 (tdd, *J* = 8.7, 7.0, 3.1 Hz, 1H), 2.28 - 2.19 (m, 1H), 2.11 (s, 3H) | 443.3 |

(continued)

| Example | Structure | Name of Compound | $^1$H NMR | LC-MS $[M+H]^+$ |
|---|---|---|---|---|
| 2 | 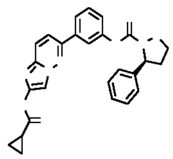 | (S)-N-(3-(2-(cyclopropanecarboxamido)imidazo [1,2-b]pyridazin-6-yl)phenyl)-3-phenylisoxazolidine-2-caiboxamide | $^1$H NMR (400 MHz, DMSO-$d_6$) $\delta$ = 11.20 (s, 1H), 9.60 (s, 1H), 835 (d, $J$ = 2.0 Hz, 1H), 826 (s, 1H), 8.07 (d, $J$ = 9.5 Hz, 1H), 7.85 - 7.80 (m, 1H), 7.71 - 7.65 (m, 2H), 7.45 (t, $J$ = 7.9 Hz, 1H), 7.42 - 7.35 (m, 4H), 7.30 - 7.25 (m, 1H), 5.44 (dd, $J$ = 8.6, 5.9 Hz, 1H), 427 (td, $J$ = 8.0, 3.0 Hz, 1H), 3.92 (dt, $J$ = 9.2, 7.3 Hz, 1H), 2.87 (tdd, $J$ = 9.7, 7.7, 2.9 Hz, 1H), 2.29 - 220 (m, 1H), 1.97 (dq, $J$ = 7.7, 4.9, 3.9 Hz, 1H), 0.85 (ddd, $J$ = 9.5, 6.2, 2.5 Hz, 4H) | 469.3 |

(continued)

| Example | Structure | Name of Compound | $^1$H NMR | LC-MS $[M+H]^+$ |
|---|---|---|---|---|
| 3 | 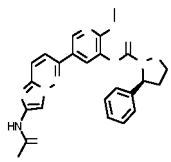 | (S)-N-(5-(2-acetamidoimidazo[1,2-b]pyridazin-6-yl)-2-methoxypyridin-3-yl)-3-phenylisoxazolidine-2-caiboxamide | $^1$H NMR (400 MHz, DMSO-$d_6$) $\delta$ = 10.90 (s, 1H), 8.92 (d, *J* = 22 Hz, 1H), 8.56 (d, *J* = 23 Hz, 1H), 8.52 (s, 1H), 827 (s, 1H), 8.05 (d, *J* = 9.4 Hz, 1H), 7.76 (d, *J* = 9.5 Hz, 1H), 7.41-7.37 (m, 4H), 7.31-7.26 (m, 1H), 5.42 (dd, *J* = 8.5, 6.1 Hz, 1H), 4.33 (td, *J* = 7.8, 3.0 Hz, 1H), 4.05 (s, 3H), 4.01 - 3.96 (m, 1H), 2.92 (ddd, *J* = 8.7, 5.3, 2.5 Hz, 1H), 2.31-224 (m, 1H), 2.11 (s, 3H) | 474.3 |

(continued)

| Example | Structure | Name of Compound | $^1$H NMR | LC-MS $[M+H]^+$ |
|---|---|---|---|---|
| 4 | 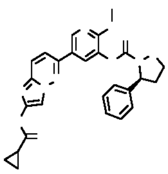 | (S)-N-(5-(2-(cyclopropanecarboxamido)imidazo [1,2-b]pyridazin-6-yl)-2-methoxypyridin-3-yl)-3-phenylisoxazolidine-2-caiboxamide | $^1$H NMR (400 MHz, DMSO-$d_6$) δ = 11.18 (s, 1H), 8.91 (d, *J* = 23 Hz, 1H), 8.56 (d, *J* = 23 Hz, 1H), 8.52 (s, 1H), 824 (s, 1H), 8.05 (d, *J* = 9.5 Hz, 1H), 7.76 (d, *J* = 9.5 Hz, 1H), 7.43 -7.35 (m, 4H), 7.31-7.25 (m, 1H), 5.41 (dd, *J* = 8.5, 6.2 Hz, 1H), 4.32 (td, *J* = 7.8, 3.0 Hz, 1H), 4.05 (s, 3H), 4.00 (dt, *J* = 9.4, 7.7 Hz, 1H), 2.92 (dddd, *J* = 11.9, 9.3, 6.7, 3.0 Hz, 1H), 2.28 (dq, *J* = 12.2, 7.6 Hz, 1H), 1.96 (td, *J* = 7.6, 4.2 Hz, 1H), 0.84 (tt, *J* = 8.2, 3.0 Hz, 4H) | 500.4 |

(continued)

| Example | Structure | Name of Compound | $^1$H NMR | LC-MS $[M+H]^+$ |
|---|---|---|---|---|
| 5 | 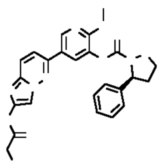 | (S)-N-(5-(2-(2-hydroxyacetamido)imidazo[1,2-b] pyridazin-6-yl)-2-methoxypyridin-3-yl)-3-phenylisoxazolidine-2-caiboxamide | $^1$H NMR (400 MHz, DMSO-$d_6$) δ = 10.34 (s, 1H), 8.93 (d, *J* = 22 Hz, 1H), 8.56 (d, *J* = 23 Hz, 1H), 8.51 (s, 1H), 832 (s, 1H), 8.07 (d, *J* = 9.4 Hz, 1H), 7.77 (d, *J* = 9.5 Hz, 1H), 7.43 - 7.35 (m, 4H), 7.32 - 726 (m, 1H), 5.55 (s, 1H), 5.42 (dd, *J* = 8.5, 6.2 Hz, 1H), 4.33 (td, *J* = 7.8, 3.0 Hz, 1H), 4.14 - 4.08 (m, 2H), 4.05 (s, 3H), 4.04 - 3.97 (m, 1H), 2.92 (tdt, *J* = 9.3, 7.0, 3.0 Hz, 1H), 2.28 (dddd, *J* = 12.1, 9.6, 7.6, 6.0 Hz, 1H) | 490.3 |

(continued)

| Example | Structure | Name of Compound | $^1H$ NMR | LC-MS $[M+H]^+$ |
|---|---|---|---|---|
| 6 | 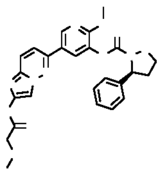 | (S)-N-(2-methoxy-5-(2-(2-methoxyacetamido) imidazo [1,2-b]pyridazin-6-yl)pyridin-3-yl)-3-phenylisoxazolidine-2-caiboxamide | $^1H$ NMR (400 MHz, DMSO-$d_6$) δ = 10.69 (s, 1H), 8.93 (d, *J* = 22 Hz, 1H), 8.57 (d, *J* = 23 Hz, 1H), 8.52 (s, 1H), 832 (s, 1H), 8.07 (d, *J* = 9.5 Hz, 1H), 7.78 (d, *J* = 9.5 Hz, 1H), 7.43 - 7.35 (m, 4H), 7.31-7.26 (m, 1H), 5.42 (dd, *J* = 8.5, 6.2 Hz, 1H), 4.33 (td, *J* = 7.8, 3.0 Hz, 1H), 4.10 (s, 2H), 4.06 (s, 3H), 4.04 - 3.96 (m, 1H), 3.37 (s, 3H), 2.92 (tq, *J* = 8.7, 3.0 Hz, 1H), 228 (ddt, *J* = 12.3, 9.8, 7.4 Hz, 1H) | 504.3 |

(continued)

| Example | Structure | Name of Compound | $^1$H NMR | LC-MS $[M+H]^+$ |
|---|---|---|---|---|
| 7 | 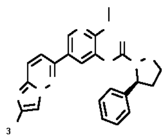 | (S)-N-(5-(2-aminoimidazo[1,2-b]pyridazin-6-yl)-2-methoxypyridin-3-yl)-3-phenylisoxazolidine-2-caiboxamide | $^1$H NMR (400 MHz, DMSO-$d_6$) δ = 8.88 (d, *J* = 2.2 Hz, 1H), 8.49 (d, *J* = 2.0 Hz, 2H), 7.75 (d, *J* = 9.2 Hz, 1H), 7.55 (d, *J* = 9.3 Hz, 1H), 7.41 - 7.36 (m, 5H), 7.29 (td, *J* = 6.2, 3.0 Hz, 1H), 5.41 (dd, *J* = 8.5, 6.2 Hz, 1H), 4.32 (td, *J* = 7.8, 3.0 Hz, 1H), 4.04 (s, 3H), 4.02 - 3.96 (m, 1H), 2.92 (tdt, *J* = 9.5, 7.1, 3.0 Hz, 1H), 2.32 - 2.22 (m, 1H) | 432.3 |

(continued)

| Example | Structure | Name of Compound | $^1$H NMR | LC-MS $[M+H]^+$ |
|---|---|---|---|---|
| 8 | 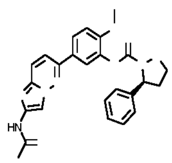 | (S)-N-(5-(2-acetamidoimidazo[1,2-b]pyridazin-6-yl)-2-methoxyphenyl)-3-phenylisoxazolidine-2-caiboxamide | $^1$H NMR (400 MHz, DMSO-$d_6$) δ = 10.88 (s, 1H), 8.72 (d, *J* = 2.3 Hz, 1H), 8.59 (s, 1H), 8.24 (s, 1H), 8.01 (d, *J* = 9.5 Hz, 1H), 7.77 (dd, *J* = 8.6, 2.3 Hz, 1H), 7.67 (d, *J* = 9.5 Hz, 1H), 7.43 - 735 (m, 4H), 7.31 - 7.26 (m, 1H), 7.23 (d, *J* = 8.7 Hz, 1H), 5.43 (dd, *J* = 8.5, 6.0 Hz, 1H), 4.31 (td, *J* = 7.8, 3.1 Hz, 1H), 4.01 - 3.92 (m, 4H), 2.90 (dtt, *J* = 9.6, 6.9, 3.1 Hz, 1H), 2.27 (dddd, *J* = 12.3, 9.6, 7.8, 6.1 Hz, 1H), 2.10 (s, 3H) | 473.3 |

(continued)

| Example | Structure | Name of Compound | $^1H$ NMR | LC-MS $[M+H]^+$ |
|---|---|---|---|---|
| 9 | 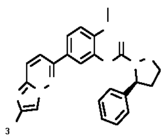 | (S)-N-(5-(2-aminoimidazo[1,2-b]pyridazin-6-yl)-2-methoxyphenyl)-3-phenylisoxazolidine-2-caiboxamide | $^1H$ NMR (400 MHz, DMSO-$d_6$) $\delta$ = 8.68 (d, $J$ = 2.2 Hz, 1H), 8.57 (s, 1H), 7.78 (d, $J$ = 9.3 Hz, 1H), 7.70 (dd, $J$ = 8.6, 2.3 Hz, 1H), 7.54 (d, $J$ = 9.3 Hz, 1H), 7.41 - 7.36 (m, 5H), 7.30 - 7.26 (m, 1H), 7.21 (d, $J$ = 8.7 Hz, 1H), 5.41 (dd, $J$ = 8.5, 6.1 Hz, 1H), 431 (td, $J$ = 7.8, 3.1 Hz, 1H), 3.96 (s, 4H), 2.90 (dddd, $J$ = 12.0, 9.4, 6.9, 3.1 Hz, 1H), 2.26 (dddd, $J$ = 9.5, 6.2, 4.0, 1.7 Hz, 1H) | 431.3 |

(continued)

| Example | Structure | Name of Compound | $^1$H NMR | LC-MS $[M+H]^+$ |
|---|---|---|---|---|
| 10 | 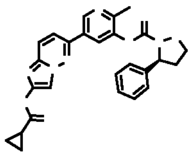 | (S)-N-(5-(2-(cyclopropanecarboxamido)imidazo [1,2-b]pyridazin-6-yl)-2-methylpyridin-3-yl)-3-phenylisoxazolidine-2-caiboxamide | $^1$H NMR (400 MHz, DMSO-$d_6$) δ 11.21 (s, 1H), 9.23 (s, 1H), 8.93 (t, *J* = 1.5 Hz, 1H), 8.38 (d, *J* = 2.0 Hz, 1H), 8.28 (s, 1H), 8.09 (d, *J* = 9.4 Hz, 1H), 7.82 (d, *J* = 9.5 Hz, 1H), 7.41 - 736 (m, 4H), 7.28 (s, 1H), 5.42 (t, *J* = 7.3 Hz, 2H), 4.30 (d, *J* = 8.2 Hz, 2H), 4.02 (q, *J* = 7.9 Hz, 2H), 2.94 - 2.86 (m, 2H), 2.47 (s, 1H), 2.27 (s, 2H), 1.97 (s, 2H) | 484.2 |

(continued)

| Example | Structure | Name of Compound | $^1H$ NMR | LC-MS $[M+H]^+$ |
|---|---|---|---|---|
| 11 | 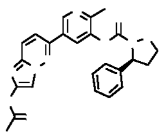 | (S)-N-(5-(2-acetamidoimidazo[1,2-b]pyridazin-6-yl)-2-methylpyridin-3-yl)-3-phenylisoxazolidine-2-caiboxamide | $^1H$ NMR (400 MHz, DMSO-$d_6$) $\delta$ 10.93 (s, 1H), 9.22 (s, 1H), 8.94 (d, $J$ = 2.2 Hz, 1H), 8.40 (d, $J$ = 2.1 Hz, 1H), 8.31 (s, 1H), 8.09 (d, $J$ = 9.4 Hz, 1H), 7.82 (d, $J$ = 9.5 Hz, 1H), 7.42 - 737 (m, 4H), 729 (d, $J$ = 7.0 Hz, 1H), 5.41 (d, $J$ = 6.9 Hz, 1H), 431 (dd, $J$ = 9.3, 6.5 Hz, 1H), 4.02 (d, $J$ = 83 Hz, 1H), 2.90 (s, 1H), 2.47 (s, 1H), 2.29 - 2.25 (m, 1H), 2.17 (s, 1H), 2.11 (s, 3H), 2.06 (s, 1H) | 458.2 |

(continued)

| Example | Structure | Name of Compound | $^1$H NMR | LC-MS $[M+H]^+$ |
|---|---|---|---|---|
| 12 | 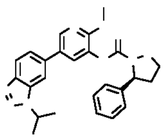 | (S)-N-(5-(1-isopropyl-1H-benzo[d][1,2,3]triazol-6-yl)-2-methoxypyridin-3-yl)-3-phenylisoxazolidine-2-caiboxamide | $^1$H NMR (400 MHz, DMSO-$d_6$) δ = 8.65 (d, *J* = 2.3 Hz, 1H), 8.52 (s, 1H), 833 (d, *J* = 2.3 Hz, 1H), 8.13 (s, 1H), 8.11 (d, *J* = 8.8 Hz, 1H), 7.64 (dd, *J* = 8.6, 1.5 Hz, 1H), 7.40 - 7.35 (m, 4H), 7.30 - 7.26 (m, 1H), 5.40 (dd, *J* = 8.5, 6.2 Hz, 1H), 531 (p, *J* = 6.7 Hz, 1H), 4.33 (td, *J* = 7.8, 2.9 Hz, 1H), 4.04 (s, 3H), 4.02 - 3.95 (m, 1H), 2.91 (dddd, *J* = 11.9, 9.3, 6.8, 3.0 Hz, 1H), 2.32 - 2.22 (m, 1H), 1.65 (d, *J* = 6.7 Hz, 6H) | 459.4 |

(continued)

| Example | Structure | Name of Compound | $^1$H NMR | LC-MS $[M+H]^+$ |
|---|---|---|---|---|
| 13 | 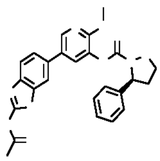 | (S)-N-(5-(2-acetamidobenzo[d]oxazol-6-yl)-2-methoxypyridin-3-yl)-3-phenylisoxazolidine-2-caiboxamide | $^1$H NMR (400 MHz, DMSO-$d_6$) δ = 11.70 (s, 1H), 8.57 (d, *J* = 2.3 Hz, 1H), 8.48 (s, 1H), 8.24 (d, *J* = 2.3 Hz, 1H), 7.88 (d, *J* = 1.7 Hz, 1H), 7.63 (d, *J* = 8.2 Hz, 1H), 7.53 (dd, *J* = 8.2, 1.7 Hz, 1H), 7.42 - 7.34 (m, 4H), 7.31 - 7.25 (m, 1H), 5.40 (dd, *J* = 8.5, 6.2 Hz, 1H), 4.32 (td, *J* = 7.8, 3.0 Hz, 1H), 4.02 (s, 3H), 4.00 - 3.92 (m, 1H), 2.91 (dddd, *J* = 11.8, 9.1, 6.7, 3.0 Hz, 1H), 2.32 - 2.25 (m, 1H), 2.23 (s, 3H) | 474.3 |

(continued)

| Example | Structure | Name of Compound | $^1$H NMR | LC-MS $[M+H]^+$ |
|---|---|---|---|---|
| 14 | 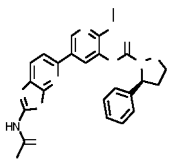 | (S)-N-(5-(2-acetamidothiazolo[5,4-b]pyridin-5-yl)-2-methoxypyridin-3-yl)-3-phenylisoxazolidine-2-caiboxamide | $^1$H NMR (400 MHz, DMSO-$d_6$) $\delta$ = 12.49 (s, 1H), 9.00 (d, $J$ = 22 Hz, 1H), 8.60 (d, $J$ = 22 Hz, 1H), 8.49 (s, 1H), 8.14 (d, $J$ = 8.6 Hz, 1H), 8.01 (d, $J$ = 8.5 Hz, 1H), 7.39 (td, $J$ = 8.4, 6.2 Hz, 4H), 7.31-7.26 (m, 1H), 5.42 (dd, $J$ = 8.5, 6.2 Hz, 1H), 4.32 (td, $J$ = 7.8, 3.0 Hz, 1H), 4.04 (s, 3H), 4.03 - 3.96 (m, 1H), 2.92 (tdt, $J$ = 9.4, 6.9, 3.1 Hz, 1H), 228 (ddt, $J$ = 11.5, 5.9, 3.9 Hz, 1H), 223 (s, 3H) | 491.3 |

(continued)

| Example | Structure | Name of Compound | $^1H$ NMR | LC-MS $[M+H]^+$ |
|---|---|---|---|---|
| 15 | 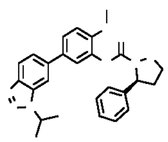 | (S)-N-(5-(1-isopropyl-1H-benzo[d][1,2,3]triazol-6-yl)-2-methoxyphenyl)-3-phenylisoxazolidine-2-carboxamide | $^1H$ NMR (400 MHz, DMSO-$d_6$) $\delta$ = 8.59 (s, 1H), 8.44 (d, *J* = 2.4 Hz, 1H), 8.07 (d, *J* = 8.7 Hz, 1H), 8.02 (t, *J* = 1.1 Hz, 1H), 7.61 (dd, *J* = 8.7, 1.5 Hz, 1H), 7.51 (dd, *J* = 8.5, 2.4 Hz, 1H), 7.40 - 7.36 (m, 4H), 7.30 - 7.25 (m, 1H), 722 (d, *J* = 8.4 Hz, 1H), 5.42 (dd, *J* = 8.5, 6.1 Hz, 1H), 5.30 (dt, *J* = 13.6, 6.8 Hz, 1H), 4.32 (td, *J* = 7.9, 3.0 Hz, 1H), 3.97 - 3.94 (m, 4H), 2.90 (ddq, *J* = 11.9, 7.0, 3.1 Hz, 1H), 2.31 - 2.22 (m, 1H), 1.65 (dd, *J* = 6.7, 1.2 Hz, 6H) | 458.3 |

(continued)

| Example | Structure | Name of Compound | $^1$H NMR | LC-MS $[M+H]^+$ |
|---|---|---|---|---|
| 16 | 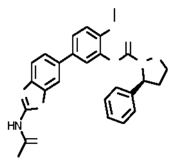 | (S)-N-(5-(2-acetamidobenzo[d]oxazol-6-yl)-2-methoxyphenyl)-3-phenylisoxazolidine-2-carboxamide | $^1$H NMR (400 MHz, DMSO-$d_6$) $\delta$ = 11.67 (s, 1H), 8.56 (s, 1H), 8.36 (d, $J$ = 2.3 Hz, 1H), 7.78 (d, $J$ = 1.7 Hz, 1H), 7.60 (d, $J$ = 8.3 Hz, 1H), 7.51 (dd, $J$ = 8.3, 1.7 Hz, 1H), 7.43 - 7.35 (m, 5H), 7.30 - 7.25 (m, 1H), 7.17 (d, $J$ = 8.6 Hz, 1H), 5.41 (dd, $J$ = 8.5, 6.0 Hz, 1H), 4.31 (td, $J$ = 7.8, 3.1 Hz, 1H), 4.00 - 3.94 (m, 1H), 3.94 (s, 3H), 2.94 - 2.85 (m, 1H), 2.31 - 2.24 (m, 1H), 2.23 (s, 3H) | 473.3 |

(continued)

| Example | Structure | Name of Compound | $^1H$ NMR | LC-MS $[M+H]^+$ |
|---|---|---|---|---|
| 17 | 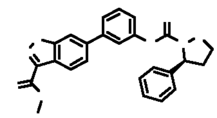 | (S)-N-(3-(3-(methylcarbamoyl)-1H-indazol-6-yl) phenyl)-3-phenylisoxazolidine-2-carboxamide | $^1H$ NMR (400 MHz, DMSO-$d_6$) δ 13.58 (s, 1H), 9.49 (s, 1H), 832 (d, *J* = 4.7 Hz, 1H), 8.19 (d, *J* = 8.5 Hz, 1H), 8.04 (s, 1H), 7.71 (s, 1H), 7.67 (d, *J* = 3.4 Hz, 1H), 7.48 (d, *J* = 8.5 Hz, 1H), 7.39 - 729 (m, 6H), 723 (t, *J* = 6.9 Hz, 1H), 5.40 (dd, *J* = 8.3, 6.0 Hz, 1H), 4.23 (dd, *J* = 7.9, 5.0 Hz, 1H), 3.88 (dd, *J* = 16.7, 7.8 Hz, 1H), 2.87 - 2.81 (m, 1H), 2.79 (d, *J* = 4.6 Hz, 3H), 2.25 - 2.11 (m, 1H) | 442.3 |

(continued)

| Example | Structure | Name of Compound | $^1H$ NMR | LC-MS $[M+H]^+$ |
|---|---|---|---|---|
| 18 | 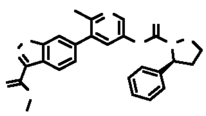 | (S)-N-(6-methyl-5-(3-(methylcaibamoyl)-lH-indazol-6-yl)pyridin-3-yl)-3-phenylisoxazolidine-2-caiboxamide | $^1H$ NMR (400 MHz, CDC13) δ = 10.21 (s, 1H), 8.48 (d, *J* = 2.6 Hz, 1H), 8.43 (dd, *J* = 8.4, 0.8 Hz, 1H), 8.07 (d, *J* = 2.6 Hz, 1H), 7.93 (s, 1H), 7.40 (dd, *J* = 6.7, 1.2 Hz, 2H), 7.35 (td, *J* = 6.8, 6.3, 1.7 Hz, 2H), 7.29 -7.27 (m, 1H), 7.25 (d, *J* = 2.4 Hz, 2H), 7.07 - 7.02 (m, 1H), 5.50 (dd, *J* = 8.5, 5.9 Hz, 1H), 4.29 (td, *J* = 7.8, 3.6 Hz, 1H), 4.03 - 3.96 (m, 1H), 3.08 (d, *J* = 5.1 Hz, 3H), 2.90 - 2.81 (m, 1H), 2.47 (s, 4H) | 457.3 |

(continued)

| Example | Structure | Name of Compound | $^1$H NMR | LC-MS $[M+H]^+$ |
|---|---|---|---|---|
| 19 | 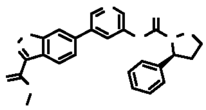 | (S)-N-(5-(3-(methylcaibamoyl)-1H-indazol-6-yl) pyridin-3-yl)-3-phenylisoxazolidine-2-carboxamide | $^1$H NMR (400 MHz, CDC13) δ = 10.64 (s, 1H), 8.61 (s, 1H), 8.54 (s, 1H), 8.48 - 8.42 (m, 2H), 8.05 (s, 1H), 7.64 (s, 1H), 7.52 (dd, *J* = 8.4, 1.4 Hz, 1H), 7.44 - 7.40 (m, 2H), 7.37 (ddd, *J* = 7.8, 6.8, 1.3 Hz, 2H), 7.31 - 7.27 (m, 1H), 7.06 (d, *J* = 5.5 Hz, 1H), 5.53 (dd, *J* = 8.5, 6.0 Hz, 1H), 4.31 (td, *J* = 7.8, 3.5 Hz, 1H), 4.01 (ddd, *J* = 9.2, 7.9, 6.9 Hz, 1H), 3.07 (d, *J* = 5.0 Hz, 3H), 2.93 - 2.84 (m, 1H), 2.50 - 2.43 (m, 1H) | 443.3 |

(continued)

| Example | Structure | Name of Compound | $^1$H NMR | LC-MS [M+H]$^+$ |
|---|---|---|---|---|
| 20 | 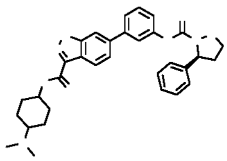 | (S)-N-(3-(3-((4-(dimethylamino)cyclohexyl) carbamoyl)-1H-indazol-6-yl)phenyl)-3-phenylisoxazolidine-2-carboxamide | $^1$H NMR (400 MHz, CDC13) δ = 10.31 (s, 1H), 8.41 ( dd, *J* = 8.5, 0.8 Hz, 1H), 8.01 (s, 1H), 7.93 (d, *J* = 2 0 Hz, 1H), 7.65 (t, *J* = 1.1 Hz, 1H), 7.55 (dd, *J* = 8. 5, 1.5 Hz, 1H), 7.45 - 7.34 (m, 7H), 7.30 - 7.27 (m, 1H), 6.89 (d, *J* = 8.3 Hz, 1H), 5.57 (dd, *J* = 8.5, 5.7 Hz, 1H), 4.27 (dt, *J* = 7.8, 3.9 Hz, 1H), 3.99 (ddd, *J* = 9.0, 7.9, 7.0 Hz, 2H), 2.90 - 2.81 (m, 1H), 2.49 - 2.40 (m, 1H), 2.34 (s, 6H), 2.24 (s, 2H), 2.17 (s, 5H), 2.00 (d, *J* = 12.7 Hz, 2H) | 553.7 |

(continued)

| Example | Structure | Name of Compound | $^1$H NMR | LC-MS [M+H]$^+$ |
|---|---|---|---|---|
| 21 | 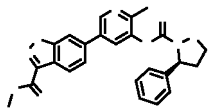 | (S)-N-(2-methyl-5-(3-(methylcaibamoyl)-lH-indazol-6-yl)pyridin-3-yl)-3-phenylisoxazolidine-2-caiboxamide | $^1$H NMR (400 MHz, CDC13) δ = 10.48 (s, 1H), 8.66 ( d, *J* = 2.1 Hz, 1H), 8.55 (d, *J* = 2.1 Hz, 1H), 8.45 (d d, *J* = 8.5, 0.8 Hz, 1H), 7.92 (s, 1H), 7.66 (t, *J* = 1.1 Hz, 1H), 7.54 (dd, *J* = 8.5, 1.5 Hz, 1H), 7.46 - 7.41 (m, 2H), 7.40 - 7.34 (m, 2H), 7.32 - 7.28 (m, 1H), 7. 04 (d, *J* = 5.3 Hz, 1H), 5.55 (dd, *J* = 8.5, 5.9 Hz, 1H ), 4.34 (td, *J* = 7.8, 3.6 Hz, 1H), 4.09 - 4.00 (m, 1H) , 3.07 (d, *J* = 5.1 Hz, 3H), 2.95 - 2.85 (m, 1H), 2.61 (s, 3H), 2.48 (dddd, *J* = 13.1, 9.1, 7.7, 5.9 Hz, 1H) | 457.3 |

(continued)

| Example | Structure | Name of Compound | $^1$H NMR | LC-MS $[M+H]^+$ |
|---|---|---|---|---|
| 22 | 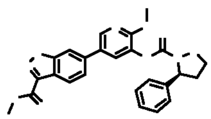 | (S)-N-(2-methoxy-5-(3-(methylcaibamoyl)-1H-indazol-6-yl)pyridin-3-yl)-3-phenylisoxazolidine-2-caiboxamide | $^1$H NMR (400 MHz, DMSO) $\delta$ = 13.60 (s, 1H), 8.64 (d, *J* = 2.3 Hz, 1H), 8.49 (s, 1H), 8.36 (q, *J* = 4.6 H z, 1H), 8.28 (d, *J* = 2.3 Hz, 1H), 8.23 (dd, *J* = 8.5, 0 8 Hz, 1H), 7.74 (t, *J* = 1.2 Hz, 1H), 7.49 (dd, *J* = 8. 5, 1.5 Hz, 1H), 7.42 - 7.34 (m, 4H), 7.28 (ddt, *J* = 8. | 473.3 |
| | | | 5, 6.0, 1.9 Hz, 1H), 5.41 (dd, *J* = 8.5, 6.2 Hz, 1H), 4. 33 (td, *J* = 7.8, 3.1 Hz, 1H), 4.04 (s, 3H), 4.02 - 3.9 5 (m, 1H), 2.95 - 2.86 (m, 1H), 2.83 (d, *J* = 4.7 Hz, 3H), 2.31 - 2.24 (m, 1H) | |

(continued)

| Example | Structure | Name of Compound | $^1$H NMR | LC-MS [M+H]$^+$ |
|---|---|---|---|---|
| 23 | 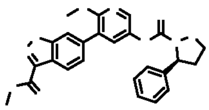 | (S)-N-(6-methoxy-5-(3-(methylcaibamoyl)-lH-indazol-6-yl)pyridin-3-yl)-3-phenylisoxazolidine-2-caiboxamide | $^1$H NMR (400 MHz, DMSO) δ = 9.57 (s, 1H), 8.44 ( d, *J* = 2.6 Hz, 1H), 8.37 (q, *J* = 4.7 Hz, 1H), 820 (d d, *J* = 8.5, 0.8 Hz, 1H), 8.11 (d, *J* = 2.6 Hz, 1H), 7.7 6 (t, *J* = 1.2 Hz, 1H), 7.42 - 7.32 (m, 5H), 7.30 - 7. 23 (m, 1H), 5.41 (dd, *J* = 8.6, 5.8 Hz, 1H), 4.27 (td, *J* = 7.9, 3.1 Hz, 1H), 4.03 (q, *J* = 7.1 Hz, 1H), 3.95 - 3.89 (m, 1H), 3.88 (s, 3H), 2.91 - 2.85 (m, 1H), 2. 83 (d, *J* = 4.7 Hz, 3H), 2.27 - 220 (m, 1H) | 473.3 |

(continued)

| Example | Structure | Name of Compound | $^1$H NMR | LC-MS $[M+H]^+$ |
|---|---|---|---|---|
| 24 | 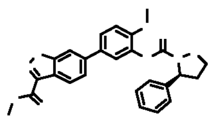 | (S)-N-(2-methoxy-5-(3-(methylcaibamoyl)-1H-indazol-6-yl)phenyl)-3-phenylisoxazolidine-2-caiboxamide | $^1$H NMR (400 MHz, DMSO-$d_6$) $\delta$ = 13.51 (s, 1H), 8.56 (s, 1H), 8.44 (d, *J* = 2.3 Hz, 1H), 8.34 (q, *J* = 4.6 Hz, 1H), 8.19 (dd, *J* = 8.5, 0.8 Hz, 1H), 7.68 (t, *J* = 1.1 Hz, 1H), 7.47 (ddd, *J* = 10.7, 8.5, 1.9 Hz, 2H), 7.42 - 7.34 (m, 4H), 7.30 - 7.25 (m, 1H), 7.19 (d, *J* = 8.6 Hz, 1H), 5.42 (dd, *J* = 8.5, 6.0 Hz, 1H), 4.31 (td, *J* = 7.8, 3.1 Hz, 1H), 4.01 - 3.95 (m, 1H), 3.95 (s, 3H), 2.94 - 2.85 (m, 1H), 2.82 (d, *J* = 4.7 Hz, 3H), 2.27 (ddt, *J* = 12.1, 9.5, 6.7 Hz, 1H) | 472.3 |

(continued)

| Example | Structure | Name of Compound | $^1$H NMR | LC-MS $[M+H]^+$ |
|---|---|---|---|---|
| 25 | 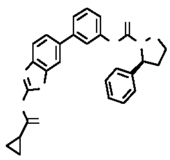 | (S)-N-(3-(2-(cyclopropanecarboxamido)benzo[d]thiazol-6-yl)phenyl)-3-phenylisoxazolidine-2-carboxamide | $^1$H NMR (400 MHz, DMSO) δ = 12.67 (s, 1H), 9.48 (s, 1H), 8.21 (d, *J* = 1.9 Hz, 1H), 8.06 - 8.02 (m, 1H), 7.79 (d, *J* = 8.4 Hz, 1H), 7.68 (ddd, *J* = 7.9, 5.3, 2.0 Hz, 2H), 7.40 - 7.36 (m, 5H), 7.30 - 7.24 (m, 2H), 5.43 (dd, *J* = 8.7, 5.9 Hz, 1H), 4.27 (td, *J* = 7.9, 3.0 Hz, 1H), 3.95 - 3.87 (m, 1H), 2.87 (ddt, *J* = 12.4, 6.1, 3.4 Hz, 1H), 2.28 - 220 (m, 1H), 2.03 - 1.98 (m, 1H), 0.98 - 0.93 (m, 4H) | 485.3 |

(continued)

| Example | Structure | Name of Compound | $^1$H NMR | LC-MS $[M+H]^+$ |
|---|---|---|---|---|
| 26 | 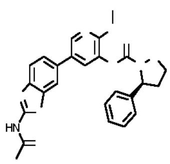 | (S)-N-(5 -(2-acetamidobenzo [d]thiazol-6-yl)-2-methoxypyridin-3-yl)-3-phenylisoxazolidine-2-caiboxamide | $^1$H NMR (400 MHz, DMSO) δ = 12.36 (s, 1H), 8.60 (d, *J* = 2.3 Hz, 1H), 8.48 (s, 1H), 823 (dd, *J* = 3.0, 2.0 Hz, 2H), 7.79 (d, *J* = 8.4 Hz, 1H), 7.65 (dd, *J* = 8.5, 1.9 Hz, 1H), 7.42 - 7.34 (m, 4H), 7.30 - 7.26 (m, 1H), 539 (dd, *J* = 8.5, 6.2 Hz, 1H), 4.33 (td, *J* = 7.8, 3.0 Hz, 1H), 4.03 (s, 3H), 4.01 - 3.95 (m, 1H), 2.91 (dddd, *J* = 11.9, 9.3, 6.7, 3.0 Hz, 1H), 2.30 - 224 (m, 1H), 2.21 (s, 3H) | 490.3 |

(continued)

| Example | Structure | Name of Compound | $^1$H NMR | LC-MS $[M+H]^+$ |
|---|---|---|---|---|
| 27 | 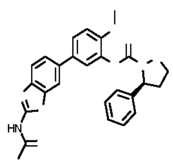 | (S)-N-(5 -(2-acetamidobenzo [d]thiazol-6-yl)-2-methoxyphenyl)-3-phenylisoxazolidine-2-caiboxamide | $^1$H NMR (400 MHz, DMSO-$d_6$) $\delta$ = 12.35 (s, 1H), 8.56 (s, 1H), 8.39 (d, $J$ = 2.3 Hz, 1H), 8.17 (d, $J$ = 1.9 Hz, 1H), 7.77 (d, $J$ = 8.4 Hz, 1H), 7.62 (dd, $J$ = 8.5, 1.9 Hz, 1H), 7.43 - 7.35 (m, 5H), 7.31 - 7.25 (m, 1H), 7.18 (d, $J$ = 8.6 Hz, 1H), 5.41 (dd, $J$ = 8.5, 6.1 Hz, 1H), 4.31 (td, $J$ = 7.8, 3.0 Hz, 1H), 4.01 - 3.95 (m, 1H), 3.94 (s, 3H), 2.90 (dddd, $J$ = 11.9, 9.4, 6.9, 3.1 Hz, 1H), 2.32 - 2.23 (m, 1H), 2.21 (s, 3H) | 489.3 |

(continued)

| Example | Structure | Name of Compound | $^1$H NMR | LC-MS $[M+H]^+$ |
|---|---|---|---|---|
| 28 | 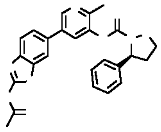 | (S)-N-(5 -(2-acetamidobenzo[d]thiazol-6-yl)-2-methylpyridin-3-yl)-3-phenylisoxazolidine-2-caiboxamide | $^1$H NMR (400 MHz, DMSO) δ = 12.41 (s, 1H), 9.15 (s, 1H), 8.66 (s, 1H), 8.33 (s, 1H), 8.09 (s, 1H), 7.86 - 7.67 (m, 2H), 7.50 - 7.23 (m, 5H), 5.42 (m, 1H), 4.31 (m, 1H), 4.01 (m, 1H), 2.88 (m, 1H), 2.33 (m, 1H), 2.22 (s, 3H), 1.23 (s, 3H) | 474.2 |

(continued)

| Example | Structure | Name of Compound | $^1H$ NMR | LC-MS $[M+H]^+$ |
|---|---|---|---|---|
| 29 | 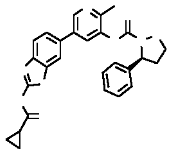 | (S)-N-(5-(2-(cyclopropanecarboxamido)benzo[d] thiazol-6-yl)-2-methylpyridin-3-yl)-3-phenylisoxazolidine-2-caiboxamide | $^1H$ NMR (400 MHz, DMSO) δ = 12.71 (s, 1H), 9.15 (s, 1H), 8.73 - 8.60 (m, 1H), 833 (s, 1H), 8.10 (s, 1H), 7.87 - 7.70 (m, 2H), 7.47 - 7.22 (m, 5H), 5.42 (m, 1H), 4.30 (m, 1H), 4.01 (m, 1H), 2.92 (m, 1H), 2.26 (m, 2H), 2.02 (m, 1H), 1.24 (m, 2H), 0.96 (s, 3H), 0.85 (m, 1H) | 500.2 |

(continued)

| Example | Structure | Name of Compound | $^1$H NMR | LC-MS $[M+H]^+$ |
|---|---|---|---|---|
| 30 | 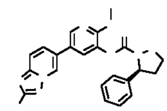 | (S)-N-(5-(2-amino-[1,2,4]triazolo[1,5-a]pyridin-6-yl)-2-methoxypyridin-3-yl)-3-phenylisoxazolidine-2-caiboxamide | $^1$H NMR (400 MHz, DMSO-$d_6$) δ 8.84 (d, *J* = 2.2 Hz, 1H), 8.56 - 8.46 (m, 2H), 8.25 (t, *J* = 2.4 Hz, 1H), 7.67 (dt, *J* = 9.1, 2.2 Hz, 1H), 7.47 - 7.34 (m, 5H), 728 (td, *J* = 6.4, 2.3 Hz, 1H), 6.09 (s, 2H), 5.39 (ddd, *J* = 8.5, 6.0, 2.2 Hz, 1H), 4.36 - 429 (m, 1H), 4.05 - 3.94 (m, 4H), 2.91 (ddd, *J* = 12.3, 9.0, 6.4 Hz, 1H), 227 (p, *J* = 9.2, 8.3 Hz, 1H) | 432.2 |

(continued)

| Example | Structure | Name of Compound | $^1$H NMR | LC-MS $[M+H]^+$ |
|---|---|---|---|---|
| 31 | 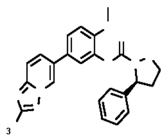 | (S)-N-(5-(2-amino-[1,2,4]triazolo[1,5-a]pyridin-6-yl)-2-methoxyphenyl)-3-phenylisoxazolidine-2-caiboxamide | $^1$H NMR (400 MHz, DMSO-$d_6$) $\delta$ = 8.71 (d, $J$ = 1.8 Hz, 1H), 8.56 (s, 1H), 8.32 (d, $J$ = 2.3 Hz, 1H), 7.64 (dd, $J$ = 9.2, 1.9 Hz, 1H), 7.45 - 737 (m, 5H), 736 (d, $J$ = 8.0 Hz, 1H), 7.30 - 7.25 (m, 1H), 7.17 (d, $J$ = 8.6 Hz, 1H), 6.04 (s, 2H), 5.41 (dd, $J$ = 8.5, 6.1 Hz, 1H), 4.31 (td, $J$ = 7.8, 3.1 Hz, 1H), 4.00 - 3.94 (m, 1H), 3.93 (s, 3H), 2.89 (dddd, $J$ = 12.0, 8.6, 6.8, 3.1 Hz, 1H), 2.26 (dddd, $J$ = 12.2, 9.4, 7.7, 6.0 Hz, 1H) | 431.3 |

(continued)

| Example | Structure | Name of Compound | $^1H$ NMR | LC-MS $[M+H]^+$ |
|---|---|---|---|---|
| 32 | 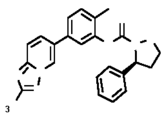 | (S)-N-(5-(2-amino-[1,2,4]triazolo[1,5-a]pyridin-6-yl)-2-methylphenyl)-3-phenylisoxazolidine-2-caiboxamide | $^1H$ NMR (400 MHz, DMSO-$d_6$) δ 8.96 (s, 1H), 8.80 (s, 1H), 7.78 - 7.68 (m, 2H), 7.50 - 7.45 (m, 1H), 7.44 - 7.26 (m, 6H), 6.05 (s, 2H), 5.42 (dd, *J* = 8.7, 5.7 Hz, 1H), 427 (td, *J* = 7.9, 3.1 Hz, 1H), 4.00 (q, *J* = 8.1 Hz, 1H), 2.89 (dd, *J* = 10.6, 6.4 Hz, 2H), 2.26 (s, 4H) | 415.2 |

(continued)

| Example | Structure | Name of Compound | $^1$H NMR | LC-MS $[M+H]^+$ |
|---|---|---|---|---|
| 33 | 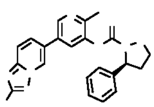 | (S)-N-(5-(2-amino-[1,2,4]triazolo[1,5-a]pyridin-6-yl)-2-methylpyridin-3-yl)-3-phenylisoxazolidine-2-caiboxamide | $^1$H NMR (400 MHz, DMSO-$d_6$) δ 9.18 (s, 1H), 8.96 (d, *J* = 1.9 Hz, 1H), 8.66 (d, *J* = 22 Hz, 1H), 8.08 (d, *J* = 22 Hz, 1H), 7.77 (dd, *J* = 9.1, 1.9 Hz, 1H), 7.45 (d, *J* = 9.1 Hz, 1H), 7.42 - 734 (m, 4H), 7.30 - 7.25 (m, 1H), 6.10 (s, 2H), 5.41 (dd, *J* = 8.6, 5.8 Hz, 1H), 4.30 (td, *J* = 7.9, 3.0 Hz, 1H), 4.00 (dt, *J* = 9.1, 7.4 Hz, 1H), 2.89 (tdd, *J* = 9.5, 7.6, 3.0 Hz, 1H), 2.47 (s, 3H), 2.30 - 2.22 (m, 1H) | 416.3 |

(continued)

| Example | Structure | Name of Compound | $^1$H NMR | LC-MS $[M+H]^+$ |
|---|---|---|---|---|
| 34 | 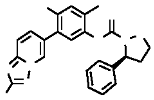 | (S)-N-(5-(2-amino-[1,2,4]triazolo[1,5-a]pyridin-6-yl)-4-fluoro-2-methylphenyl)-3-phenylisoxazolidine-2-caiboxamide | $^1$H NMR (400 MHz, DMSO-$d_6$) δ 9.02 (s, 1H), 8.69 (s, 1H), 7.67 - 7.54 (m, 4H), 7.45 (d, *J* = 9.1 Hz, 1H), 7.42 - 7.33 (m, 4H), 7.31-7.21 (m, 2H), 5.40 (dd, *J* = 8.7, 5.6 Hz, 1H), 4.26 (td, *J* = 8.0, 3.2 Hz, 1H), 3.98 (dt, *J* = 9.1, 7.5 Hz, 1H), 2.87 (dtd, *J* = 11.7, 7.8, 3.1 Hz, 1H), 2.27 (s, 4H) | 433.2 |

(continued)

| Example | Structure | Name of Compound | $^1H$ NMR | LC-MS $[M+H]^+$ |
|---|---|---|---|---|
| 35 | 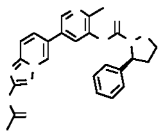 | (S)-N-(5-(2-acetamido-[1,2,4]triazolo[1,5-a]pyridin-6-yl)-2-methylpyridin-3-yl)-3-phenylisoxazolidine-2-caiboxamide | $^1H$ NMR (400 MHz, DMSO) δ = 10.83 (s, 1H), 9.31 (s, 1H), 9.21 (s, 1H), 8.73 (s, 1H), 8.16 (s, 1H), 7.97 (s, 1H), 7.78 (d, *J* = 8.5 Hz, 1H), 7.39 (m, *J* = 9.0 Hz, 4H), 7.29 (d, *J* = 6.5 Hz, 1H), 5.42 (m, 1H), 4.31 (m, 1H), 4.02 (m, 1H), 2.90 (m, 1H), 2.27 (m, 1H), 2.16 (s, 3H), 1.23 (s, 3H) | 458.2 |

(continued)

| Example | Structure | Name of Compound | $^1H$ NMR | LC-MS $[M+H]^+$ |
|---|---|---|---|---|
| 36 | 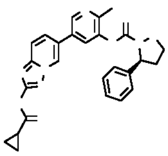 | (S)-N-(5-(2-(cyclopropanecaiboxamido)-[1,2,4] triazolo[1,5-a]pyridin-6-yl)-2-methylpyridin-3-yl)-3-phenylisoxazolidine-2-carboxamide | $^1H$ NMR (400 MHz, DMSO) δ = 11.11 (s, 1H), 930 (s, 1H), 9.22 (s, 1H), 8.74 (s, 1H), 8.18 (s, 1H), 7.99 (d, *J* = 11.0 Hz, 1H), 7.78 (d, *J* = 9.4 Hz, 1H), 7.50 - 7.18 (m, 6H), 5.47 - 5.38 (m, 1H), 4.31 (s, 1H), 4.07 - 396 (m, 1H), 2.93 (d, *J* = 16.1 Hz, 2H), 2.26 (d, *J* = 10.8 Hz, 1H), 2.08 (d, *J* = 9.8 Hz, 2H), 1.19 (s, 1H), 0.85 (s, 3H) | 484.2 |

(continued)

| Example | Structure | Name of Compound | $^1$H NMR | LC-MS $[M+H]^+$ |
|---|---|---|---|---|
| 37 | 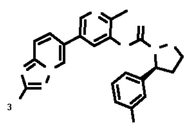 | (S)-N-(5-(2-amino-[1,2,4]triazolo[1,5-a]pyridin-6-yl)-2-methylpyridin-3-yl)-3-(3-fluoropheiryl) isoxazolidine-2-caiboxamide | $^1$H NMR (400 MHz, DMSO) δ = 9.27 (s, 1H), 9.01 (s, 1H), 8.71 (d, *J* = 2.4 Hz, 1H), 8.16 (s, 1H), 7.80 (d, *J* = 9.4 Hz, 1H), 7.48 (t, *J* = 8.8 Hz, 1H), 7.42 (q, *J* = 7.9 Hz, 1H), 7.23 (dd, *J* = 20.3, 8.6 Hz, 2H), 7.11 (td, *J* = 8.6, 2.9 Hz, 1H), 6.21 (s, 2H), 5.44 (dd, *J* = 8.9, 5.6 Hz, 1H), 4.31 (d, *J* = 3.3 Hz, 1H), 4.01 (d, *J* = 9.3 Hz, 1H), 2.91 (dd, *J* = 7.1, 3.5 Hz, 1H), 2.54 ( s, 3H), 2.27 (m, 1H) | 434.3 |

(continued)

| Example | Structure | Name of Compound | $^1$H NMR | LC-MS $[M+H]^+$ |
|---|---|---|---|---|
| 38 | 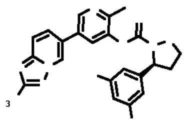 | (S)-N-(5-(2-amino-[1,2,4]triazolo[1,5-a]pyridin-6-yl)-2-methylpyridin-3-yl)-3-(3,5-difluorophenyl) isoxazolidine-2-carboxamide | $^1$H NMR (400 MHz, DMSO) δ = 926 (s, 1H), 8.97 ( dd, *J* = 1.9, 0.9 Hz, 1H), 8.68 (d, *J* = 2.3 Hz, 1H), 8. 08 (d, *J* = 2.3 Hz, 1H), 7.78 (dd, *J* = 9.2, 1.9 Hz, 1H ), 7.45 (dd, *J* = 9.1, 0.9 Hz, 1H), 7.19 - 7.08 (m, 3H ), 6.11 (s, 2H), 5.46 (dd, *J* = 8.8, 5.6 Hz, 1H), 4.29 (t d, *J* = 7.5, 3.2 Hz, 1H), 4.00 (dt, *J* = 9.4, 7.1 Hz, 1H ), 2.95 - 2.87 (m, 1H), 2.47 (s, 3H), 2.30 - 223 (m, 1H) | 452.2 |

(continued)

| Example | Structure | Name of Compound | $^1H$ NMR | LC-MS $[M+H]^+$ |
|---|---|---|---|---|
| 39 | 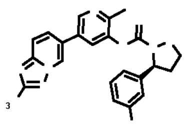 | (S)-N-(5-(2-amino-[1,2,4]triazolo[1,5-a]pyridin-6-yl)-2-methylpyridin-3-yl)-3-(3-chlorophenyl) isoxazolidine-2-carboxamide | $^1H$ NMR (400 MHz, DMSO) δ = 922 (s, 1H), 8.97 ( d, *J* = 2.3 Hz, 1H), 8.67 (d, *J* = 2.3 Hz, 1H), 8.09 (d, *J* = 2.4 Hz, 1H), 7.77 (dd, *J* = 9.1, 2.0 Hz, 1H), 7.4 5 (td, *J* = 4.6, 4.1, 2.6 Hz, 2H), 7.42 - 7.33 (m, 3H), 6.10 (s, 2H), 5.43 (dd, *J* = 8.8, 5.7 Hz, 1H), 4.30 (td, *J* = 8.1, 3.4 Hz, 1H), 4.00 (q, *J* = 7.9 Hz, 1H), 2.91 (dd, *J* = 7.8, 4.3 Hz, 1H), 2.47 (s, 3H), 2.28 - 2.22 ( m, 1H) | 450.2 |

(continued)

| Example | Structure | Name of Compound | $^1$H NMR | LC-MS $[M+H]^+$ |
|---|---|---|---|---|
| 40 | 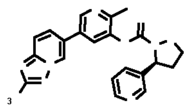 | (S)-N-(5-(2-amino-[1,2,4]triazolo[1,5-a]pyridin-6-yl)-2-methylpyridin-3-yl)-3-(pyridin-3-yl) isoxazolidine-2-caiboxamide | $^1$H NMR (400 MHz, DMSO) δ = 925 (s, 1H), 8.97 ( d, *J* = 2.0 Hz, 1H), 8.68 (d, *J* = 2.5 Hz, 1H), 8.64 (s, 1H), 8.51 (s, 1H), 8.09 (d, *J* = 2.5 Hz, 1H), 7.85 - 7.75 (m, 2H), 7.49 - 7.40 (m, 2H), 6.11 (s, 2H), 5.48 ( dd, *J* = 8.9, 5.6 Hz, 1H), 4.33 (q, *J* = 4.9 Hz, 1H), 4. 04 (d, *J* = 9.8 Hz, 1H), 2.93 (dd, *J* = 7.6, 4.1 Hz, 1H ), 2.47 (s, 3H), 2.32 (dq, *J* = 9.6, 6.4, 4.9 Hz, 1H) | 417.3 |

(continued)

| Example | Structure | Name of Compound | $^1$H NMR | LC-MS $[M+H]^+$ |
|---|---|---|---|---|
| 41 | 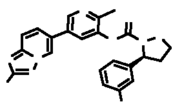 | (S)-N-(5-(2-amino-[1,2,4]triazolo[1,5-a]pyridin-6-yl)-2-methylpyridin-3-yl)-3-(3-(trifluoromethyl) phenyl)isoxazolidine-2-carboxamide | $^1$H NMR (400 MHz, DMSO) δ = 926 (s, 1H), 8.97 ( d, $J$ = 2.0 Hz, 1H), 8.67 (d, $J$ = 2.3 Hz, 1H), 8.09 (d , $J$ = 2.3 Hz, 1H), 7.80 - 7.70 (m, 3H), 7.69 - 7.60 ( m, 2H), 7.45 (d, $J$ = 9.1 Hz, 1H), 6.10 (s, 2H), 5.54 ( dd, $J$ = 8.9, 5.9 Hz, 1H), 4.32 (td, $J$ = 7.7, 2.8 Hz, 1 H), 4.06 - 3.98 (m, 1H), 2.99 - 2.93 (m, 1H), 2.47 (s | 484.2 |
| | | | , 3H), 228 (td, $J$ = 9.6, 8.8, 3.8 Hz, 1H) | |

(continued)

| Example | Structure | Name of Compound | $^1$H NMR | LC-MS $[M+H]^+$ |
|---|---|---|---|---|
| 42 | 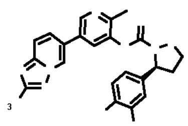 | (S)-N-(5-(2-amino-[1,2,4]triazolo[1,5-a]pyridin-6-yl)-2-methylpyridin-3-yl)-3-(3-chloro-4-fluorophenyl)isoxazolidine-2-carboxamide | $^1$H NMR (400 MHz, DMSO) δ = 923 (s, 1H), 8.97 ( dd, *J* = 2.1, 0.8 Hz, 1H), 8.67 (d, *J* = 2.4 Hz, 1H), 8. 08 (d, *J* = 2.3 Hz, 1H), 7.77 (dd, *J* = 9.2, 1.9 Hz, 1H ), 7.59 (d, *J* = 7.8 Hz, 1H), 7.48 - 7.38 (m, 3H), 6.1 0 (s, 2H), 5.43 (dd, *J* = 8.6, 5.9 Hz, 1H), 4.30 (td, *J* = 7.9, 3.0 Hz, 1H), 4.04 - 3.96 (m, 1H), 2.92 - 2.87 (m, 1H), 2.47 (s, 3H), 2.30 - 2.23 (m, 1H) | 468.2 |

(continued)

| Example | Structure | Name of Compound | $^1$H NMR | LC-MS $[M+H]^+$ |
|---|---|---|---|---|
| 43 | 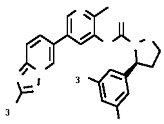 | (S)-N-(5-(2-amino-[1,2,4]triazolo[1,5-a]pyridin-6-yl)-2-methylpyridin-3-yl)-3-(3-fluoro-5-(trifluoromethyl)phenyl)isoxazolidine-2-carboxamide | $^1$H NMR (400 MHz, DMSO-$d_6$) δ 9.30 (s, 1H), 8.97 (d, *J* = 1.8 Hz, 1H), 8.68 (d, *J* = 22 Hz, 1H), 8.09 (d, *J* = 23 Hz, 1H), 7.78 (dd, *J* = 9.1, 1.9 Hz, 1H), 7.64 - 7.55 (m, 3H), 7.45 (d, *J* = 9.1 Hz, 1H), 6.10 (s, 2H), 5.56 (dd, *J* = 8.7, 5.6 Hz, 1H), 4.32 (td, *J* = 8.0, 3.0 Hz, 1H), 4.02 (q, *J* = 8.1 Hz, 1H), 2.98 - 2.92 (m, 1H), 2.47 (s, 3H), 2.34 - 228 (m, 1H) | 502.2 |

(continued)

| Example | Structure | Name of Compound | $^1H$ NMR | LC-MS $[M+H]^+$ |
|---|---|---|---|---|
| 44 | 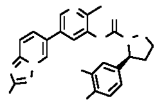 | (S)-N-(5-(2-amino-[1,2,4]triazolo[1,5-a]pyridin-6-yl)-2-methylpyridin-3-yl)-3-(4-chloro-3-(trifluoromethyl)phenyl)isoxazolidine-2-carboxamide | $^1H$ NMR (400 MHz, DMSO-$d_6$) δ 9.28 (s, 1H), 8.97 (*d, J* = 1.9 Hz, 1H), 8.68 (*d, J* = 22 Hz, 1H), 8.08 (d, *J* = 2.3 Hz, 1H), 7.85 (d, *J* = 1.7 Hz, 1H), 7.79 - 7.72 (m, 3H), 7.45 (d, *J* = 9.2 Hz, 1H), 6.10 (s, 2H), 5.53 (dd, *J* = 8.6, 5.7 Hz, 1H), 4.34 - 4.28 (m, 1H), 4.02 (q, *J* = 8.3 Hz, 1H), 2.97 - 2.91 (m, 1H), 2.47 (s, 3H), 2.29 (d, *J* = 6.0 Hz, 1H) | 518.2 |

(continued)

| Example | Structure | Name of Compound | $^1$H NMR | LC-MS $[M+H]^+$ |
|---|---|---|---|---|
| 45 | 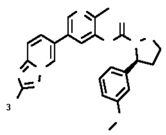 | (S)-N-(5-(2-amino-[1,2,4]triazolo[1,5-a]pyridin-6-yl)-2-methylpyridin-3-yl)-3-(3-methoxyphenyl) isoxazolidine-2-carboxamide | $^1$H NMR (400 MHz, DMSO-$d_6$) δ 9.17 (s, 1H), 8.96 (t, *J* = 1.3 Hz, 1H), 8.66 (d, *J* = 23 Hz, 1H), 8.09 (d, *J* = 23 Hz, 1H), 7.77 (dd, *J* = 9.2, 1.9 Hz, 1H), 7.45 (d, *J* = 9.2 Hz, 1H), 728 (t, *J* = 7.9 Hz, 1H), 7.01 - 6.92 (m, 2H), 6.87 - 6.81 (m, 1H), 6.10 (s, 2H), 5.39 (dd, *J* = 8.6, 5.7 Hz, 1H), 4.31 - 426 (m, 1H), 4.01 (d, *J* = 8.2 Hz, 1H), 3.76 (s, 3H), 2.90 - 2.84 (m, 1H), 2.47 (s, 3H), 226 (dd, *J* = 10.0, 6.3 Hz, 1H) | 446.3 |

(continued)

| Example | Structure | Name of Compound | $^1$H NMR | LC-MS $[M+H]^+$ |
|---|---|---|---|---|
| 46 | 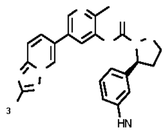 | (S)-N-(5-(2-amino-[1,2,4]triazolo[1,5-a]pyridin-6-yl)-2-methylpyridin-3-yl)-3-(3-cyanophenyl) isoxazolidine-2-carboxamide | $^1$H NMR (400 MHz, DMSO-$d_6$) δ 9.26 (s, 1H), 8.97 (s, 1H), 8.68 (d, *J* = 2.3 Hz, 1H), 8.08 (d, *J* = 2.2 Hz, 1H), 7.84 (s, 1H), 7.77 (d, *J* = 8.5 Hz, 3H), 7.60 (t, *J* = 7.8 Hz, 1H), 7.45 (d, *J* = 9.1 Hz, 1H), 6.10 (s, 2H), 5.51 - 5.46 (m, 1H), 4.31 (s, 1H), 4.02 (d, *J* = 8.3 Hz, 1H), 2.95 - 2.92 (m, 1H), 2.47 (s, 3H), 2.29 (s, 1H) | 441.2 |

(continued)

| Example | Structure | Name of Compound | $^1$H NMR | LC-MS $[M+H]^+$ |
|---|---|---|---|---|
| 47 | 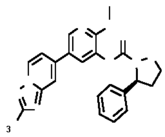 | (S)-N-(5-(2-amino-[1,2,4]triazolo[1,5-a]pyridin-7-yl)-2-methoxypyridin-3-yl)-3-phenylisoxazolidine-2-caiboxamide | $^1$H NMR (400 MHz, DMSO-$d_6$) δ 8.65 - 8.54 (m, 2H), 8.51 (s, 1H), 8.35 (d, *J* = 2.3 Hz, 1H), 7.57 (d, *J* = 1.9 Hz, 1H), 7.44 - 732 (m, 4H), 7.31 - 7.25 (m, 1H), 7.13 (dd, *J* = 7.0, 2.0 Hz, 1H), 6.05 (s, 2H), 5.40 (dd, *J* = 8.5, 6.1 Hz, 1H), 4.32 (td, *J* = 7.8, 3.0 Hz, 1H), 4.03 (s, 3H), 4.00 - 3.94 (m, 1H), 2.94 - 2.87 (m, 1H), 2.30 - 2.23 (m, 1H) | 432.3 |

(continued)

| Example | Structure | Name of Compound | $^1$H NMR | LC-MS $[M+H]^+$ |
|---|---|---|---|---|
| 48 | 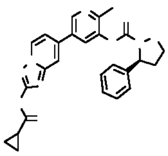 | (S)-N-(5-(2-(cyclopropanecaiboxamido)-[1,2,4] triazolo[1,5-a]pyridin-7-yl)-2-methylpyridin-3-yl)-3-phenylisoxazolidine-2-carboxamide | $^1$H NMR (400 MHz, DMSO) $\delta$ = 11.10 (s, 1H), 9.22 (s, 1H), 8.91 (d, $J$ = 7.3 Hz, 1H), 8.81 (s, 1H), 8.24 (s, 1H), 8.05 (s, 1H), 7.54 - 7.23 (m, 6H), 5.47 - 5.36 (m, 1H), 4.31 (m, 1H), 4.02 (m, 1H), 2.90 (m, 2H), 230 (d, $J$ = 25.0 Hz, 2H), 2.06 (m, 2H), 1.24 (m, 1H), 0.83 (s, 3H) | 484.2 |

(continued)

| Example | Structure | Name of Compound | $^1$H NMR | LC-MS $[M+H]^+$ |
|---|---|---|---|---|
| 49 | 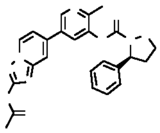 | (S)-N-(5-(2-acetamido-[1,2,4]triazolo[1,5-a]pyridin-7-yl)-2-methylpyridin-3-yl)-3-phenylisoxazolidine-2-caiboxamide | $^1$H NMR (400 MHz, DMSO) δ = 10.83 (s, 1H), 9.23 (s, 1H), 8.92 (d, *J* = 7.3 Hz, 1H), 8.80 (s, 1H), 8.24 (s, 1H), 8.04 (s, 1H), 7.57 - 7.18 (m, 6H), 5.43 (m, 1H), 4.31 (m, 1H), 4.08 - 3.95 (m, 1H), 3.21 (s, 3H), 2.91 (m, 1H), 2.27 (m, 1H), 2.16 (s, 3H) | 458.2 |

(continued)

| Example | Structure | Name of Compound | $^1$H NMR | LC-MS [M+H]$^+$ |
|---|---|---|---|---|
| 50 | 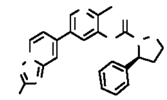 | (S)-N-(5-(2-amino-[1,2,4]triazolo[1,5-a]pyridin-7-yl)-2-methylpyridin-3-yl)-3-phenylisoxazolidine-2-caiboxamide | $^1$H NMR (400 MHz, DMSO) δ = 9.19 (s, 1H), 8.74 (s, 1H), 8.62 (d, *J* = 6.9 Hz, 1H), 8.17 (s, 1H), 7.70 (s, 1H), 738 (dd, *J* = 13.0, 5.9 Hz, 3H), 7.32 - 7.19 (m, 2H), 6.08 (s, 2H), 5.41 (d, *J* = 7.2 Hz, 1H), 4.30 (s, 1H), 4.00 (t, *J* = 8.3 Hz, 1H), 2.93 - 2.86 (m, 1H), 2.30 - 2.24 (m, 1H), 1.24 (s, 3H), 0.85 (d, *J* = 8.0 Hz, 1H) | 416.3 |

(continued)

| Example | Structure | Name of Compound | $^1$H NMR | LC-MS $[M+H]^+$ |
|---|---|---|---|---|
| 51 | 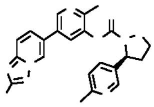 | (S)-N-(5-(2-amino-[1,2,4]triazolo[1,5-a]pyridin-6-yl)-2-methylpyridin-3-yl)-3-(6-methylpyridin-3-yl) isoxazolidine-2-carboxamide | $^1$H NMR (400 MHz, DMSO-$d_6$) δ = 9.21 (s, 1H), 8.97 (d, *J* = 1.8 Hz, 1H), 8.67 (d, *J* = 2.2 Hz, 1H), 8.47 (d, *J* = 2.3 Hz, 1H), 8.08 (d, *J* = 2.2 Hz, 1H), 7.77 (dd, *J* = 9.1, 1.9 Hz, 1H), 7 68 (dd, *J* = 8.1, 2.4 Hz, 1H), 7.45 (d, *J* = 9.1 Hz, 1H), 7.26 (d, *J* = 8.0 Hz, 1H), 6.10 (s, 2H), 5.44 (dd, *J* = 8.5, 5.6 Hz, 1H), 431 (td, *J* = 7.9, 3.2 Hz, 1H), 4.03 (q, *J* = 8.1 Hz, 1H), 2.88 (dtd, *J* = 11.9, 7.8, 3.2 Hz, 1H), 2.46 (d, *J* = 1.7 Hz, 6H), 2.29 (dq, *J* = 14.0, 8.2 Hz, 1H) | 431.2 |

(continued)

| Example | Structure | Name of Compound | $^1$H NMR | LC-MS $[M+H]^+$ |
|---|---|---|---|---|
| 52 | 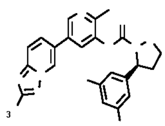 | (S)-N-(5-(2-amino-[1,2,4]triazolo[1,5-a]pyridin-6-yl)-2-methylpyridin-3-yl)-3-(3-chloro-5-fluorophenyl)isoxazolidine-2-carboxamide | $^1$H NMR (400 MHz, DMSO-$d_6$) $\delta$ = 9.28 (s, 1H), 8.99 (d, *J* = 1.8 Hz, 1H), 8.70 (d, *J* = 22 Hz, 1H), 8.12 (d, *J* = 2.2 Hz, 1H), 7.79 (dd, *J* = 9.2, 1.9 Hz, 1H), 7.46 (d, *J* = 9.1 Hz, 1H), 7.38 - 7.31 (m, 2H), 724 (dt, *J* = 9.8, 1.9 Hz, 1H), 6.14 (s, 2H), 5.46 (dd, *J* = 8.7, 5.7 Hz, 1H), 4.30 (td, *J* = 7.9, 2.9 Hz, 1H), 4.00 (dt, *J* = 9.4, 7.5 Hz, 1H), 2.92 (tdt, *J* = 9.6, 7.2, 3.0 Hz, 1H), 2.48 (s, 3H), 227 (dtd, *J* = 12.3, 8.0, 5.7 Hz, 1H) | 468.2 |

(continued)

| Example | Structure | Name of Compound | $^1$H NMR | LC-MS $[M+H]^+$ |
|---|---|---|---|---|
| 53 | 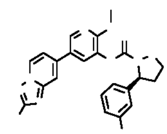 | (S)-N-(5-(2-amino-[1,2,4]triazolo[1,5-a]pyridin-7-yl)-2-methoxypyridin-3-yl)-3-(3-fluorophenyl) isoxazolidine-2-carboxamide | $^1$H NMR (400 MHz, DMSO-$d_6$) δ = 8.62 - 8.58 (m, 2H), 8.55 (s, 1H), 8.37 (d, *J* = 2.3 Hz, 1H), 7.58 (dd, *J* = 2.0, 0.9 Hz, 1H), 7.43 (td, *J* = 8.0, 6.1 Hz, 1H), 7.25 (d, *J* = 7.7 Hz, 1H), 7.21 (dt, *J* = 10.2, 2.2 Hz, 1H), 7.17 - 7.09 (m, 2H), 6.08 (s, 2H), 5.43 (dd, *J* = 8.5, 6.2 Hz, 1H), 4.32 (td, *J* = 7.8, 3.0 Hz, 1H), 4.03 (s, 3H), 4.01 - 3.96 (m, 1H), 2.92 (dddd, *J* = 12.1, 9.4, 6.8, 3.0 Hz, 1H), 2.32 - 222 (m, 1H) | 450.1 |

(continued)

| Example | Structure | Name of Compound | $^1$H NMR | LC-MS $[M+H]^+$ |
|---|---|---|---|---|
| 54 | 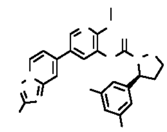 | (S)-N-(5-(2-amino-[1,2,4]triazolo[1,5-a]pyridin-7-yl)-2-methoxypyridin-3-yl)-3-(3,5-difluorophenyl) isoxazolidine-2-carboxamide | $^1$H NMR (400 MHz, DMSO-$d_6$) $\delta$ = 8.62 - 8.56 (m, 3H), 8.38 (d, $J$ = 2.3 Hz, 1H), 7.59 (dd, $J$ = 2.0, 0.9 Hz, 1H), 7.22 - 7.07 (m, 4H), 6.08 (s, 2H), 5.44 (dd, $J$ = 8.5, 6.1 Hz, 1H), 4.31 (td, $J$ = 7.9, 2.9 Hz, 1H), 4.03 (s, 3H), 4.01 - 3.95 (m, 1H), 2.93 (tdd, $J$ = 12.0, 6.7, 3.0 Hz, 1H), 2.27 (t, $J$ = 10.3 Hz, 1H) | 468.1 |

(continued)

| Example | Structure | Name of Compound | $^1$H NMR | LC-MS $[M+H]^+$ |
|---|---|---|---|---|
| 55 | 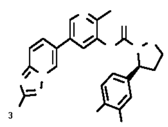 | (S)-N-(5-(2-amino-[1,2,4]triazolo[1,5-a]pyridin-6-yl)-2-methylpyridin-3-yl)-3-(3,4-difluorophenyl) isoxazolidine-2-carboxamide | $^1$H NMR (400 MHz, DMSO-$d_6$) $\delta$ = 9.23 (s, 1H), 8.97 (d, *J* = 1.9 Hz, 1H), 8.68 (t, *J* = 2.9 Hz, 1H), 8.08 (d, *J* = 2.3 Hz, 1H), 7.78 (dt, *J* = 9.2, 2.9 Hz, 1H), 7.49 - 7.39 (m, 3H), 7.30 - 723 (m, 1H), 6.10 (s, 2H), 5.43 (dd, *J* = 8.7, 5.4 Hz, 1H), 4.29 (dt, *J* = 8.0, 4.1 Hz, 1H), 4.00 (q, *J* = 8.0 Hz, 1H), 2.89 (dtd, *J* = 11.8, 7.8, 3.0 Hz, 1H), 2.47 (s, 3H), 2.27 (ddd, *J* = 14.0, 8.9, 6.0 Hz, 1H) | 452.2 |

(continued)

| Example | Structure | Name of Compound | $^1$H NMR | LC-MS $[M+H]^+$ |
|---|---|---|---|---|
| 56 | 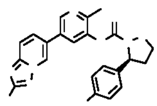 | (S)-N-(5-(2-amino-[1,2,4]triazolo[1,5-a]pyridin-6-yl)-2-methylpyridin-3-yl)-3-(4-fluoropheiryl) isoxazolidine-2-caiboxamide | $^1$H NMR (400 MHz, DMSO-$d_6$) δ = 9.19 (s, 1H), 8.97 (d, *J* = 1.8 Hz, 1H), 8.67 (d, *J* = 22 Hz, 1H), 8.08 (d, *J* = 2.2 Hz, 1H), 7.77 (dd, *J* = 9.2, 1.9 Hz, 1H), 7.48 - 7.40 (m, 3H), 7.19 (t, *J* = 8.8 Hz, 2H), 6.10 (s, 2H), 5.42 (dd, *J* = 8.6, 5.8 Hz, 1H), 4.30 (td, *J* = 7.9, 3.0 Hz, 1H), 4.00 (q, *J* = 8.1 Hz, 1H), 2.88 (dtd, *J* = 11.5, 8.0, 3.1 Hz, 1H), 2.47 (s, 3H), 2.29 - 2.21 (m, 1H) | 434.1 |

(continued)

| Example | Structure | Name of Compound | $^1H$ NMR | LC-MS $[M+H]^+$ |
|---|---|---|---|---|
| 57 | 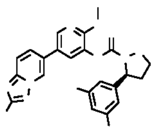 | (S)-N-(5-(2-amino-[1,2,4]triazolo[1,5-a]pyridin-6-yl)-2-methoxypyridin-3-yl)-3-(3,5-difluorophenyl) isoxazolidine-2-carboxamide | $^1H$ NMR (400 MHz, DMSO-$d_6$) δ = 8.84 (d, *J* = 1.9 Hz, 1H), 8.59 - 8.48 (m, 2H), 8.26 (d, *J* = 2.3 Hz, 1H), 7.67 (dd, *J* = 9.2, 1.9 Hz, 1H), 7.44 (d, *J* = 9.1 Hz, 1H), 7.15 (ddd, *J* = 12.6, 9.0, 6.8 Hz, 3H), 6.08 (s, 2H), 5.43 (dd, *J* = 8.6, 6.1 Hz, 1H), 4.31 (dt, *J* = 8.0, 4.0 Hz, 1H), 4.01 (s, 4H), 2.98 - 2.87 (m, 1H), 2.36 - 2.22 (m, 1H) | 468.2 |

(continued)

| Example | Structure | Name of Compound | $^1$H NMR | LC-MS $[M+H]^+$ |
|---|---|---|---|---|
| 58 | 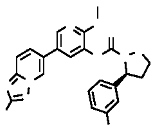 | (S)-N-(5-(2-amino-[1,2,4]triazolo[1,5-a]pyridin-6-yl)-2-methoxypyridin-3-yl)-3-(3-fluorophenyl) isoxazolidine-2-carboxamide | $^1$H NMR (400 MHz, DMSO-$d_6$) δ = 8.84 (dd, *J* = 1.9, 0.9 Hz, 1H), 8.56 - 8.49 (m, 2H), 8.25 (d, *J* = 2.3 Hz, 1H), 7.67 (dd, *J* = 9.1, 1.9 Hz, 1H), 7.46 - 7.39 (m, 2H), 7.27 - 7.17 (m, 2H), 7.11 (td, *J* = 8.6, 2.7 Hz, 1H), 6.07 (s, 2H), 5.42 (dd, *J* = 8.5, 6.1 Hz, 1H), 4.32 (td, *J* = 7.8, 3.0 Hz, 1H), 4.02 (s, 4H), 2.96 - 2.87 (m, 1H), 2.32 - 2.22 (m, 1H) | 450.2 |

(continued)

| Example | Structure | Name of Compound | $^1H$ NMR | LC-MS $[M+H]^+$ |
|---|---|---|---|---|
| 59 | 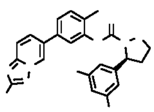 | (S)-N-(5-(2-amino-[1,2,4]triazolo[1,5-a]pyridin-6-yl)-2-methylphenyl)-3-(3,5-difluorophenyl) isoxazolidine-2-carboxamide | $^1H$ NMR (400 MHz, DMSO-$d_6$) δ = 9.05 (s, 1H), 8.82 (d, *J* = 1.9 Hz, 1H), 7.72 (dd, *J* = 6.8, 2.1 Hz, 2H), 7.49 (dd, *J* = 7.9, 2.0 Hz, 1H), 7.42 (d, *J* = 9.1 Hz, 1H), 732 (d, *J* = 7.9 Hz, 1H), 7.16 - 7.08 (m, 3H), 6.05 (s, 2H), 5.46 (dd, *J* = 8.8, 5.6 Hz, 1H), 4.27 (td, *J* = 8.0, 3.1 Hz, 1H), 4.00 (q, *J* = 8.1 Hz, 1H), 2.89 (dtd, *J* = 11.8, 8.2, 3.0 Hz, 1H), 2.26 (s, 4H) | 451.2 |

(continued)

| Example | Structure | Name of Compound | $^1$H NMR | LC-MS $[M+H]^+$ |
|---|---|---|---|---|
| 60 | 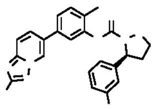 | (S)-N-(5-(2-amino-[1,2,4]triazolo[1,5-a]pyridin-6-yl)-2-methylphenyl)-3-(3-fluoropheiryl) isoxazolidine-2-caiboxamide | $^1$H NMR (400 MHz, DMSO-$d_6$) $\delta$ = 9.07 - 9.02 (m, 2H), 8.02 (dd, *J* = 9.1, 1.8 Hz, 1H), 7.79 (d, *J* = 2.0 Hz, 1H), 7.63 (d, *J* = 9.1 Hz, 1H), 7.53 (dd, *J* = 7.9, 2.0 Hz, 1H), 7.42 (td, *J* = 8.0, 6.1 Hz, 1H), 736 (d, *J* = 8.1 Hz, 1H), 725 (d, *J* = 7.7 Hz, 1H), 721 (dt, *J* = 10.3, 2.2 Hz, 1H), 7.11 (td, *J* = 8.7, 2.7 Hz, 1H), 5.44 (dd, *J* = 8.6, 5.6 Hz, 1H), 428 (td, *J* = 8.0, 3.2 Hz, 1H), 3.99 (dd, *J* = 7.9, 1.7 Hz, 1H), 2.89 (dddd, *J* = 12.1, 8.7, 7.2, 3.2 Hz, 1H), 2.32 - 2.21 (m, 4H) | 433.2 |

(continued)

| Example | Structure | Name of Compound | $^1H$ NMR | LC-MS $[M+H]^+$ |
|---|---|---|---|---|
| 61 | 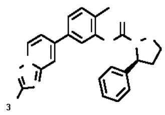 | (S)-N-(5-(2-amino-[1,2,4]triazolo[1,5-a]pyridin-7-yl)-2-methylphenyl)-3-phenylisoxazolidine-2-caiboxamide | $^1H$ NMR (400 MHz, DMSO-$d_6$) δ = 9.01 (s, 1H), 8.57 (dd, *J* = 6.9, 0.8 Hz, 1H), 7.83 (d, *J* = 2.0 Hz, 1H), 7.58 (dd, *J* = 2.1, 0.9 Hz, 1H), 7.57 - 7.53 (m, 1H), 7.44 - 7.33 (m, 5H), 7.31 - 7.24 (m, 1H), 7.16 (dd, *J* = 7.1, 2.0 Hz, 1H), 6.05 (s, 2H), 5.42 (dd, *J* = 8.6, 5.7 Hz, 1H), 428 (td, *J* = 7.9, 3.1 Hz, 1H), 4.00 (dt, *J* = 9.1, 7.4 Hz, 1H), 2.89 (tdd, *J* = 12.0, 7.2, 3.1 Hz, 1H), 228 (s, 3H), 224 (ddd, *J* = 9.4, 7.9, 4.8 Hz, | 415.1 |
| | | | 1H) | |

(continued)

| Example | Structure | Name of Compound | $^1$H NMR | LC-MS $[M+H]^+$ |
|---|---|---|---|---|
| 62 | 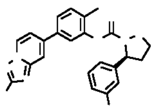 | (S)-N-(5-(2-amino-[1,2,4]triazolo[1,5-a]pyridin-7-yl)-2-methylphenyl)-3-(3-fluoropheiryl) isoxazolidine-2-caiboxamide | $^1$H NMR (400 MHz, DMSO-$d_6$) $\delta$ = 9.05 (s, 1H), 8.57 (d, $J$ = 7.0 Hz, 1H), 7.82 (d, $J$ = 2.0 Hz, 1H), 7.60 - 7.53 (m, 2H), 7.43 (td, $J$ = 8.0, 6.1 Hz, 1H), 7.36 (d, $J$ = 8.0 Hz, 1H), 7.26 (d, $J$ = 7.8 Hz, 1H), 721 (dt, $J$ = 10.3, 2.2 Hz, 1H), 7.16 (dd, $J$ = 7.0, 2.0 Hz, 1H), 7.11 (td, $J$ = 8.7, 2.7 Hz, 1H), 6.05 (s, 2H), 5.45 (dd, $J$ = 8.6, 5.6 Hz, 1H), 428 (td, $J$ = 8.0, 3.1 Hz, 1H), 4.01 (dt, $J$ = 9.2, 7.5 Hz, 1H), 2.90 (dtd, $J$ = 11.6, 8.1, 3.1 Hz, 1H), 2.28 (s, 3H), 2.27 - 221 (m, 1H) | 433.1 |

(continued)

| Example | Structure | Name of Compound | $^1$H NMR | LC-MS $[M+H]^+$ |
|---|---|---|---|---|
| 63 | 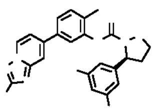 | (S)-N-(5-(2-amino-[1,2,4]triazolo[1,5-a]pyridin-7-yl)-2-methylphenyl)-3-(3,5-difluorophenyl) isoxazolidine-2-carboxamide | $^1$H NMR (400 MHz, DMSO-$d_6$) δ = 9.09 (s, 1H), 8.57 (dd, *J* = 7.0, 0.8 Hz, 1H), 7.81 (d, *J* = 2.0 Hz, 1H), 7.60 - 7.55 (m, 2H), 7.36 (d, *J* = 8.1 Hz, 1H), 7.19 - 7.09 (m, 4H), 6.05 (s, 2H), 5.47 (dd, *J* = 8.7, 5.6 Hz, 1H), 428 (td, *J* = 8.0, 3.1 Hz, 1H), 4.00 (dt, *J* = 9.2, 7.5 Hz, 1H), 2.90 (dddd, *J* = 12.1, 8.8, 7.1, 3.1 Hz, 1H), 2.28 (s, 3H), 2.27 - 2.19 (m, 1H) | 451.1 |

(continued)

| Example | Structure | Name of Compound | $^1$H NMR | LC-MS $[M+H]^+$ |
|---|---|---|---|---|
| 64 | 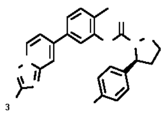 | (S)-N-(5-(2-amino-[1,2,4]triazolo[1,5-a]pyridin-7-yl)-2-methylphenyl)-3-(4-fluoropheiryl) isoxazolidine-2-caiboxamide | $^1$H NMR (400 MHz, DMSO-$d_6$) $\delta$ = 9.02 (s, 1H), 8.57 (dd, *J* = 7.1, 0.8 Hz, 1H), 7.82 (d, *J* = 2.0 Hz, 1H), 7.59 - 7.54 (m, 2H), 7.47 - 7.42 (m, 2H), 7.35 (d, *J* = 8.0 Hz, 1H), 7.23 - 7.17 (m, 2H), 7.16 (dd, *J* = 7.0, 2.0 Hz, 1H), 6.05 (s, 2H), 5.43 (dd, *J* = 8.6, 5.7 Hz, 1H), 428 (td, *J* = 8.0, 3.1 Hz, 1H), 4.00 (dt, *J* = 9.1, 7.4 Hz, 1H), 2.94 - 2.82 (m, 1H), 2.28 (s, 3H), 2.26 - 220 (m, 1H) | 433.1 |

(continued)

| Example | Structure | Name of Compound | $^1H$ NMR | LC-MS $[M+H]^+$ |
|---|---|---|---|---|
| 65 | 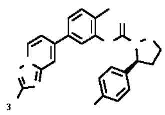 | (S)-N-(5-(2-amino-[1,2,4]triazolo[1,5-a]pyridin-7 -yl)-2-methylphenyl)-3-(4-chlorophenyl) isoxazolidine-2-carboxamide | $^1H$ NMR (400 MHz, DMSO-$d_6$) $\delta$ = 9.04 (s, 1H), 8.57 (dd, *J* = 7.0, 0.8 Hz, 1H), 7.81 (d, *J* = 2.0 Hz, 1H), 7.60 - 7.54 (m, 2H), 7.44 (s, 4H), 7.35 (d, *J* = 8.1 Hz, 1H), 7.16 (dd, *J* = 7.0, 2.0 Hz, 1H), 6.05 (s, 2H), 5.43 (dd, *J* = 8.6, 5.6 Hz, 1H), 428 (td, *J* = 8.0, 3.1 Hz, 1H), 4.00 (dt, *J* = 9.2, 7.4 Hz, 1H), 2.95 - 2.84 (m, 1H), 2.28 (s, 3H), 2.26 - 2.17 (m, 1H) | 449.0 |

(continued)

| Example | Structure | Name of Compound | $^1$H NMR | LC-MS $[M+H]^+$ |
|---|---|---|---|---|
| 66 | 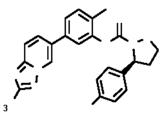 | (S)-N-(5-(2-amino-[1,2,4]triazolo[1,5-a]pyridin-6-yl)-2-methylphenyl)-3-(4-fluoropheiryl) isoxazolidine-2-caiboxamide | $^1$H NMR (400 MHz, DMSO-$d_6$) δ = 9.01 - 8.96 (m, 2H), 7.93 (dd, *J* = 9.2, 1.9 Hz, 1H), 7.78 (d, *J* = 2.1 Hz, 1H), 7.56 (d, *J* = 9.1 Hz, 1H), 7.51 (dd, *J* = 7.9, 2.1 Hz, 1H), 7.43 (qd, *J* = 6.9, 6.2, 1.7 Hz, 3H), 7.35 (d, *J* = 8.4 Hz, 1H), 7.19 (td, *J* = 8.9, 2.9 Hz, 3H), 5.45 - 5.39 (m, 1H), 4.28 (td, *J* = 7.8, 3.3 Hz, 1H), 4.03 - 3.96 (m, 1H), 2.87 (dddd, *J* = 12.1, 8.8, 7.2, 3.3 Hz, 1H), 2.27 (s, 3H), 2.24 - 2.19 (m, 1H) | 433.2 |

(continued)

| Example | Structure | Name of Compound | $^1H$ NMR | LC-MS $[M+H]^+$ |
|---|---|---|---|---|
| 67 | 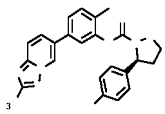 | (S)-N-(5-(2-amino-[1,2,4]triazolo[1,5-a]pyridin-6-yl)-2-methylphenyl)-3-(4-chlorophenyl) isoxazolidine-2-carboxamide | $^1H$ NMR (400 MHz, DMSO-$d_6$) $\delta$ = 9.03 - 8.97 (m, 2H), 7.93 (dd, *J* = 9.1, 1.8 Hz, 1H), 7.77 (d, *J* = 2.0 Hz, 1H), 7.56 (d, *J* = 9.1 Hz, 1H), 7.51 *(dd, J* = 7.9, 2.0 Hz, 1H), 7.42 (d, *J* = 4.9 Hz, 5H), 7.35 (d, *J* = 8.0 Hz, 1H), 5.42 (dd, *J* = 8.5, 5.7 Hz, 1H), 428 (td, *J* = 8.0, 3.2 Hz, 1H), 4.02 - 3.97 (m, 1H), 2.88 (dtd, *J* = 12.1, 8.4, 3.2 Hz, 1H), 2.30 - 2.17 (m, 5H) | 449.2 |

(continued)

| Example | Structure | Name of Compound | $^1$H NMR | LC-MS $[M+H]^+$ |
|---|---|---|---|---|
| 68 | 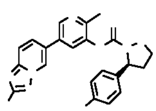 | (S)-N-(5-(2-amino-[1,2,4]triazolo[1,5-a]pyridin-6-yl)-2-methylpyridin-3-yl)-3-(4-cyanophenyl) isoxazolidine-2-carboxamide | $^1$H NMR (400 MHz, DMSO-$d_6$) $\delta$ = 9.28 (s, 1H), 8.98 (d, *J* = 1.8 Hz, 1H), 8.69 (d, *J* = 2.2 Hz, 1H), 8.10 (d, *J* = 2.3 Hz, 1H), 7.88 - 7.82 (m, 2H), 7.78 (dd, *J* = 9.2, 1.9 Hz, 1H), 7.63 - 7.58 (m, 2H), 7.46 (d, *J* = 9.1 Hz, 1H), 6.12 (s, 2H), 5.50 (dd, *J* = 8.7, 5.9 Hz, 1H), 4.31 (td, *J* = 8.0, 2.9 Hz, 1H), 4.01 (dt, *J* = 9.4, 7.3 Hz, 1H), 2.95 (tdd, *J* = 9.4, 7.2, 2.9 Hz, 1H), 2.47 (s, 3H), 2.28 - 220 (m, 1H) | 441.2 |

(continued)

| Example | Structure | Name of Compound | $^1H$ NMR | LC-MS $[M+H]^+$ |
|---|---|---|---|---|
| 69 | 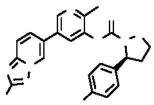 | (S)-N-(5-(2-amino-[1,2,4]triazolo[1,5-a]pyridin-6-yl)-2-methylpyridin-3-yl)-3-(4-chlorophenyl) isoxazolidine-2-carboxamide | $^1H$ NMR (400 MHz, DMSO-$d_6$) $\delta$ = 9.24 (s, 1H), 8.99 (d, *J* = 1.8 Hz, 1H), 8.69 (d, *J* = 2.2 Hz, 1H), 8.13 (d, *J* = 2.3 Hz, 1H), 7.79 (dd, *J* = 9.2, 1.9 Hz, 1H), 7.47 (d, *J* = 9.2 Hz, 1H), 7.43 (s, 4H), 6.15 (s, 2H), 5.42 (dd, *J* = 8.6, 5.8 Hz, 1H), 430 (td, *J* = 7.9, 3.0 Hz, 1H), 4.00 (dt, *J* = 9.1, 7.4 Hz, 1H), 2.93 - 2.85 (m, 1H), 2.48 (s, 3H), 224 (dddd, *J* = 12.1, 9.3, 7.8, 5.7 Hz, 1H) | 450.1 |

(continued)

| Example | Structure | Name of Compound | $^1H$ NMR | LC-MS $[M+H]^+$ |
|---|---|---|---|---|
| 70 | 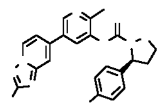 | (S)-N-(5-(2-amino-[1,2,4]triazolo[1,5-a]pyridin-7-yl)-2-methylpyridin-3-yl)-3-(4-cyanophenyl) isoxazolidine-2-carboxamide | $^1H$ NMR (400 MHz, DMSO-$d_6$) δ = 9.27 (s, 1H), 8.75 (d, *J* = 2.2 Hz, 1H), 8.61 (d, *J* = 7.0 Hz, 1H), 8.15 (d, *J* = 2.2 Hz, 1H), 7.91 - 7.79 (m, 2H), 7.70 (d, *J* = 2.0 Hz, 1H), 7.61 (dd, *J* = 8.3, 1.8 Hz, 2H), 7.22 (dd, *J* = 7.0, 2.0 Hz, 1H), 6.07 (s, 2H), 5.51 (dd, *J* = 8.7, 5.9 Hz, 1H), 4.36 - 4.25 (m, 1H), 4.02 (q, *J* = 8.1 Hz, 1H), 3.01 - 2.90 (m, 1H), 2.49 (s, 3H), 2.30 - 2.19 (m, 1H) | 441.2 |

(continued)

| Example | Structure | Name of Compound | $^1H$ NMR | LC-MS $[M+H]^+$ |
|---|---|---|---|---|
| 71 | 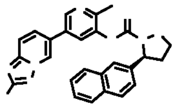 | (S)-N-(5-(2-amino-[1,2,4]triazolo[1,5-a]pyridin-6-yl)-2-methylpyridin-3-yl)-3-(naphthalen-2-yl) isoxazolidine-2-carboxamide | $^1H$ NMR (400 MHz, DMSO-$d_6$) δ = 9.22 (s, 1H), 8.97 (d, *J* = 1.8 Hz, 1H), 8.67 (d, *J* = 2.2 Hz, 1H), 8.11 (d, *J* = 2.2 Hz, 1H), 7.93 (ddt, *J* = 8.8, 5.2, 3.1 Hz, 4H), 7.77 (dd, *J* = 9.2, 1.9 Hz, 1H), 7.57 (dd, *J* = 8.5, 1.8 Hz, 1H), 7.54 - 7.48 (m, 2H), 7.45 (d, *J* = 9.1 Hz, 1H), 6.10 (s, 2H), 5.59 (dd, *J* = 8.6, 5.8 Hz, 1H), 4.36 (td, *J* = 7.9, 2.9 Hz, 1H), 4.06 (dt, *J* = 9.2, 7.4 Hz, 1H), 2.98 (ddt, *J* = 12.7, 9.1, 4.3 Hz, 1H), 2.49 (s, 3H), 2.41 - 2.32 (m, 1H) | 466.2 |

(continued)

| Example | Structure | Name of Compound | $^1$H NMR | LC-MS $[M+H]^+$ |
|---|---|---|---|---|
| 72 | 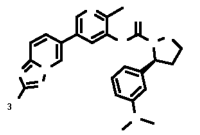 | (S)-N-(5-(2-amino-[1,2,4]triazolo[1,5-a]pyridin-6-yl)-2-methylpyridin-3-yl)-3-(3-(dimethylamino) phenyl)isoxazolidine-2-carboxamide | $^1$H NMR (400 MHz, DMSO-$d_6$) $\delta$ = 9.14 (s, 1H), 8.97 - 8.94 (m, 1H), 8.66 (d, *J* = 2.2 Hz, 1H), 8.09 (d, *J* = 2.3 Hz, 1H), 7.77 (dd, *J* = 9.2, 1.9 Hz, 1H), 7.45 (d, *J* = 9.1 Hz, 1H), 7.16 (t, *J* = 7.9 Hz, 1H), 6.73 (t, *J* = 2.1 Hz, 1H), 6.69 (*d, J* = 7.5 Hz, 1H), 6.62 (dd, *J* = 8.1, 2.6 Hz, 1H), 6.10 (s, 2H), 5.34 (dd, *J* = 8.6, 5.6 Hz, 1H), 428 (td, *J* = 7.9, 3.2 Hz, 1H), 3.98 (dt, *J* = 9.3, 7.5 Hz, 1H), 2.90 (s, 7H), 2.47 (s, 3H), 2.29 - 220 (m, 1H) | 459.2 |

(continued)

| Example | Structure | Name of Compound | $^1$H NMR | LC-MS $[M+H]^+$ |
|---|---|---|---|---|
| 73 | 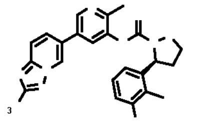 | (S)-N-(5-(2-amino-[1,2,4]triazolo[1,5-a]pyridin-6-yl)-2-methylpyridin-3-yl)-3-(3-chloro-2-methylphenyl)isoxazolidine-2-carboxamide | $^1$H NMR (400 MHz, DMSO-$d_6$) δ = 923 (s, 1H), 8.96 (dd, *J* = 2.0, 0.9 Hz, 1H), 8.67 (d, *J* = 2.2 Hz, 1H), 8.08 (d, *J* = 2.2 Hz, 1H), 7.77 (dd, *J*= 9.2, 1.9 Hz, 1H), 7.50 -7.39 (m, 2H), 7.37 (dd, *J* = 8.0, 1.4 Hz, 1H), 7.25 (t, *J* = 7.8 Hz, 1H), 6.10 (s, 2H), 5.60 (dd, *J* = 8.7, 5.6 Hz, 1H), 4.32 (td, *J* = 8.0, 3.0 Hz, 1H), 4.03 (q, *J* = 8.0 Hz, 1H), 3.02 - 2.94 (m, 1H), 2.47 (s, 3H), 2.40 (s, 3H), 2.15 - 2.05 (m, 1H) | 464.2 |

(continued)

| Example | Structure | Name of Compound | $^1H$ NMR | LC-MS $[M+H]^+$ |
|---|---|---|---|---|
| 74 | 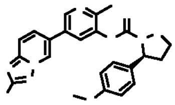 | (S)-N-(5-(2-amino-[1,2,4]triazolo[1,5-a]pyridin-6-yl)-2-methylpyridin-3-yl)-3-(4-methoxyphenyl) isoxazolidine-2-carboxamide | $^1H$ NMR (400 MHz, DMSO-$d_6$) $\delta$ = 9.16 (s, 1H), 8.98 (d, *J* = 1.8 Hz, 1H), 8.68 (d, *J* = 2.2 Hz, 1H), 8.12 (d, *J* = 2.3 Hz, 1H), 7.79 (dd, *J* = 9.2, 1.9 Hz, 1H), 7.46 (d, *J* = 9.1 Hz, 1H), 7.34 - 7.28 (m, 2H), 6.95 - 6.90 (m, 2H), 6.14 (s, 2H), 5.36 (dd, *J* = 8.5, 5.6 Hz, 1H), 4.29 (td, *J* = 8.0, 3.3 Hz, 1H), 4.00 (dt, *J* = 9.3, 7.5 Hz, 1H), 3.75 (s, 3H), 2.89 - 2.78 (m, 1H), 2.47 (s, 3H), 2.31 - 220 (m, 1H) | 446.2 |

(continued)

| Example | Structure | Name of Compound | $^1$H NMR | LC-MS [M+H]$^+$ |
|---|---|---|---|---|
| 75 | 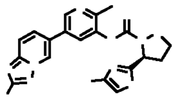 | (S)-N-(5-(2-amino-[1,2,4]triazolo[1,5-a]pyridin-6-yl)-2-methylpyridin-3-yl)-3-(4-(trifluoromethyl) thiazole-2-yl)isoxazolidine-2-carboxamide | $^1$H NMR (400 MHz, DMSO-$d_6$) δ = 9.46 (s, 1H), 8.99 (d, *J* = 1.8 Hz, 1H), 8.71 (d, *J* = 2.2 Hz, 1H), 8.48 (d, *J* = 1.1 Hz, 1H), 8.09 (d, *J* = 2.2 Hz, 1H), 7.79 (dd, *J* = 9.1, 1.9 Hz, 1H), 7.49 - 7.42 (m, 1H), 6.11 (s, 2H), 5.75 (dd, *J* = 8.8, 5.0 Hz, 1H), 4.35 (td, *J* = 8.1, 3.2 Hz, 1H), 4.06 (q, *J* = 8.2 Hz, 1H), 3.01 - 2.91 (m, 1H), 2.66 (dtd, *J* = 13.1, 8.6, 5.0 Hz, 1H), 2.48 (s, 3H) | 491.1 |

(continued)

| Example | Structure | Name of Compound | $^1$H NMR | LC-MS $[M+H]^+$ |
|---|---|---|---|---|
| 76 | 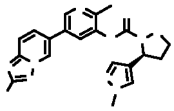 | (S)-N-(5-(2-amino-[1,2,4]triazolo[1,5-a]pyridin-6-yl)-2-methylpyridin-3-yl)-3-(1-methyl-1H-pyrazol-4-yl)isoxazolidine-2-carboxamide | $^1$H NMR (400 MHz, DMSO-$d_6$) δ = 9.09 (s, 1H), 8.96 (dd, *J* = 2.0, 0.9 Hz, 1H), 8.66 (d, *J* = 2.3 Hz, 1H), 8.07 (d, *J* = 2.4 Hz, 1H), 7.77 (dd, *J* = 9.2, 1.9 Hz, 1H), 7.66 (s, 1H), 7.46 (dd, *J* = 9.1, 0.8 Hz, 1H), 7.39 (s, 1H), 6.10 (s, 2H), 5.36 (dd, *J* = 8.2, 4.6 Hz, 1H), 4.24 (d, *J* = 1.7 Hz, 1H), 4.03 (q, *J* = 8.0 Hz, 1H), 3.80 (s, 3H), 2.65 (ddd, *J* = 12.5, 8.2, 4.6 Hz, 1H), 2.45 (s, 3H), 232 (dt, *J* = 12.3, 3.7 Hz, 1H) | 420.1 |

(continued)

| Example | Structure | Name of Compound | $^1$H NMR | LC-MS $[M+H]^+$ |
|---|---|---|---|---|
| 77 | 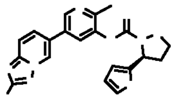 | (S)-N-(5-(2-amino-[1,2,4]triazolo[1,5-a]pyridin-6-yl)-2-methylpyridin-3-yl)-3-(furan-2-yl) isoxazolidine-2-carboxamide | $^1$H NMR (400 MHz, DMSO-$d_6$) $\delta$ = 9.18 (s, 1H), 8.97 (s, 1H), 8.67 (d, *J* = 2.2 Hz, 1H), 8.06 (d, *J* = 2.2 Hz, 1H), 7.77 (dt, *J* = 9.1, 1.5 Hz, 1H), 7.64 (s, 1H), 7.45 (d, *J* = 9.1 Hz, 1H), 6.46 - 6.36 (m, 2H), 6.10 (s, 2H), 5.48 (dd, *J* = 8.8, 5.0 Hz, 1H), 4.33 (td, *J* = 8.4, 3.5 Hz, 1H), 3.99 (q, *J* = 8.2 Hz, 1H), 330 (s, 1H), 2.73 - 2.66 (m, 1H), 2.45 (s, 3H) | 406.1 |

(continued)

| Example | Structure | Name of Compound | $^1$H NMR | LC-MS $[M+H]^+$ |
|---|---|---|---|---|
| 78 | 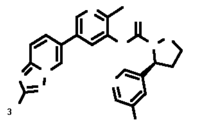 | (S)-N-(5-(2-amino-[1,2,4]triazolo[1,5-a]pyridin-6-yl)-2-methylpyridin-3-yl)-3-(5-fluoropyridin-3-yl) isoxazolidine-2-carboxamide | $^1$H NMR (400 MHz, DMSO-$d_6$) δ = 9.29 (s, 1H), 8.97 (d, *J* = 1.9 Hz, 1H), 8.68 (d, *J* = 2.3 Hz, 1H), 8.52 (t, *J* = 2.8 Hz, 2H), 8.09 (d, *J* = 2.2 Hz, 1H), 7.78 (dd, *J* = 9.2, 1.9 Hz, 1H), 7.71 (dt, *J* = 9.9, 2.3 Hz, 1H), 7.46 (d, *J* = 9.1 Hz, 1H), 6.11 (s, 2H), 5.54 (dd, *J* = 8.7, 5.6 Hz, 1H), 4.33 (td, *J* = 7.9, 3.2 Hz, 1H), 4.04 (q, *J* = 8.1 Hz, 1H), 2.93 (dtd, *J* = 11.9, 7.9, 3.2 Hz, 1H), 2.47 (s, 3H), 2.39 - 2.29 (m, 1H) | 435.2 |

(continued)

| Example | Structure | Name of Compound | $^1$H NMR | LC-MS $[M+H]^+$ |
|---|---|---|---|---|
| 79 | 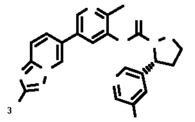 | (R)-N-(5-(2-amino-[1,2,4]triazolo[1,5-a]pyridin-6-yl)-2-methylpyridin-3-yl)-3-(5-fluoropyridin-3-yl) isoxazolidine-2-carboxamide | $^1$H NMR (400 MHz, DMSO-$d_6$) δ = 9.30 (s, 1H), 8.98 (d, *J* = 1.9 Hz, 1H), 8.69 (d, *J* = 2.2 Hz, 1H), 8.52 (d, *J* = 3.1 Hz, 2H), 8.10 (d, *J* = 2.2 Hz, 1H), 7.79 (dd, *J* = 9.2, 1.9 Hz, 1H), 7.71 (dt, *J* = 9.9, 2.4 Hz, 1H), 7.46 (d, *J* = 9.1 Hz, 1H), 6.13 (s, 2H), 5.54 (dd, *J* = 8.7, 5.7 Hz, 1H), 4.33 (td, *J* = 8.0, 3.2 Hz, 1H), 4.04 (q, *J* = 8.1 Hz, 1H), 2.93 (dtd, *J* = 11.6, 7.9, 3.1 Hz, 1H), 2.48 (s, 3H), 2.38 - 2.31 (m, 1H) | 435.2 |

(continued)

| Example | Structure | Name of Compound | $^1$H NMR | LC-MS $[M+H]^+$ |
|---|---|---|---|---|
| 80 | 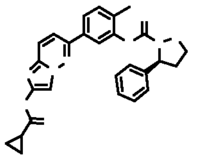 | (S)-N-(5-(2-(cyclopropanecarboxamido)imidazo [1,2-b]pyridazin-6-yl)-2-methylphenyl)-3-phenylisoxazolidine-2-caiboxamide | $^1$H NMR (400 MHz, DMSO-$d_6$) δ = 11.18 (s, 1H), 9.03 (s, 1H), 8.25 (s, 1H), 8.10 - 8.00 (m, 2H), 7.79 (dd, *J* = 8.0, 2.0 Hz, 1H), 7.72 (d, *J* = 9.5 Hz, 1H), 7.43 - 7.34 (m, 5H), 7.30 - 7.25 (m, 1H), 5.42 (dd, *J* = 8.6, 5.6 Hz, 1H), 428 (td, *J* = 8.2, 7.8, 3.6 Hz, 1H), 4.00 (dt, *J* = 9.4, 7.3 Hz, 1H), 2.89 (dddd, *J* = 12.0, 8.6, 7.1, 3.1 Hz, 1H), 2.30 (s, 3H), 226 (tdd, *J* = 7.9, 3.9, 1.8 Hz, 1H), 2.01 - 1.93 (m, 1H), 0.88 - 0.80 (m, 4H) | 483.3 |

(continued)

| Example | Structure | Name of Compound | $^1$H NMR | LC-MS [M+H]$^+$ |
|---|---|---|---|---|
| 81 | 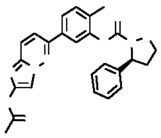 | (S)-N-(5-(2-acetamidoimidazo[1,2-b]pyridazin-6-yl)-2-methylphenyl)-3-phenylisoxazolidine-2-caiboxamide | $^1$H NMR (400 MHz, DMSO-$d_6$) δ = 10.90 (s, 1H), 9.03 (s, 1H), 8.27 (s, 1H), 8.09 (d, *J* = 2.0 Hz, 1H), 8.03 (dd, *J* = 9.4, 0.8 Hz, 1H), 7.79 (dd, *J* = 7.9, 2.1 Hz, 1H), 7.72 (d, *J* = 9.5 Hz, 1H), 7.43 -7.34 (m, 5H), 7.31-7.25 (m, 1H), 5.43 (dd, *J* = 8.6, 5.7 Hz, 1H), 428 (td, *J* = 7.4, 2.8 Hz, 1H), 4.05 - 3.96 (m, 1H), 2.94 - 2.85 (m, 1H), 2.31 (s, 3H), 2.29 - 2.23 (m, 1H), 2.11 (s, 3H) | 457.3 |

(continued)

| Example | Structure | Name of Compound | $^1$H NMR | LC-MS $[M+H]^+$ |
|---|---|---|---|---|
| 82 | 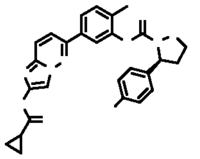 | (S)-N-(5-(2-(cyclopropanecarboxamido)imidazo [1,2-b]pyridazin-6-yl)-2-methylphenyl)-3-(4-fluoropheiryl)isoxazolidine-2-caiboxamide | $^1$H NMR (400 MHz, DMSO-$d_6$) δ = 11.18 (s, 1H), 9.05 (s, 1H), 8.25 (s, 1H), 8.10 - 8.00 (m, 2H), 7.79 (dd, *J* = 7.9, 2.1 Hz, 1H), 7.72 (d, *J* = 9.5 Hz, 1H), 7.48 - 7.35 (m, 3H), 7.25 - 7.13 (m, 2H), 5.43 (dd, *J* = 8.6, 5.8 Hz, 1H), 428 (td, *J* = 7.8, 3.4 Hz, 1H), 4.00 (dt, *J* = 9.4, 7.3 Hz, 1H), 2.88 (dddd, *J* = 12.1, 8.6, 7.2, 3.3 Hz, 1H), 230 (s, 3H), 2.28 - 2.21 (m, 1H), 1.96 (tt, *J* = 7.5, 4.9 Hz, 1H), 0.88 - 0.80 (m, 4H) | 501.3 |

(continued)

| Example | Structure | Name of Compound | $^1H$ NMR | LC-MS $[M+H]^+$ |
|---|---|---|---|---|
| 83 | 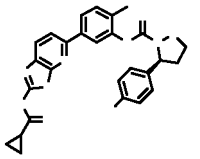 | (S)-N-(5-(2-(cyclopropanecarboxamido)thiazolo [5,4-b]pyridin-5-yl)-2-methylphenyl)-3-(4-fluoropheiryl)isoxazolidine-2-caiboxamide | $^1H$ NMR (400 MHz, DMSO-$d_6$) $\delta$ = 12.80 (s, 1H), 9.00 (s, 1H), 8.18 - 8.10 (m, 2H), 8.00 (d, *J* = 8.6 Hz, 1H), 7.86 (dd, *J* = 8.0, 2.0 Hz, 1H), 7.49 - 7.39 (m, 2H), 7.35 (d, *J* = 8.4 Hz, 1H), 7.23 - 7.14 (m, 2H), 5.43 (dd, *J* = 8.5, 5.6 Hz, 1H), 428 (td, *J* = 7.9, 3.3 Hz, 1H), 4.01 (dt, *J* = 9.4, 7.4 Hz, 1H), 2.88 (dddd, *J* = 12.3, 8.8, 7.3, 3.3 Hz, 1H), 2.30 - 2.19 (m, 4H), 2.03 (tt, *J* = 7.0, 5.3 Hz, 1H), 1.05 - 0.93 (m, 4H) | 518.2 |

(continued)

| Example | Structure | Name of Compound | $^1$H NMR | LC-MS $[M+H]^+$ |
|---|---|---|---|---|
| 84 | 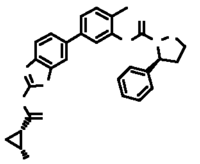 | (S)-N-(5-(2-((1R,2R)-2-fluorocyclopropane-1-carboxamido)benzo[d]thiazol-6-yl)-2-methylphenyl)-3-phenylisoxazolidine-2-carboxamide | $^1$H NMR (400 MHz, DMSO-$d_6$) $\delta$ = 12.73 (s, 1H), 8.94 (s, 1H), 8.25 (d, *J* = 2.0 Hz, 1H), 7.82 - 7.76 (m, 2H), 7.70 (dd, *J* = 8.5, 1.9 Hz, 1H), 7.48 (dd, *J* = 7.9, 2.0 Hz, 1H), 7.43 - 7.24 (m, 6H), 5.42 (dd, *J* = 8.6, 5.8 Hz, 1H), 5.15 - 4.94 (m, 1H), 428 (td, *J* = 8.1, 3.4 Hz, 1H), 4.00 (dt, *J* = 9.4, 7.6 Hz, 1H), 2.93 - 2.83 (m, 1H), 2.31 - 2.19 (m, 5H), 1.75 (dtd, *J* = 23.1, 6.8, 3.8 Hz, 1H), 1.31 (ddd, *J* = 13.0, 6.4, 2.8 Hz, 1H) | 517.3 |

(continued)

| Example | Structure | Name of Compound | $^1$H NMR | LC-MS $[M+H]^+$ |
|---|---|---|---|---|
| 85 | 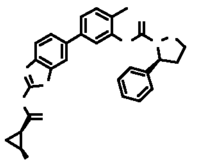 | (S)-N-(5-(2-((1S,2S)-2-fluorocyclopropane-1-carboxamido)benzo[d]thiazol-6-yl)-2-methylphenyl)-3-phenylisoxazolidine-2-carboxamide | $^1$H NMR (400 MHz, DMSO-$d_6$) δ = 12.73 (s, 1H), 8.94 (s, 1H), 8.25 (d, *J* = 2.0 Hz, 1H), 7.82 - 7.76 (m, 2H), 7.70 (dd, *J* = 8.4, 1.9 Hz, 1H), 7.47 (dd, *J* = 7.9, 2.1 Hz, 1H), 7.43 - 7.24 (m, 6H), 5.42 (dd, *J* = 8.6, 5.6 Hz, 1H), 5.04 (dtd, *J* =65.7, 6.1, 3.8 Hz, 1H), 428 (td, *J* = 7.9, 3.3 Hz, 1H), 4.00 (dt, *J* = 9.4, 7.3 Hz, 1H), 2.88 (dtt, *J* = 10.3, 7.1, 3.2 Hz, 1H), 2.31 - 2.18 (m, 5H), 1.75 (dtd, *J* = 23.5, 6.9, 3.8 Hz, 1H), 1.32 (ddd, *J* = 12.9, 8.9, 6.1 Hz, 1H) | 517.3 |

(continued)

| Example | Structure | Name of Compound | $^1$H NMR | LC-MS $[M+H]^+$ |
|---|---|---|---|---|
| 86 | 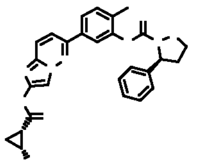 | (S)-N-(5-(2-((1R,2R)-2-fluorocyclopropane-1-carboxamido)imidazo[1,2-b]pyridazin-6-yl)-2-methylphenyl)-3-phenylisoxazolidine-2-carboxamide | $^1$H NMR (400 MHz, DMSO-$d_6$) δ = 11.24 (s, 1H), 9.04 (s, 1H), 8.27 (s, 1H), 8.10 - 8.01 (m, 2H), 7.79 (dd, *J* = 8.0, 2.0 Hz, 1H), 7.73 (d, *J* = 9.5 Hz, 1H), 7.44 - 7.33 (m, 5H), 7.30 - 7.24 (m, 1H), 5.42 (dd, *J* = 8.8, 5.5 Hz, 1H), 5.04 - 4.85 (m, 1H), 428 (td, *J* = 7.8, 3.1 Hz, 1H), 4.04 - 3.96 (m, 1H), 2.94 - 2.84 (m, 1H), 2.30 (s, 4H), 2.20 - 2.12 (m, 1H), 1.73 - 1.63 (m, 1H), 1.22 - 1.15 (m, 1H) | 501.3 |

(continued)

| Example | Structure | Name of Compound | $^1$H NMR | LC-MS $[M+H]^+$ |
|---|---|---|---|---|
| 87 | 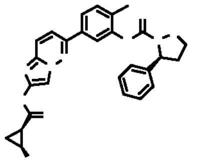 | (S)-N-(5-(2-((1S,2S)-2-fluorocyclopropane-1-carboxamido)imidazo[1,2-b]pyridazin-6-yl)-2-methylphenyl)-3-phenylisoxazolidine-2-carboxamide | $^1$H NMR (400 MHz, DMSO-$d_6$) δ = 11.23 (s, 1H), 9.04 (s, 1H), 8.27 (s, 1H), 8.10 - 8.01 (m, 2H), 7.79 (dd, *J* = 8.0, 2.0 Hz, 1H), 7.73 (d, *J* = 9.5 Hz, 1H), 7.44 - 7.33 (m, 5H), 7.30 - 7.24 (m, 1H), 5.43 (dd, *J* = 8.6, 5.7 Hz, 1H), 5.05 - 4.84 (m, 1H), 428 (td, *J* = 8.0, 3.2 Hz, 1H), 4.01 (dt, *J* = 9.1, 7.4 Hz, 1H), 2.89 (dddd, *J* = 12.0, 8.7, 7.2, 3.2 Hz, 1H), 2.34 - 2.22 (m, 4H), 2.16 (ddd, *J* = 9.2, 6.7, 4.7 Hz, 1H), 1.68 (dtd, *J* = 23.3, 6.7, 3.7 Hz, 1H), 1.19 (ddd, *J* = 12.3, 6.1, 2.9 Hz, 1H) | 501.3 |

(continued)

| Example | Structure | Name of Compound | $^1$H NMR | LC-MS $[M+H]^+$ |
|---|---|---|---|---|
| 88 | 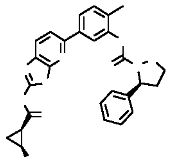 | (S)-N-(5-(2-((1S,2S)-2-fluorocyclopropane-1-carboxamido)thiazolo[5,4-b]pyridin-5-yl)-2-methylphenyl)-3-phenylisoxazolidine-2-carboxamide | $^1$H NMR (400 MHz, DMSO-$d_6$) δ = 12.86 (s, 1H), 8.99 (s, 1H), 8.19 - 8.13 (m, 2H), 8.01 (d, *J* = 8.6 Hz, 1H), 7.86 (dd, *J* = 7.9, 2.0 Hz, 1H), 7.44 - 7.33 (m, 5H), 7.31 - 7.24 (m, 1H), 5.43 (dd, *J* = 8.6, 5.7 Hz, 1H), 5.17 - 4.96 (m, 1H), 428 (td, *J* = 7.9, 3.2 Hz, 1H), 4.01 (dt, *J* = 9.3, 7.4 Hz, 1H), 2.94 - 2.85 (m, 1H), 2.31 - 2.22 (m, 5H), 1.77 (dtd, *J* = 23.3, 6.8, 3.8 Hz, 1H), 1.33 (ddt, *J* = 12.6, 9.1, 6.4 Hz, 1H) | 518.3 |
| 89 | 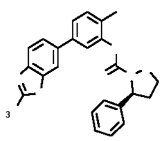 | (S)-N-(5-(2-aminobenzo[d]thiazol-6-yl)-2-methylphenyl)-3-phenylisoxazolidine-2-carboxamide | | 431.2 |
| 90 | 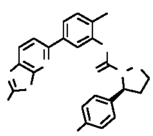 | (S)-N-(5-(2-aminothiazolo[5,4-b]pyridin-5-yl)-2-methylphenyl)-3-(4-fluorophenyl)isoxazolidine-2-carboxamide | | 450.1 |

(continued)

| Example | Structure | Name of Compound | $^1$H NMR | LC-MS $[M+H]^+$ |
|---|---|---|---|---|
| 91 | 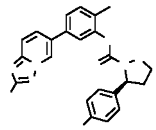 | (S)-N-(5-(2-amino-[1,2,4]triazolo[1,5-a]pyridin-6-yl)-2-methylphenyl)-3-(4-cyanophenyl) isoxazolidine-2-carboxamide | $^1$H NMR (400 MHz, DMSO-$d_6$) δ = 9.05 (s, 1H), 8.81 (dd, *J* = 1.9, 0.9 Hz, 1H), 7.88 - 7.81 (m, 2H), 7.75 - 7.69 (m, 2H), 7.65 - 7.57 (m, 2H), 7.48 (dd, *J* = 7.9, 2.0 Hz, 1H), 7.42 (dd, *J* = 9.1, 0.9 Hz, 1H), 7.32 (d, *J* = 8.0 Hz, 1H), 6.05 (s, 2H), 5.51 (dd, *J* = 8.7, 5.8 Hz, 1H), 428 (td, *J* = 8.0, 3.0 Hz, 1H), 4.00 (q, *J* = 8.2 Hz, 1H), 2.99 - 2.89 (m, 1H), 2.26 (s, 3H), 2.24 - 2.18 (m, 1H) | 440.3 |

(continued)

| Example | Structure | Name of Compound | $^1H$ NMR | LC-MS $[M+H]^+$ |
|---|---|---|---|---|
| 92 | 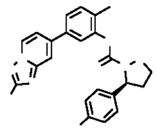 | (S)-N-(5-(2-amino-[1,2,4]triazolo[1,5-a]pyridin-7-yl)-2-methylphenyl)-3-(4-cyanophenyl) isoxazolidine-2-carboxamide | $^1H$ NMR (400 MHz, DMSO-$d_6$) $\delta$ = 9.06 (s, 1H), 8.56 (d, *J* = 7.0 Hz, 1H), 7.88 - 7.77 (m, 3H), 7.65 - 7.52 (m, 4H), 7.35 (d, *J* = 8.0 Hz, 1H), 7.15 (dd, *J* = 7.1, 2.0 Hz, 1H), 6.03 (s, 2H), 5.51 (dd, *J* = 8.7, 5.8 Hz, 1H), 428 (td, *J* = 8.0, 3.0 Hz, 1H), 4.01 (q, *J* = 8.2 Hz, 1H), 2.99 - 2.88 (m, 1H), 2.28 (s, 3H), 2.26 - 2.18 (m, 1H) | 440.2 |

(continued)

| Example | Structure | Name of Compound | $^1H$ NMR | LC-MS $[M+H]^+$ |
|---|---|---|---|---|
| 93 | 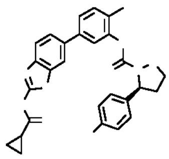 | (S)-3-(4-cyanophenyl)-N-(5-(2-(cyclopropanecarboxamido) benzo[d]thiazol-6-yl)-2-methylphenyl) isoxazolidine-2-carboxamide | $^1H$ NMR (400 MHz, DMSO-$d_6$) $\delta$ = 12.67 (s, 1H), 9.03 (s, 1H), 8.23 (d, $J$ = 1.8 Hz, 1H), 7.88 - 7.82 (m, 2H), 7.80 - 7.73 (m, 2H), 7.69 (dd, $J$ = 8.5, 1.9 Hz, 1H), 7.64 - 7.58 (m, 2H), 7.48 (dd, $J$ = 7.9, 2.0 Hz, 1H), 7.32 (d, $J$ = 8.0 Hz, 1H), 5.51 (dd, $J$ = 8.7, 5.8 Hz, 1H), 428 (td, $J$ = 7.9, 3.0 Hz, 1H), 4.05 - 3.96 (m, 1H), 2.99 - 2.89 (m, 1H), 2.27 (s, 3H), 2.25 - 2.18 (m, 1H), 2.01 (tt, $J$ = 7.0, 4.2 Hz, 1H), 1.02 - 0.90 (m, 4H) | 524.2 |

(continued)

| Example | Structure | Name of Compound | $^1$H NMR | LC-MS $[M+H]^+$ |
|---|---|---|---|---|
| 94 | 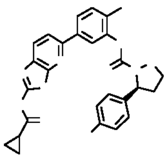 | (S)-3-(4-cyanophenyl)-N-(5-(2-(cyclopropanecarboxamido) thiazolo[5,4-b]pyridin-5-yl)-2-methylphenyl) isoxazolidine-2-carboxamide | $^1$H NMR (400 MHz, DMSO-$d_6$) $\delta$ = 12.78 (s, 1H), 9.08 (s, 1H), 8.17 - 8.10 (m, 2H), 8.00 (d, *J* = 8.6 Hz, 1H), 7.89 - 7.82 (m, 3H), 7.65 - 7.58 (m, 2H), 7.35 (d, *J* = 8.1 Hz, 1H), 5.52 (dd, *J* = 8.7, 5.8 Hz, 1H), 428 (td, *J* = 7.9, 3.0 Hz, 1H), 4.01 (q, *J* = 8.2 Hz, 1H), 3.00 - 2.90 (m, 1H), 2.28 (s, 3H), 2.26 - 2.19 (m, 1H), 2.03 (p, *J* = 6.4 Hz, 1H), 1.02 - 0.95 (m, 4H) | 525.2 |

(continued)

| Example | Structure | Name of Compound | $^1H$ NMR | LC-MS $[M+H]^+$ |
|---|---|---|---|---|
| 95 | 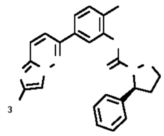 | (S)-N-(5-(2-aminoimidazo[1,2-b]pyridazin-6-yl)-2-methylphenyl)-3-phenylisoxazolidine-2-caiboxamide | $^1H$ NMR (400 MHz, DMSO-$d_6$) $\delta$ = 8.97 (s, 1H), 7.77 (d, $J$ = 2.0 Hz, 1H), 7.59 - 7.54 (m, 1H), 7.45 - 7.32 (m, 6H), 7.31 - 7.20 (m, 3H), 5.44 (dq, $J$ = 12.4, 6.7, 6.1 Hz, 1H), 4.27 (td, $J$ = 7.8, 3.4 Hz, 1H), 4.10 - 3.98 (m, 1H), 2.97 - 2.87 (m, 1H), 2.31 - 2.23 (m, 4H), 2.14 (s, 2H) | 415.3 |

(continued)

| Example | Structure | Name of Compound | $^1$H NMR | LC-MS $[M+H]^+$ |
|---|---|---|---|---|
| 96 | 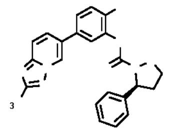 | (S)-N-(5-(2-amino-[1,2,4]triazolo[1,5-a]pyridin-6-yl)-2-fluorophenyl)-3-phenylisoxazolidine-2-caiboxamide | $^1$H NMR (400 MHz, DMSO-$d_6$) $\delta$ = 9.08 (s, 1H), 8.83 (dd, *J* = 2.0, 0.9 Hz, 1H), 7.95 (dd, *J* = 7.4, 2.4 Hz, 1H), 7.70 (dd, *J* = 9.1, 1.9 Hz, 1H), 7.55 (ddd, *J* = 8. 6, 4.6, 2.5 Hz, 1H), 7.45 - 7.32 (m, 6H), 7.30 - 7.24 (m, 1H), 6.08 (s, 2H), 5.40 (dd, *J* = 8.6, 5.8 Hz, 1H), 428 (td, *J* = 7.9, 3.1 Hz, 1H), 3.96 (dt, *J* = 9.2, 7.3 Hz, 1H), 2.89 (dddd, *J* = 11.9, 9.0, 6.9, 3.0 Hz, 1H), 2.25 (dddd, *J* = 12.2, 9.6, 7.8, 5.9 Hz, 1H) | 419.2 |

(continued)

| Example | Structure | Name of Compound | $^1$H NMR | LC-MS [M+H]$^+$ |
|---|---|---|---|---|
| 97 | 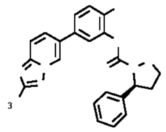 | (S)-N-(5-(2-amino-[1,2,4]triazolo[1,5-a]pyridin-6-yl)-2-chlorophenyl)-3-phenylisoxazolidine-2-caiboxamide | $^1$H NMR (400 MHz, DMSO-$d_6$) δ = 9.08 (s, 1H), 8.83 (dd, *J* = 2.0, 0.9 Hz, 1H), 7.95 (dd, *J* = 7.4, 2.4 Hz, 1H), 7.70 (dd, *J* = 9.1, 1.9 Hz, 1H), 7.55 (ddd, *J* = 8. 6, 4.6, 2.5 Hz, 1H), 7.46 - 7.32 (m, 6H), 7.31 - 7.24 (m, 1H), 6.08 (s, 2H), 5.40 (dd, *J* = 8.6, 5.8 Hz, 1H), | 435.2 |
| | | | 4.28 (td, *J* = 7.9, 3.1 Hz, 1H), 3.96 (dt, *J* = 9.2, 7.3 Hz, 1H), 2.89 (dddd, *J* = 11.9, 9.0, 6.9, 3.0 Hz, 1H), 2.25 (dddd, *J* = 12.2, 9.6, 7.8, 5.9 Hz, 1H) | |

(continued)

| Example | Structure | Name of Compound | $^1$H NMR | LC-MS $[M+H]^+$ |
|---|---|---|---|---|
| 98 | 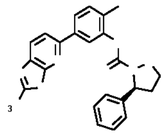 | (S)-N-(5-(2-aminothiazolo[5,4-b]pyridin-5-yl)-2-methylphenyl)-3-phenylisoxazolidine-2-carboxamide | $^1$H NMR (400 MHz, DMSO-$d_6$) δ = 8.94 (s, 1H), 8.08 (d, *J* = 1.9 Hz, 1H), 7.83 (s, 2H), 7.79 - 7.72 (m, 2H ), 7.65 (d, *J* = 8.4 Hz, 1H), 7.43 - 7.34 (m, 4H), 7.3 2 - 7.25 (m, 2H), 5.42 (dd, *J* = 8.6, 5.6 Hz, 1H), 42 7 (td, *J* = 7.9, 3.2 Hz, 1H), 4.04 - 3.95 (m, 1H), 2.94 - 2.83 (m, 1H), 2.27 (s, 4H) | 432.2 |

(continued)

| Example | Structure | Name of Compound | $^1$H NMR | LC-MS $[M+H]^+$ |
|---|---|---|---|---|
| 99 | 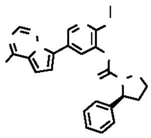 | (S)-N-(5-(4-aminopyrrolo[2,1-f][1,2,4]triazin-7-yl)-2-methoxypyridin-3-yl)-3-phenylisoxazolidine-2-carboxamide | $^1$H NMR (400 MHz, DMSO-$d_6$) δ = 8.90 (d, *J* = 2.2 Hz, 1H), 8.53 - 8.46 (m, 2H), 7.90 (s, 1H), 7.78 (s, 2 H), 7.41 - 7.34 (m, 4H), 7.28 (ddt, *J* = 8.6, 5.9, 2.1 Hz, 1H), 7.02 - 6.93 (m, 2H), 5.40 (dd, *J* = 8.5, 6.1 Hz, 1H), 4.31 (td, *J* = 7.8, 3.1 Hz, 1H), 4.04 - 3.94 ( m, 4H), 2.91 (dddd, *J* = 11.9, 8.5, 6.8, 3.1 Hz, 1H), 2 29 - 220 (m, 1H) | 432.2 |

(continued)

| Example | Structure | Name of Compound | $^1H$ NMR | LC-MS $[M+H]^+$ |
|---|---|---|---|---|
| 100 | 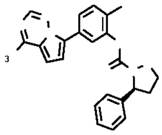 | (S)-N-(5-(4-aminopyrrolo[2,1-f][1,2,4]triazin-7-yl)-2-methylphenyl)-3-phenylisoxazolidine-2-caiboxamide | $^1H$ NMR (400 MHz, DMSO-$d_6$) δ = 8.98 (s, 1H), 8.06 (d, *J* = 1.8 Hz, 1H), 7.91 (s, 1H), 7.83 (dd, *J* = 8.0, 1.9 Hz, 1H), 7.73 (s, 2H), 7.43 - 7.33 (m, 4H), 7.31 - 7.24 (m, 2H), 7.01 - 6.94 (m, 2H), 5.41 (dd, *J* = 8. 7, 5.6 Hz, 1H), 4.27 (td, *J* = 7.9, 3.3 Hz, 1H), 3.98 ( dt, *J* = 9.0, 7.4 Hz, 1H), 2.92 - 2.82 (m, 1H), 2.31 - 2.19 (m, 4H) | 415.2 |

(continued)

| Example | Structure | Name of Compound | $^1$H NMR | LC-MS $[M+H]^+$ |
|---|---|---|---|---|
| 101 | 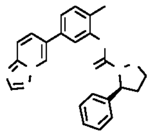 | (S)-N-(5-([1,2,4]triazolo[1,5-a]pyridin-6-yl)-2-methylphenyl)-3-phenylisoxazolidine-2-carboxamide | $^1$H NMR (400 MHz, DMSO-$d_6$) δ = 922 (dd, *J* = 1.9, 1.0 Hz, 1H), 8.98 (s, 1H), 8.49 (s, 1H), 7.95 (dd, *J* = 9.4, 1.8 Hz, 1H), 7.89 (dd, *J* = 9.3, 1.0 Hz, 1H), 7.79 (d, *J* = 2.0 Hz, 1H), 7.53 (dd, *J* = 7.9, 2.1 Hz, 1H), 7.41 - 7.29 (m, 5H), 7.27 - 7.19 (m, 1H), 5.38 (dd, *J* = 8.7, 5.7 Hz, 1H), 424 (td, *J* = 8.0, 3.2 Hz, 1H), 3.96 (dt, *J* = 9.0, 7.4 Hz, 1H), 2.90 - 2.79 (m, 1H), 2.25 (s, 3H), 2.23 - 2.18 (m, 1H) | 400.2 |

(continued)

| Example | Structure | Name of Compound | $^1H$ NMR | LC-MS $[M+H]^+$ |
|---|---|---|---|---|
| 102 | 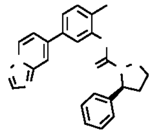 | (S)-N-(5-([1,2,4]triazolo[1,5-a]pyridin-7-yl)-2-methylphenyl)-3-phenylisoxazolidine-2-carboxamide | $^1H$ NMR (400 MHz, DMSO-$d_6$) δ = 9.04 (s, 1H), 9.01 (dd, *J* = 7.2, 0.8 Hz, 1H), 8.52 (s, 1H), 8.10 (dd, *J* = 2.0, 0.9 Hz, 1H), 7.91 (d, *J* = 2.0 Hz, 1H), 7.65 (dd, *J* = 7.9, 2.1 Hz, 1H), 7.52 (dd, *J* = 7.2, 2.0 Hz, 1H), 7.43 - 7.35 (m, 5H), 7.31 - 7.25 (m, 1H), 5.43 (dd, *J* = 8.6, 5.7 Hz, 1H), 4.29 (td, *J* = 7.9, 3.1 Hz, 1H), 4.01 (dt, *J* = 9.1, 7.4 Hz, 1H), 2.89 (dtd, *J* = 11.8, 8.0, 3.1 Hz, 2H), 230 (s, 3H), 2.27 - 2.21 (m, 1H) | 400.2 |

(continued)

| Example | Structure | Name of Compound | $^1$H NMR | LC-MS $[M+H]^+$ |
|---|---|---|---|---|
| 103 | 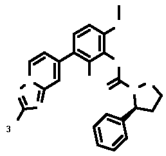 | (S)-N-(3-(2-amino-[1,2,4]triazolo[1,5-a]pyridin-7-yl)-2-fluoro-6-methoxyphenyl)-3-phenylisoxazolidine-2-carboxamide | $^1$H NMR (400 MHz, DMSO-$d_6$) δ = 8.80 (s, 1H), 8.60 (d, *J* = 7.0 Hz, 1H), 7.58 (t, *J* = 8.8 Hz, 1H), 7.45 (d, *J* = 1.9 Hz, 1H), 7.41 - 7.32 (m, 4H), 7.30 - 7.23 (m, 1H), 7.04 (td, *J* = 6.8, 3.3 Hz, 2H), 5.37 (dd, *J* = 8.7, 5.5 Hz, 1H), 425 (td, *J* = 7.9, 3.2 Hz, 1H), 3.96 - 3.83 (m, 4H), 2.86 (ddt, *J* = 12.5, 8.8, 4.1 Hz, 1H), 223 (dtd, *J* = 16.1, 8.1, 4.1 Hz, 1H) | 449.2 |

(continued)

| Example | Structure | Name of Compound | $^1$H NMR | LC-MS $[M+H]^+$ |
|---|---|---|---|---|
| 104 | 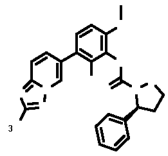 | (S)-N-(3-(2-amino-[1,2,4]triazolo[1,5-a]pyridin-6-yl)-2-fluoro-6-methoxyphenyl)-3-phenylisoxazolidine-2-carboxamide | $^1$H NMR (400 MHz, DMSO-$d_6$) $\delta$ = 8.79 (s, 1H), 8.66 - 8.62 (m, 1H), 7.58 - 7.48 (m, 2H), 7.45 - 7.32 (m, 5H), 7.30 - 7.22 (m, 1H), 7.06 - 7.00 (m, 1H), 6.07 ( s, 2H), 537 (dd, *J* = 8.6, 5.5 Hz, 1H), 4.25 (td, *J* = 7.9, 3.1 Hz, 1H), 3.85 (s, 4H), 2.90 - 2.79 (m, 1H), 2 28 - 2.17 (m, 1H) | 449.2 |

(continued)

| Example | Structure | Name of Compound | $^1H$ NMR | LC-MS $[M+H]^+$ |
|---|---|---|---|---|
| 105 | 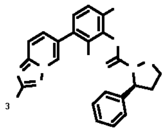 | (S)-N-(3-(2-amino-[1,2,4]triazolo[1,5-a]pyridin-6-yl)-6-chloro-2-fluorophenyl)-3-phenylisoxazolidine-2-caiboxamide | $^1H$ NMR (400 MHz, DMSO-$d_6$) δ = 9.37 (s, 1H), 8.77 - 8.73 (m, 1H), 7.63 - 7.54 (m, 2H), 7.52 - 7.43 (m, 2H), 7.41 - 7.34 (m, 4H), 7.30 - 7.25 (m, 1H), 6.13 (s, 2H), 5.38 (dd, *J* = 8.7, 5.6 Hz, 1H), 430 (td, *J* = 8.0, 3.1 Hz, 1H), 3.95 (q, *J* = 8.2 Hz, 1H), 2.88 (ddd, *J* = 15.5, 11.9, 3.2 Hz, 1H), 225 (dt, *J* = 9.3, 5.3 Hz, 1H) | 453.2 |
| 106 | 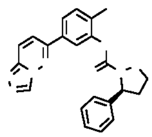 | (S)-N-(5-([1,2,4]triazolo[4,3-b]pyridazin-6-yl)-2-methylphenyl)-3-phenylisoxazolidine-2-carboxamide | | 401.2 |

(continued)

| Example | Structure | Name of Compound | $^1$H NMR | LC-MS $[M+H]^+$ |
|---|---|---|---|---|
| 107 | 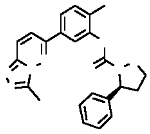 | (S)-N-(2-methyl-5-(3-methyl-[1,2,4]triazolo[4,3-b] pyridazin-6-yl)phenyl)-3-phenylisoxazolidine-2-carboxamide | $^1$H NMR (400 MHz, DMSO-$d_6$) $\delta$ = 9.11 (s, 1H), 8.38 (d, $J$ = 9.8 Hz, 1H), 8.15 (d, $J$ = 1.9 Hz, 1H), 7.91 - 7.84 (m, 2H), 7.45 (d, $J$ = 8.1 Hz, 1H), 7.44 - 7.35 (m, 4H), 7.32 - 7.25 (m, 1H), 5.43 (dd, $J$ = 8.6, 5.7 Hz, 1H), 430 (td, $J$ = 8.0, 3.1 Hz, 1H), 4.01 (dt, $J$ = 9.2, 7.4 Hz, 1H), 2.90 (dddd, $J$ = 12.0, 8.6, 7.1, 3.2 Hz, 1H), 2.33 (s, 3H), 2.26 (dddd, $J$ = 12.1, 9.4, 7.9, 5.6 Hz, 1H) | 415.3 |

(continued)

| Example | Structure | Name of Compound | $^1$H NMR | LC-MS $[M+H]^+$ |
|---|---|---|---|---|
| 108 | 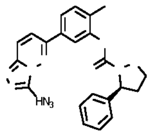 | (S)-N-(2-methyl-5-(3-(trifluoromethyl)-[1,2,4] triazolo[4,3-b]pyridazin-6-yl)phenyl)-3-phenylisoxazolidine-2-carboxamide | $^1$H NMR (400 MHz, DMSO-$d_6$) $\delta$ = 9.15 (s, 1H), 8.66 (d, $J$ = 9.9 Hz, 1H), 8.20 - 8.15 (m, 2H), 7.88 (dd, $J$ = 8.0, 2.0 Hz, 1H), 7.49 (d, $J$ = 8.1 Hz, 1H), 7.45 - 7.35 (m, 4H), 7.32 - 7.25 (m, 1H), 5.43 (dd, $J$ = 8.6, 5.6 Hz, 1H), 430 (td, $J$ = 7.9, 3.2 Hz, 1H), 4.00 (dt, $J$ = 9.1, 7.4 Hz, 1H), 2.90 (dddd, $J$ = 12.1, 8.7, 7.2, 3.2 Hz, 1H), 2.34 (s, 3H), 2.29 - 2.23 (m, 1H) | 469.2 |

(continued)

| Example | Structure | Name of Compound | $^1$H NMR | LC-MS $[M+H]^+$ |
|---|---|---|---|---|
| 109 | 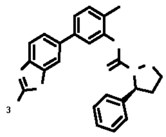 | (S)-N-(5-(2-aminobenzo[d]oxazol-6-yl)-2-methylphenyl)-3-phenylisoxazolidine-2-carboxamide | $^1$H NMR (400 MHz, DMSO-$d_6$) δ = 8.92 (s, 1H), 7.79 (s, 2H), 7.69 (d, *J* = 1.9 Hz, 1H), 7.62 (d, *J* = 1.6 Hz, 1H), 7.43 - 7.33 (m, 6H), 7.27 (dd, *J* = 7.9, 5.3 Hz, 3H), 5.42 (dd, *J* = 8.6, 5.6 Hz, 1H), 4.27 (td, *J* = 7.9, 3.2 Hz, 1H), 3.99 (dt, *J* = 9.2, 7.5 Hz, 1H), 2.87 (dtt, *J* = 10.3, 7.2, 3.2 Hz, 1H), 2.30 - 2.19 (m, 4H) | 415.3 |

(continued)

| Example | Structure | Name of Compound | $^1$H NMR | LC-MS $[M+H]^+$ |
|---|---|---|---|---|
| 110 | 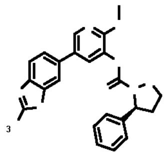 | (S)-N-(5-(2-aminobenzo[d]oxazol-6-yl)-2-methoxypyridin-3-yl)-3-phenylisoxazolidine-2-caiboxamide | $^1$H NMR (400 MHz, DMSO-$d_6$) δ = 8.52 (d, *J* = 2.3 Hz, 1H), 8.46 (s, 1H), 8.17 (d, *J* = 2.3 Hz, 1H), 7.70 (s, 2H), 7.61 (d, *J* = 1.7 Hz, 1H), 7.41 - 7.32 (m, 5H), 7.31 - 7.24 (m, 2H), 539 (dd, *J* = 8.5, 6.2 Hz, 1H), 4.32 (td, *J* = 7.8, 3.0 Hz, 1H), 4.01 (s, 4H), 2.90 (dtt, *J* = 9.4, 6.9, 3.4 Hz, 1H), 2.32 - 2.21 (m, 1H) | 432.2 |

(continued)

| Example | Structure | Name of Compound | $^1$H NMR | LC-MS $[M+H]^+$ |
|---|---|---|---|---|
| 111 | 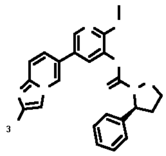 | (S)-N-(5-(2-aminoimidazo[1,2-a]pyridin-6-yl)-2-methoxypyridin-3-yl)-3-phenylisoxazolidine-2-caiboxamide | $^1$H NMR (400 MHz, DMSO-$d_6$) $\delta$ = 8.80 (s, 1H), 8.57 - 8.43 (m, 2H), 8.41 - 8.28 (m, 1H), 820 (dd, *J* = 14.5, 2.4 Hz, 1H), 7.43 - 7.32 (m, 4H), 7.29 (td, *J* = 6.0, 2.7 Hz, 1H), 7.22 (s, 1H), 7.10 (s, 1H), 6.97 (s, 1H), 536 (dd, *J* = 8.5, 6.4 Hz, 1H), 4.93 (s, 1H), 4.33 (td, *J* = 7.8, 2.9 Hz, 1H), 4.07 - 3.90 (m, 4H), 2.94 - 2.86 (m, 1H), 228 (ddt, *J* = 17.8, 12.8, 7.2 Hz, 1H) | 431.2 |

(continued)

| Example | Structure | Name of Compound | $^1H$ NMR | LC-MS $[M+H]^+$ |
|---|---|---|---|---|
| 112 | 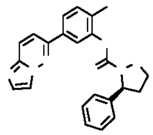 | (S)-N-(5-(imidazo[1,2-b]pyridazin-6-yl)-2-methylphenyl)-3-phenylisoxazolidine-2-carboxamide | $^1H$ NMR (400 MHz, DMSO-$d_6$) δ = 9.02 (s, 1H), 8.34 (t, *J* = 1.0 Hz, 1H), 8.19 (dd, *J* = 9.6, 0.8 Hz, 1H), 8.14 (d, *J* = 1.9 Hz, 1H), 7.83 - 7.73 (m, 3H), 7.44 - 7.34 (m, 5H), 7.31 - 7.25 (m, 1H), 5.42 (dd, *J* = 8.6, 5.8 Hz, 1H), 4.29 (td, *J* = 8.0, 3.2 Hz, 1H), 4.00 (dt, *J* = 9.1, 7.4 Hz, 1H), 2.95 - 2.84 (m, 1H), 2.35 - 2.22 (m, 4H) | 400.3 |

(continued)

| Example | Structure | Name of Compound | $^1H$ NMR | LC-MS $[M+H]^+$ |
|---|---|---|---|---|
| 113 | 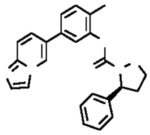 | (S)-N-(5-(imidazo[1,2-a]pyridin-6-yl)-2-methylphenyl)-3-phenylisoxazolidine-2-carboxamide | $^1H$ NMR (400 MHz, DMSO-$d_6$) δ = 8.96 (s, 1H), 8.88 (dd, *J* = 1.9, 1.0 Hz, 1H), 7.97 (t, *J* = 1.0 Hz, 1H), 7.76 (d, *J* = 2.0 Hz, 1H), 7.66 - 7.61 (m, 1H), 7.59 (d, *J* = 1.2 Hz, 1H), 7.53 (dd, *J* = 9.4, 1.9 Hz, 1H), 7.47 - 7.32 (m, 6H), 7.30 - 7.24 (m, 1H), 5.42 (dd, *J* = 8.6, 5.7 Hz, 1H), 4.29 (td, *J* = 8.0, 3.2 Hz, 1H), 3.99 (dt, *J* = 9.2, 7.4 Hz, 1H), 2.88 (dddd, *J* = 12.0, 8.7, 7.1, 3.1 Hz, 1H), 2.31 - 2.21 (m, 4H) | 399.3 |

(continued)

| Example | Structure | Name of Compound | $^1$H NMR | LC-MS $[M+H]^+$ |
|---|---|---|---|---|
| 114 | 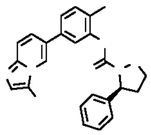 | (S)-N-(5-(3-cyanoimidazo[1,2-a]pyridin-6-yl)-2-methylphenyl)-3-phenylisoxazolidine-2-carboxamide | $^1$H NMR (400 MHz, DMSO-$d_6$) $\delta$ = 9.01 (s, 1H), 8.74 (t, *J* = 1.3 Hz, 1H), 8.47 (s, 1H), 7.95 - 7.87 (m, 2H), 7.84 (d, *J* = 2.0 Hz, 1H), 7.57 (dd, *J* = 7.9, 2.1 Hz, 1H), 7.43 - 7.32 (m, 5H), 7.30 - 7.24 (m, 1H), 5.42 (dd, *J* = 8.6, 5.7 Hz, 1H), 428 (td, *J* = 7.9, 3.1 Hz, 1H), 4.00 (dt, *J* = 9.1, 7.4 Hz, 1H), 2.89 (ddt, *J* = 12.4, 8.8, 4.3 Hz, 1H), 2.29 (s, 4H) | 424.2 |

(continued)

| Example | Structure | Name of Compound | $^1$H NMR | LC-MS $[M+H]^+$ |
|---|---|---|---|---|
| 115 | 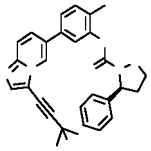 | (S)-N-(5-(3-(3-hydroxy-3-methylbut-1-yn-1-yl) imidazo[1,2-a]pyridin-6-yl)-2-methylphenyl)-3-phenylisoxazolidine-2-carboxamide | $^1$H NMR (400 MHz, DMSO-$d_6$) δ = 8.98 (s, 1H), 8.50 (d, *J* = 1.5 Hz, 1H), 7.92 (s, 1H), 7.82 - 7.76 (m, 2H), 7.72 (dd, *J* = 9.3, 1.8 Hz, 1H), 7.49 (dd, *J* = 7.9, 2.0 Hz, 1H), 7.43 - 7.34 (m, 5H), 7.31 - 7.25 (m, 1H), 5.71 (s, 1H), 5.41 (dd, *J* = 8.6, 5.7 Hz, 1H), 4.29 (td, *J* = 7.9, 3.1 Hz, 1H), 3.99 (q, *J* = 8.1 Hz, 1H), 2.90 - 2.85 (m, 1H), 2.30 (s, 4H), 1.55 (d, *J* = 1.1 Hz, 6H) | 481.3 |

(continued)

| Example | Structure | Name of Compound | $^1$H NMR | LC-MS $[M+H]^+$ |
|---|---|---|---|---|
| 116 | 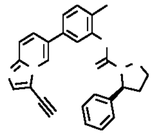 | (S)-N-(5-(3-ethynylimidazo[1,2-a]pyridin-6-yl)-2-methylphenyl)-3-phenylisoxazolidine-2-caiboxamide | $^1$H NMR (400 MHz, DMSO-$d_6$) $\delta$ = 8.99 (s, 1H), 8.50 (dd, $J$ = 1.9, 1.0 Hz, 1H), 7.98 (s, 1H), 7.81 - 7.76 (m, 2H), 7.70 (dd, $J$ = 9.4, 1.9 Hz, 1H), 7.50 (dd, $J$ = 7.9, 2.0 Hz, 1H), 7.43 - 7.34 (m, 5H), 7.30 - 7.24 (m, 1H), 5.42 (dd, $J$ = 8.6, 5.6 Hz, 1H), 5.12 (s, 1H), 4.28 (td, $J$ = 8.0, 3.2 Hz, 1H), 4.00 (q, $J$ = 8.1 Hz, 1H), 2.94 - 2.83 (m, 1H), 2.29 (s, 4H) | 423.2 |

(continued)

| Example | Structure | Name of Compound | $^1$H NMR | LC-MS $[M+H]^+$ |
|---|---|---|---|---|
| 117 | 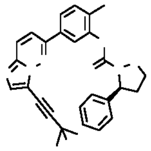 | (S)-N-(5-(3-(3-hydroxy-3-methylbut-1-yn-1-yl) imidazo[1,2-b]pyridazin-6-yl)-2-methylphenyl)-3-phenylisoxazolidine-2-carboxamide | $^1$H NMR (400 MHz, DMSO-$d_6$) $\delta$ = 9.04 (s, 1H), 8.28 - 821 (m, 2H), 8.02 (s, 1H), 7.93 - 7.85 (m, 2H), 7.46 - 7.33 (m, 5H), 7.31 - 7.25 (m, 1H), 5.74 - 5.53 (m, 1H), 5.42 (dd, *J* = 8.6, 5.7 Hz, 1H), 4.29 (td, *J* = 7.9, 3.1 Hz, 1H), 3.98 (dt, *J* = 9.3, 7.4 Hz, 1H), 2.93 - 2.81 (m, 1H), 2.36 - 2.21 (m, 4H), 1.54 (d, *J* = 1.3 Hz, 6H) | 482.2 |

(continued)

| Example | Structure | Name of Compound | $^1$H NMR | LC-MS $[M+H]^+$ |
|---|---|---|---|---|
| 118 | 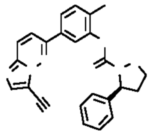 | (S)-N-(5-(3-cyanoimidazo[1,2-b]pyridazin-6-yl)-2-methylphenyl)-3-phenylisoxazolidine-2-caiboxamide | $^1$H NMR (400 MHz, DMSO-$d_6$) $\delta$ = 9.11 (s, 1H), 8.60 (s, 1H), 8.44 (d, $J$ = 9.6 Hz, 1H), 8.15 (d, $J$ = 2.0 Hz, 1H), 8.09 (d, $J$ = 9.6 Hz, 1H), 7.87 (dd, $J$ = 7.9, 2.0 Hz, 1H), 7.47 (d, $J$ = 8.0 Hz, 1H), 7.43 - 7.34 (m, 4H), 7.30 - 7.25 (m, 1H), 5.43 (dd, $J$ = 8.6, 5.7 Hz, 1H), 429 (td, $J$ = 8.0, 3.2 Hz, 1H), 4.01 (dt, $J$ = 9.1, 7.4 Hz, 1H), 2.90 (dddd, $J$ = 12.0, 8.5, 7.1, 3.2 Hz, 1H), 2.37 - 220 (m, 4H) | 425.2 |

(continued)

| Example | Structure | Name of Compound | $^{1}$H NMR | LC-MS $[M+H]^+$ |
|---|---|---|---|---|
| 119 | 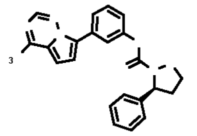 | (S)-N-(3-(4-aminopyrrolo[2,1-f][1,2,4]triazin-7-yl) phenyl)-3-phenylisoxazolidine-2-caiboxamide | $^{1}$H NMR (400 MHz, DMSO-$d_6$) δ = 9.45 (s, 1H), 8.24 (t, $J$ = 1.9 Hz, 1H), 7.92 (s, 1H), 7.76 (dt, $J$ = 7.8, 1.2 Hz, 3H), 7.61 (ddd, $J$ = 8.2, 2.2, 1.1 Hz, 1H), 7.43 - 7.32 (m, 5H), 7.31 - 7.24 (m, 1H), 7.00 (d, $J$ = 4.5 Hz, 1H), 6.93 (d, $J$ = 4.6 Hz, 1H), 5.43 (dd, $J$ = 8.6, 5.8 Hz, 1H), 426 (td, $J$ = 7.9, 3.1 Hz, 1H), 3.97 - 3.84 (m, 1H), 2.86 (dtt, $J$ = 10.1, 7.0, 3.5 Hz, 1H), 2.23 (dddd, $J$ = 12.1, 9.5, 7.9, 5.8 Hz, 1H) | 401.3 |

(continued)

| Example | Structure | Name of Compound | $^1H$ NMR | LC-MS $[M+H]^+$ |
|---|---|---|---|---|
| 120 | 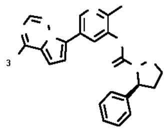 | (S)-N-(5-(4-aminopyrrolo[2,1-f][1,2,4]triazin-7-yl)-2-methylpyridin-3-yl)-3-phenylisoxazolidine-2-carboxamide | $^1H$ NMR (400 MHz, DMSO-$d_6$) $\delta$ = 9.21 (s, 1H), 8.94 (d, $J$ = 2.1 Hz, 1H), 8.43 (d, $J$ = 2.1 Hz, 1H), 7.95 (s, 1H), 7.44 - 7.34 (m, 4H), 7.32 - 7.24 (m, 1H), 7.12 (d, $J$ = 4.6 Hz, 1H), 7.03 (d, $J$ = 4.6 Hz, 1H), 5.41 (dd, $J$ = 8.6, 5.7 Hz, 1H), 430 (td, $J$ = 8.0, 3.0 Hz, 1H), 3.99 (dt, $J$ = 9.2, 7.4 Hz, 1H), 2.90 (dddd, $J$ = 11.9, 9.6, 7.0, 3.0 Hz, 1H), 2.46 (s, 3H), 2.25 (dddd, $J$ = 12.2, 9.5, 8.0, 5.8 Hz, 1H) | 416.3 |

(continued)

| Example | Structure | Name of Compound | $^1H$ NMR | LC-MS $[M+H]^+$ |
|---|---|---|---|---|
| 121 | 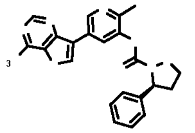 | (S)-N-(5-(4-aminothieno[3,2-d]pyrimidine-7-yl)-2-methylpyridin-3-yl)-3-phenylisoxazolidine-2-caiboxamide | $^1H$ NMR (400 MHz, DMSO-$d_6$) δ = 9.30 (s, 1H), 9.01 (d, J = 2.1 Hz, 1H), 8.54 (d, J = 3.7 Hz, 2H), 8.49 (d, J = 2.0 Hz, 1H), 7.67 (s, 2H), 7.50 - 7.40 (m, 4H), 7.37 - 7.29 (m, 1H), 5.47 (dd, J = 8.6, 5.7 Hz, 1H), 4.37 (td, J = 7.9, 3.0 Hz, 1H), 4.05 (dt, J = 9.5, 7.5 Hz, 1H), 2.96 (dddd, J = 12.0, 8.7, 7.1, 3.1 Hz, 1H), 2.54 (s, 3H), 2.32 (dddd, J = 12.1, 9.5, 7.8, 5.7 Hz, 1H) | 433.2 |

(continued)

| Example | Structure | Name of Compound | $^1$H NMR | LC-MS $[M+H]^+$ |
|---|---|---|---|---|
| 122 | 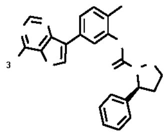 | (S)-N-(5-(4-aminothieno[3,2-d]pyrimidine-7-yl)-2-methylphenyl)-3-phenylisoxazolidine-2-caiboxamide | $^1$H NMR (400 MHz, DMSO-$d_6$) $\delta$ = 9.03 (s, 1H), 8.46 (s, 1H), 830 (s, 1H), 8.05 (d, *J* = 1.8 Hz, 1H), 7.85 (dd, *J* = 7.8, 1.9 Hz, 1H), 7.51 (s, 2H), 7.45 - 7.34 (m, 4H), 7.33 - 7.25 (m, 2H), 5.41 (dd, *J* = 8.6, 5.6 Hz, 1H), 428 (td, *J* = 8.0, 3.2 Hz, 1H), 3.98 (dt, *J* = 9.2, 7.5 Hz, 1H), 2.88 (tq, *J* = 10.4, 3.2 Hz, 1H), 2.27 (s, 3H), 2.26 - 220 (m, 1H) | 432.2 |

(continued)

| Example | Structure | Name of Compound | $^1$H NMR | LC-MS $[M+H]^+$ |
|---|---|---|---|---|
| 123 | 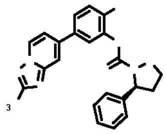 | (S)-N-(5-(2-amino-[1,2,4]triazolo[1,5-a]pyridin-7-yl)-2-fluorophenyl)-3-phenylisoxazolidine-2-caiboxamide | $^1$H NMR (400 MHz, DMSO-$d_6$) δ = 9.10 (s, 1H), 8.59 (d, *J* = 7.0 Hz, 1H), 8.05 (dd, *J* = 7.4, 2.4 Hz, 1H), 7.64 (ddd, *J* = 8.7, 4.6, 2.5 Hz, 1H), 7.58 (d, *J* = 1.9 Hz, 1H), 7.43 - 7.33 (m, 5H), 7.31 - 7.24 (m, 1H), 7 14 (dd, *J* = 7.0, 2.0 Hz, 1H), 6.06 (s, 2H), 5.40 (dd, *J* = 8.6, 5.9 Hz, 1H), 4.29 (td, *J* = 7.9, 3.1 Hz, 1H), 3.97 (dt, *J* = 9.2, 7.2 Hz, 1H), 2.89 (dddd, *J* = 11.9, 8.9, 6.9, 3.0 Hz, 1H), 2.25 (dddd, *J* = 12.1, 9.6, 7.8, 5.8 Hz, 1H) | 419.3 |

(continued)

| Example | Structure | Name of Compound | $^1$H NMR | LC-MS $[M+H]^+$ |
|---|---|---|---|---|
| 124 | 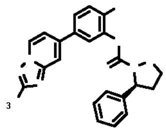 | (S)-N-(5-(2-amino-[1,2,4]triazolo[1,5-a]pyridin-7-yl)-2-chlorophenyl)-3-phenylisoxazolidine-2-caiboxamide | $^1$H NMR (400 MHz, DMSO-$d_6$) $\delta$ = 9.00 (s, 1H), 8.60 (dd, *J* = 6.9, 0.8 Hz, 1H), 8.25 (t, *J* = 1.3 Hz, 1H), 7.66 - 7.57 (m, 3H), 7.44 - 733 (m, 4H), 7.31-7.25 (m, 1H), 7.15 (dd, *J* = 7.0, 2.0 Hz, 1H), 6.07 (s, 2H), 5.41 (dd, *J* = 8.6, 6.0 Hz, 1H), 4.32 (td, *J* = 7.9, 3.0 Hz, 1H), 4.07 - 3.97 (m, 1H), 2.97 - 2.86 (m, 1H), 2.33 - 221 (m, 1H) | 435.2 |

(continued)

| Example | Structure | Name of Compound | $^1$H NMR | LC-MS $[M+H]^+$ |
|---|---|---|---|---|
| 125 | 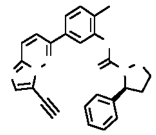 | (S)-N-(5-(3-ethynylimidazo[1,2-b]pyridazin-6-yl)-2-methylphenyl)-3-phenylisoxazolidine-2-caiboxamide | $^1$H NMR (400 MHz, DMSO-$d_6$) δ = 9.10 (s, 1H), 8.27 (d, *J* = 9.6 Hz, 1H), 8.11 (d, *J* = 5.0 Hz, 2H), 7.90 - 7.82 (m, 2H), 7.47 - 733 (m, 5H), 7.31 - 7.24 (m, 1H), 5.43 (dd, *J* = 8.6, 5.6 Hz, 1H), 4.99 (s, 1H), 4.29 (td, *J* = 7.9, 3.2 Hz, 1H), 4.05 - 3.96 (m, 1H), 2.93 - 2.85 (m, 1H), 2.32 (s, 4H) | 424.2 |

(continued)

| Example | Structure | Name of Compound | $^1$H NMR | LC-MS $[M+H]^+$ |
|---|---|---|---|---|
| 126 | 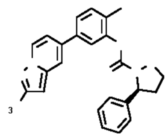 | (S)-N-(5-(2-aminopyrazolo[1,5-a]pyridin-5-yl)-2-methylphenyl)-3-phenylisoxazolidine-2-carboxamide | $^1$H NMR (400 MHz, DMSO-$d_6$) δ = 8.93 (s, 1H), 8.29 (d, $J$ = 7.1 Hz, 1H), 7.77 (d, $J$ = 2.0 Hz, 1H), 7.53 - 7.49 (m, 1H), 7.46 (dd, $J$ = 7.9, 2.0 Hz, 1H), 7.43 - 7.34 (m, 4H), 7.33 - 7.25 (m, 2H), 6.79 (dd, $J$ = 7.2, 2.1 Hz, 1H), 5.67 (d, $J$ = 0.8 Hz, 1H), 5.42 (dd, $J$ = 8.6, 5.7 Hz, 1H), 5.29 (s, 2H), 428 (td, $J$ = 7.9, 3.2 Hz, 1H), 3.99 (dt, $J$ = 9.2, 7.4 Hz, 1H), 2.93 - 2.83 (m, 1H), 2.30 - 220 (m, 4H) | 414.3 |

(continued)

| Example | Structure | Name of Compound | $^1$H NMR | LC-MS $[M+H]^+$ |
|---|---|---|---|---|
| 127 | 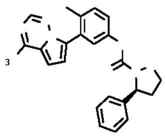 | (S)-N-(3-(4-aminopyrrolo[2,1-f][1,2,4]triazin-7-yl)-4-methylphenyl)-3-phenylisoxazolidine-2-caiboxamide | $^1$H NMR (400 MHz, DMSO-$d_6$) $\delta$ = 9.41 (s, 1H), 7.79 (s, 3H), 7.65 (d, $J$ = 2.3 Hz, 1H), 7.59 (dd, $J$ = 8.3, 2.4 Hz, 1H), 7.41 - 7.32 (m, 4H), 7.31 - 7.24 (m, 1H), 7.21 (d, $J$ = 8.4 Hz, 1H), 7.00 (d, $J$ = 4.4 Hz, 1H), 6.64 (d, $J$ = 4.4 Hz, 1H), 5.39 (dd, $J$ = 8.6, 5.8 Hz, 1H), 423 (td, $J$ = 7.9, 3.1 Hz, 1H), 3.87 (dt, $J$ = 9.1, 7.7 Hz, 1H), 2.84 (dtd, $J$ = 11.7, 8.2, 3.0 Hz, 1H), 2.26 - 2.16 (m, 1H), 2.07 (s, 3H) | 415.3 |
| 128 | 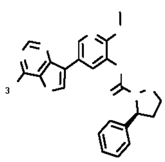 | (S)-N-(5-(4-aminothieno[3,2-d]pyrimidine-7-yl)-2-methoxypyridin-3-yl)-3-phenylisoxazolidine-2-caiboxamide | | 449.2 |

(continued)

| Example | Structure | Name of Compound | $^1H$ NMR | LC-MS $[M+H]^+$ |
|---|---|---|---|---|
| 129 | 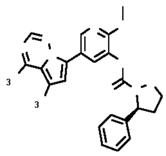 | (S)-N-(5-(4-amino-5-(trifluoromethyl)pyrrolo[2,1-f][1,2,4]triazin-7-yl)-2-methoxypyridin-3-yl)-3-phenylisoxazolidine-2-caiboxamide | $^1H$ NMR (400 MHz, DMSO-$d_6$) δ = 8.90 (d, *J* = 2.2 Hz, 1H), 8.53 (s, 1H), 8.47 (d, *J* = 2.2 Hz, 1H), 8.14 (s, 1H), 7.46 (s, 1H), 7.42 - 7.34 (m, 4H), 7.28 (ddt, *J* = 8.5, 6.0, 2.2 Hz, 1H), 5.40 (dd, *J* = 8.5, 6.2 Hz, 1H), 4.32 (td, *J* = 7.8, 2.9 Hz, 1H), 4.04 (s, 3H), 3.98 (ddd, *J* = 9.5, 8.0, 6.8 Hz, 1H), 2.91 (dddt, *J* = 12.0, 8.9, 6.9, 2.9 Hz, 1H), 2.30 - 2.21 (m, 1H) | 500.2 |

(continued)

| Example | Structure | Name of Compound | $^1H$ NMR | LC-MS $[M+H]^+$ |
|---|---|---|---|---|
| 130 | 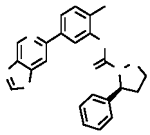 | (S)-N-(2-methyl-5-(thiazolo[4,5-c]pyridin-6-yl) phenyl)-3-phenylisoxazolidine-2-carboxamide | $^1H$ NMR (400 MHz, DMSO-$d_6$) $\delta$ = 9.49 (s, 1H), 9.40 (s, 1H), 8.99 (s, 1H), 8.79 (s, 1H), 8.25 (d, *J* = 1.9 Hz, 1H), 790 (dd, *J* = 7.9, 2.0 Hz, 1H), 7.43 - 7.36 (m, 5H), 7.31 - 7.25 (m, 1H), 5.43 (dd, *J* = 8.6, 5.7 Hz, 1H), 4.29 (td, *J* = 7.9, 32 Hz, 1H), 4.00 (dt, *J* = 9.3, 7.5 Hz, 1H), 2.93 - 2.85 (m, 1H), 2.31 - 2.23 (m, 4H) | 417.2 |

(continued)

| Example | Structure | Name of Compound | $^1$H NMR | LC-MS $[M+H]^+$ |
|---|---|---|---|---|
| 131 | 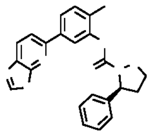 | (S)-N-(2-methyl-5-(thiazolo[5,4-b]pyridin-5-yl) phenyl)-3-phenylisoxazolidine-2-carboxamide | $^1$H NMR (400 MHz, DMSO-$d_6$) δ = 9.53 (s, 1H), 9.01 (s, 1H), 8.52 (d, *J* = 8.6 Hz, 1H), 8.24 (d, *J* = 1.9 Hz, 1H), 8.14 (d, *J* = 8.6 Hz, 1H), 7.91 (dd, *J* = 7.9, 2.0 Hz, 1H), 7.43 - 7.35 (m, 5H), 7.31 - 7.25 (m, 1H), 5.43 (dd, *J* = 8.6, 5.7 Hz, 1H), 4.29 (td, *J* = 8.0, 3.2 Hz, 1H), 4.05 - 3.97 (m, 1H), 2.89 (dq, *J* = 8.6, 4.8, 4.1 Hz, 1H), 230 (s, 4H) | 417.2 |

(continued)

| Example | Structure | Name of Compound | $^1H$ NMR | LC-MS $[M+H]^+$ |
|---|---|---|---|---|
| 132 | 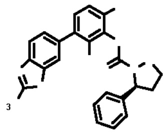 | (S)-N-(3-(2-aminobenzo[d]thiazol-6-yl)-2,6-difluorophenyl)-3-phenylisoxazolidine-2-carboxamide | $^1H$ NMR (400 MHz, DMSO-$d_6$) $\delta$ = 9.25 (s, 1H), 8.12 (s, 2H), 7.88 (s, 1H), 7.51 - 7.43 (m, 2H), 7.42 - 7.34 (m, 5H), 7.30 - 7.22 (m, 2H), 537 (dd, *J* = 8.7, 5.7 Hz, 1H), 4.29 (td, *J* = 7.9, 3.1 Hz, 1H), 3.92 - 3.83 (m, 1H), 2.92 - 2.83 (m, 1H), 2.30 - 2.19 (m, 1H) | 453.2 |

(continued)

| Example | Structure | Name of Compound | $^1$H NMR | LC-MS $[M+H]^+$ |
|---|---|---|---|---|
| 133 | 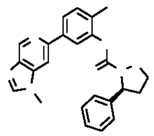 | (S)-N-(2-methyl-5-(1-methyl-1H-imidazo[4,5-c] pyridin-6-yl)phenyl)-3-phenylisoxazolidine-2-caiboxamide | $^1$H NMR (400 MHz, DMSO-$d_6$) $\delta$ = 9.01 - 8.95 (m, 2H), 833 (s, 1H), 8.21 (d, *J* = 1.9 Hz, 1H), 8.17 (d, *J* = 1.1 Hz, 1H), 7.92 (dd, *J* = 8.0, 1.9 Hz, 1H), 7.45 - 7.31 (m, 5H), 7.31 - 7.23 (m, 1H), 5.43 (dd, *J* = 8.6, 5.7 Hz, 1H), 428 (td, *J* = 8.0, 3.2 Hz, 1H), 4.00 (dt, *J* = 9.3, 7.5 Hz, 1H), 3.92 (s, 3H), 2.89 (dtd, *J* = 11.7, 8.3, 7.8, 3.2 Hz, 1H), 2.28 (s, 4H) | 414.3 |

(continued)

| Example | Structure | Name of Compound | $^1H$ NMR | LC-MS $[M+H]^+$ |
|---|---|---|---|---|
| 134 | 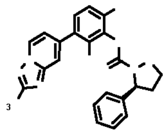 | (S)-N-(3-(2-amino-[1,2,4]triazolo[1,5-a]pyridin-7-yl)-2,6-difluorophenyl)-3-phenylisoxazolidine-2-caiboxamide | $^1H$ NMR (400 MHz, DMSO-$d_6$) $\delta$ = 9.31 (s, 1H), 8.66 (d, *J* = 6.9 Hz, 1H), 7.64 (td, *J* = 8.6, 5.9 Hz, 1H), 7.52 (s, 1H), 7.42 - 7.23 (m, 6H), 7.08 (d, *J* = 6.9 Hz, 1H), 5.37 (dd, *J* = 8.7, 5.7 Hz, 1H), 430 (td, *J* = 8.0, 3.1 Hz, 1H), 3.89 (q, *J* = 8.0 Hz, 1H), 2.88 (dtd, *J* = 11.5, 7.9, 3.0 Hz, 1H), 2.24 (ddd, *J* = 13.3, 10.7, 6.8 Hz, 1H) | 437.2 |

(continued)

| Example | Structure | Name of Compound | $^1$H NMR | LC-MS $[M+H]^+$ |
|---|---|---|---|---|
| 135 | 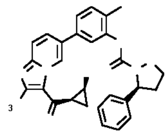 | (S)-N-(5-(2-amino-3-((1S,2S)-2-fluorocyclopropane-1-carbonyl)imidazo[1,2-a]pyridin-6-yl)-2-methylphenyl)-3-phenylisoxazolidine-2-carboxamide | $^1$H NMR (400 MHz, DMSO-$d_6$) $\delta$ = 9.84 (s, 1H), 8.98 (s, 1H), 7.79 (dd, *J* = 9.1, 2.0 Hz, 1H), 7.71 (d, *J* = 1.9 Hz, 1H), 7.47 - 7.32 (m, 7H), 7.30 - 7.24 (m, 1H), 6.66 (s, 2H), 5.43 (dd, *J* = 8.6, 5.6 Hz, 1H), 5.29 - 5.07 (m, 1H), 427 (td, *J* = 7.9, 3.2 Hz, 1H), 4.00 (dt, *J* = 9.0, 7.4 Hz, 1H), 2.94 - 2.83 (m, 1H), 2.60 (d, *J* = 8.0 Hz, 1H), 2.28 (s, 4H), 1.88 (dtd, *J* = 23.4, 6.9, 3.3 Hz, 1H), 1.12 (ddt, *J* = 12.4, 9.2, 6.2 Hz, 1H) | 500.3 |

(continued)

| Example | Structure | Name of Compound | $^1H$ NMR | LC-MS $[M+H]^+$ |
|---|---|---|---|---|
| 136 | 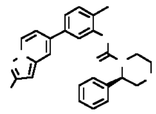 | (R)-N-(5-(2-aminopyrazolo[1,5-a]pyridin-5-yl)-2-methylphenyl)-3-phenylmorpholine-4-carboxamide | $^1H$ NMR (400 MHz, DMSO-$d_6$) δ = 8.29 (d, *J* = 7.1 Hz, 1H), 8.24 (s, 1H), 7.56 (d, *J* = 2.0 Hz, 1H), 7.49 (dd, *J* = 2.2, 0.9 Hz, 1H), 7.45 - 7.36 (m, 5H), 7.31 - 7.23 (m, 2H), 6.78 (dd, *J* = 7.2, 2.1 Hz, 1H), 5.68 (d, *J* = 0.8 Hz, 1H), 5.28 (d, *J* = 9.9 Hz, 3H), 4.38 (d, *J* = 11.9 Hz, 1H), 3.86 (td, *J* = 12.0, 11.5, 3.7 Hz, 3H), 3.56 (td, *J* = 11.6, 3.1 Hz, 1H), 3.19 (td, *J* = 12.9, 3.7 Hz, 1H), 2.13 (s, 3H) | 428.3 |

(continued)

| Example | Structure | Name of Compound | $^1H$ NMR | LC-MS $[M+H]^+$ |
|---|---|---|---|---|
| 137 | 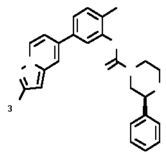 | (S)-N-(5-(2-aminopyrazolo[1,5-a]pyridin-5-yl)-2-methylphenyl)-2-phenylmorpholine-4-carboxamide | $^1H$ NMR (400 MHz, DMSO-$d_6$) $\delta$ = 8.31 - 8.23 (m, 2H), 7.58 (d, *J* = 2.0 Hz, 1H), 7.50 (dd, *J* = 2.1, 0.9 Hz, 1H), 7.46 - 7.36 (m, 5H), 7.35 - 7.30 (m, 1H), 7.28 (d, *J* = 8.0 Hz, 1H), 6.79 (dd, *J* = 7.2, 2.1 Hz, 1H), 5.67 (d, *J* = 0.8 Hz, 1H), 5.29 (s, 2H), 4.50 (dd, *J* = 10.6, 2.6 Hz, 1H), 4.20 - 4.12 (m, 1H), 4.08 - 3.99 (m, 2H), 3.67 (td, *J* = 11.7, 2.8 Hz, 1H), 3.09 - 3.00 (m, 1H), 2.85 (dd, *J* = 13.1, 10.6 Hz, 1H), 2.22 (s, 3H) | 428.3 |

(continued)

| Example | Structure | Name of Compound | $^1$H NMR | LC-MS $[M+H]^+$ |
|---|---|---|---|---|
| 138 | 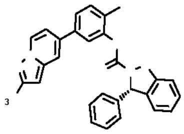 | (S)-N-(5-(2-aminopyrazolo[1,5-a]pyridin-5-yl)-2-methylphenyl)-3-phenylbenzo [d]isooxazole-2 (3H)-carboxamide | $^1$H NMR (400 MHz, DMSO-$d_6$) δ = 9.37 (s, 1H), 8.27 (d, *J* = 7.1 Hz, 1H), 7.65 (d, *J* = 2.0 Hz, 1H), 7.51 (dd, *J* = 8.0, 2.0 Hz, 2H), 7.45 - 7.28 (m, 8H), 7.16 (d, *J* = 8.0 Hz, 1H), 7.09 (td, *J* = 7.5, 0.9 Hz, 1H), 6.80 (dd, *J* = 7.2, 2.1 Hz, 1H), 6.63 (s, 1H), 5.66 (d, *J* = 0.8 Hz, 1H), 5.29 (s, 2H), 2.17 (s, 3H) | 462.3 |

(continued)

| Example | Structure | Name of Compound | $^1H$ NMR | LC-MS $[M+H]^+$ |
|---|---|---|---|---|
| 139 | 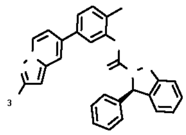 | (R)-N-(5-(2-aminopyrdzolo[1,5-a]pyridin-5-yl)-2-methylphenyl)-3-phenylbenzo[d]isoxazole-2 (3H)-carboxamide | $^1H$ NMR (400 MHz, DMSO-$d_6$) $\delta$ = 9.37 (s, 1H), 8.27 (d, *J* = 7.2 Hz, 1H), 7.65 (d, *J* = 2.0 Hz, 1H), 7.51 (dd, *J* = 7.9, 2.0 Hz, 2H), 7.45 - 7.27 (m, 8H), 7.16 (d, *J* = 8.0 Hz, 1H), 7.12 - 7.07 (m, 1H), 6.80 (dd, *J* = 7.2, 2.1 Hz, 1H), 6.63 (s, 1H), 5.66 (s, 1H), 5.29 (s, 2H), 2.17 (s, 3H) | 462.3 |

(continued)

| Example | Structure | Name of Compound | $^1$H NMR | LC-MS $[M+H]^+$ |
|---|---|---|---|---|
| 140 | 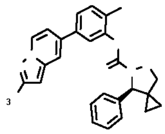 | (R)-N-(5-(2-aminopyrazolo[1,5-a]pyridin-5-yl)-2-methylphenyl)-7-phenyl-5-oxa-6-azaspiro [2.4] heptane-6-carboxamide | $^1$H NMR (400 MHz, DMSO-$d_6$) $\delta$ = 9.04 (s, 1H), 8.29 (d, $J$ = 7.1 Hz, 1H), 7.74 (d, $J$ = 2.0 Hz, 1H), 7.53 - 7.45 (m, 2H), 7.38 - 7.26 (m, 6H), 6.79 (dd, $J$ = 7.2, 2.1 Hz, 1H), 5.67 (d, $J$ = 0.8 Hz, 1H), 5.29 (s, 2H), 5.13 (s, 1H), 4.18 - 4.04 (m, 2H), 2.28 (s, 3H), 0.92 (ddd, $J$ = 10.0, 6.1, 4.3 Hz, 1H), 0.81 (ddd, $J$ = 10.3, 6.1, 4.4 Hz, 1H), 0.77 - 0.70 (m, 1H), 0.26 (dt, $J$ = 10.5, 5.3 Hz, 1H) | 440.3 |

(continued)

| Example | Structure | Name of Compound | $^1$H NMR | LC-MS [M+H]$^+$ |
|---|---|---|---|---|
| 141 | 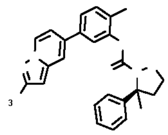 | (S)-N-(5-(2-aminopyrazolo[1,5-a]pyridin-5-yl)-2-methylphenyl)-3-methyl-3-phenylisoxazolidine-2-caiboxamide | $^1$H NMR (400 MHz, DMSO-$d_6$) $\delta$ = 8.59 (s, 1H), 830 (d, *J* = 7.1 Hz, 1H), 7.66 - 7.57 (m, 3H), 7.48 - 7.36 (m, 4H), 7.34 - 7.26 (m, 2H), 6.75 (dd, *J* = 7.2, 2.1 Hz, 1H), 5.69 (d, *J* = 0.8 Hz, 1H), 530 (s, 2H), 3.90 (ddd, *J* = 10.4, 8.1, 6.2 Hz, 1H), 3.60 (ddd, *J* = 10.3, 8.2, 6.1 Hz, 1H), 2.61 - 2.51 (m, 2H), 2.18 (s, 3H), 1.62 (s, 3H) | 428.3 |

(continued)

| Example | Structure | Name of Compound | $^1$H NMR | LC-MS $[M+H]^+$ |
|---|---|---|---|---|
| 142 | 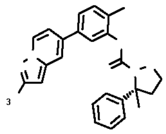 | (R)-N-(5-(2-aminopyrazolo[1,5-a]pyridin-5-yl)-2-methylphenyl)-3-methyl-3-phenylisoxazolidine-2-caiboxamide | $^1$H NMR (400 MHz, DMSO-$d_6$) δ = 8.59 (s, 1H), 830 (d, $J$ = 7.0 Hz, 1H), 7.65 - 7.57 (m, 3H), 7.49 - 7.36 (m, 4H), 7.34 - 7.25 (m, 2H), 6.75 (dd, $J$ = 7.2, 2.1 Hz, 1H), 5.69 (d, $J$ = 0.8 Hz, 1H), 530 (s, 2H), 3.90 (ddd, $J$ = 10.5, 8.1, 6.3 Hz, 1H), 3.61 (ddd, $J$ = 10.4, 8.2, 6.1 Hz, 1H), 2.61 - 2.51 (m, 2H), 2.18 (s, 3H), 1.62 (s, 3H) | 428.3 |

(continued)

| Example | Structure | Name of Compound | $^1H$ NMR | LC-MS $[M+H]^+$ |
|---|---|---|---|---|
| 143 | 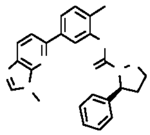 | (S)-N-(2-methyl-5-(3-methyl-3H-imidazo[4,5-b] pyridin-5-yl)phenyl)-3-phenylisoxazolidine-2-caiboxamide | $^1H$ NMR (400 MHz, DMSO-$d_6$) $\delta$ = 9.05 (s, 1H), 8.43 (s, 1H), 8.17 (d, *J* = 1.9 Hz, 1H), 8.12 (d, *J* = 8.4 Hz, 1H), 7.91 (dd, *J* = 7.9, 1.9 Hz, 1H), 7.82 (d, *J* = 8.5 Hz, 1H), 7.39 (ddd, *J* = 19.7, 8.1, 5.7 Hz, 5H), 7.31 - 7.26 (m, 1H), 5.44 (dd, *J* = 8.7, 5.6 Hz, 1H), 429 (td, *J* = 8.0, 3.2 Hz, 1H), 4.01 (dt, *J* = 9.4, 7.5 Hz, 1H), 3.90 (s, 3H), 2.90 (dtd, *J* = 11.4, 7.9, 3.1 Hz, 1H), 2.29 (s, 3H), 2.27 - 2.21 (m, 1H) | 414.3 |

(continued)

| Example | Structure | Name of Compound | $^1H$ NMR | LC-MS $[M+H]^+$ |
|---|---|---|---|---|
| 144 | 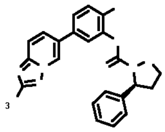 | (S)-N-(5-(2-amino-[1,2,4]triazolo[1,5-a]pyridin-6-yl)-2-cyanophenyl)-3-phenylisoxazolidine-2-caiboxamide | $^1H$ NMR (400 MHz, DMSO-$d_6$) δ = 9.71 (s, 1H), 9.02 (dd, *J* = 2.0, 0.9 Hz, 1H), 8.00 (d, *J* = 1.8 Hz, 1H), 7.90 (d, *J* = 8.2 Hz, 1H), 7.78 (ddd, *J* = 20.8, 8.7, 1. 9 Hz, 2H), 7.49 - 7.45 (m, 1H), 7.43 - 7.35 (m, 4H), 7.32 - 7.26 (m, 1H), 6.19 (s, 2H), 5.43 (dd, *J* = 8.6, 5.8 Hz, 1H), 4.32 (td, *J* = 7.8, 3.0 Hz, 1H), 4.05 - 3. 96 (m, 1H), 2.97 - 2.86 (m, 1H), 2.31 - 2.21 (m, 1H) | 426.3 |
| 145 | 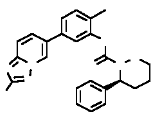 | (S)-N-(5-(2-amino-[1,2,4]triazolo[1,5-a]pyridin-6-yl)-2-methylphenyl)-3-phenyl-1,2-oxazinane-2-carboxamide | | 429.2 |
| 146 | 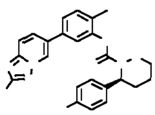 | (S)-N-(5-(2-amino-[1,2,4]triazolo[1,5-a]pyridin-6-yl)-2-methylphenyl)-3-(4-fluorophenyl)-1,2-oxazinane-2-carboxamide | | 447.2 |
| 147 | 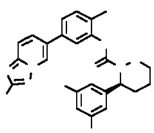 | (S)-N-(5-(2-amino-[1,2,4]triazolo[1,5-a]pyridin-6-yl)-2-methylphenyl)-3-(3,5-difluorophenyl)-1,2-oxazinane-2-carboxamide | | 465.2 |

(continued)

| Example | Structure | Name of Compound | $^1$H NMR | LC-MS [M+H]$^+$ |
|---|---|---|---|---|
| 148 | 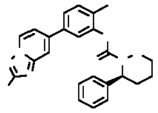 | (S)-N-(5-(2-amino-[1,2,4]triazolo[1,5-a]pyridin-7-yl)-2-methylphenyl)-3-phenyl-1,2-oxazinane-2-carboxamide | | 429.2 |
| 149 | 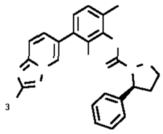 | (S)-N-(3-(2-amino-[1,2,4]triazolo[1,5-a]pyridin-6-yl)-2-fluoro-6-methylphenyl)-3-phenylisoxazolidine-2-caiboxamide | | 433.2 |
| 150 | 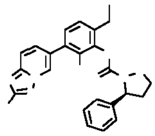 | (S)-N-(3-(2-amino-[1,2,4]triazolo[1,5-a]pyridin-6-yl)-6-ethyl-2-fluorophenyl)-3-phenylisoxazolidine-2-carboxamide | | 447.2 |
| 151 | 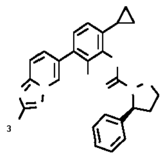 | (S)-N-(3-(2-amino-[1,2,4]triazolo[1,5-a]pyridin-6-yl)-6-cyclopropyl-2-fluorophenyl)-3-phenylisoxazolidine-2-carboxamide | | 459.2 |
| 152 | 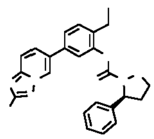 | (S)-N-(5-(2-amino-[1,2,4]triazolo[1,5-a]pyridin-6-yl)-2-ethylphenyl)-3-phenylisoxazolidine-2-caiboxamide | | 429.2 |
| 153 | 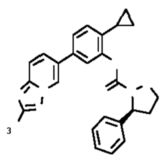 | (S)-N-(5-(2-amino-[1,2,4]triazolo[1,5-a]pyridin-6-yl)-2-cyclopropylphenyl)-3-phenylisoxazolidine-2-caiboxamide | | 441.2 |
| 154 | 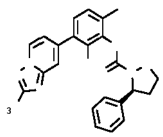 | (S)-N-(3-(2-amino-[1,2,4]triazolo[1,5-a]pyridin-7-yl)-2-fluoro-6-methylphenyl)-3-phenylisoxazolidine-2-caiboxamide | | 433.2 |
| 155 | 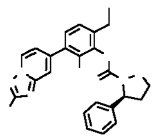 | (S)-N-(3-(2-amino-[1,2,4]triazolo[1,5-a]pyridin-7-yl)-6-ethyl-2-fluorophenyl)-3-phenylisoxazolidine-2-caiboxamide | | 447.2 |
| 156 | 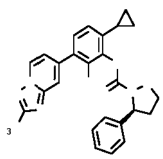 | (S)-N-(3-(2-amino-[1,2,4]triazolo[1,5-a]pyridin-7-yl)-6-cyclopropyl-2-fluorophenyl)-3-phenylisoxazolidine-2-carboxamide | | 459.2 |
| 157 | 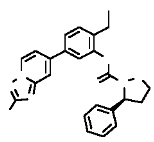 | (S)-N-(5-(2-amino-[1,2,4]triazolo[1,5-a]pyridin-7-yl)-2-ethylphenyl)-3-phenylisoxazolidine-2-caiboxamide | | 429.2 |

(continued)

| Example | Structure | Name of Compound | $^1$H NMR | LC-MS [M+H]+ |
|---|---|---|---|---|
| 158 | 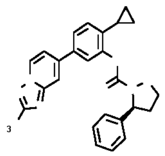 | (S)-N-(5-(2-amino-[1,2,4]triazolo[1,5-a]pyridin-7-yl)-2-cyclopropylphenyl)-3-phenylisoxazolidine-2-caiboxamide | | 441.2 |

**<Experimental Example 1> Evaluation of RIPK1 inhibitory activity of compound according to the present invention**

To evaluate the RIPK1 inhibitory activity of a compound according to the present invention, the following experiment was performed. Specifically, for Examples 3 and 30 selected from the compounds of Examples of the present invention, enzyme (kinase) selectivity was measured by DiscoverX, and the experiment was conducted using a panel for scanMAX™ kinase analysis. Here, the enzyme was treated with a drug having a concentration of 1 μM in DMSO, and a control percentage (% control) was determined in the same manner as Formula 1 below. The results are shown in Table 3 below.

[Formula 1]

(Compound of Example – Positive control)/(Negative control- Positive control) x 100

Here, the positive control refers to a compound showing a control percentage of 0%, and the negative control is DMSO showing a control percentage of 100%. In addition, in terms of the enzyme selectivity of the present invention, when the control percentage with respect to an enzyme is < 35% (i.e., less than 35%), it was determined that the compound has activity against the corresponding enzyme.

[Table 3]

| Kinase | Example 3 | Example 30 |
|---|---|---|
| RIPK1 | 1% | 0% |

As confirmed in Table 3, since compounds of Example according to the present invention have a smaller value than the control percentage (35%) with respect to RIPK1, it can be seen that the compounds have activity against RIPK1. This suggests that there is a useful effect when a compound of Example according to the present invention is used for an RIPKI-related disease, and therefore, it can be effectively used as a composition for treating or preventing an RIPKI-related disease.

**<Experimental Example 2> Evaluation of cell necrosis inhibitory activity of compound according to the present invention under necroptosis-inducing conditions in HT-29 cells**

To evaluate the cell necrosis inhibitory activity of the compound according to the present invention, an experiment was conducted as follows.

To evaluate the efficacy of inhibiting cell necrosis under TNF-α-mediated cell death-inducing conditions, a HT-29 cell line (Korean Cell Line Bank Accession No. #30038) was cultured in RPMI-1640 (HyClone #SH3027.01) medium containing 10% fetal bovine serum (FBS) and 1% penicillin/streptomycin to analyze cell viability. More specifically, for testing, the cell line was seeded in a 96-well flat bottom plate (Corning #3903) at a concentration of 10,000 cells/well and incubated at 37 °C under a 5% $CO_2$ condition for 24 hours. Each well was treated with 100 ng/mL TNF-α, 25 μM pan-caspase inhibitor (Z-VAD-FMK, Selleckchem #S8102), and 10 μM Smac mimetic LCL161 (Selleckchem #S7009) and allowed to react for 10 minutes. The compounds of Examples were treated at 11 concentrations (the highest concentration being 0.1 μM or 1 μM) with a 3-fold concentration gradient, and as a control, dimethyl sulfoxide (DMSO) was treated at a concentration of 0.05% (v/v), which is the same as those when the compounds were treated. The treated cells were incubated for 24 hours. To confirm the degree of cell viability, 100 μL of Cell Titer-Glo (Promega #G7573)

was added to the medium of the cultured cells and cultured for additional 10 minutes, and then luminescence was measured using a microplate reader. Based on the fluorescence of control cells that were not treated with the compound, the degree of cell necrosis inhibitory activity was calculated according to the treatment concentration of each compound. Here, the concentration at which the cell necrosis inhibitory activity was 50% was determined as an $EC_{50}$ (nM) value and calculated using Prism (ver. 8.4.3 #GraphPad) software. The results are summarized in Table 4.

[Table 4]

| Example | HT-29 $EC_{50}$(nM) | Example | HT-29 $EC_{50}$(nM) | Example | HT-29 $EC_{50}$(nM) |
|---|---|---|---|---|---|
| 1 | 0.9 | 54 | 0.4 | 129 | 3.6 |
| 2 | 0.2 | 55 | 0.6 | 130 | 1.4 |
| 3 | 0.6 | 56 | 0.7 | 131 | 4.0 |
| 4 | 0.2 | 57 | 4.4 | 132 | 8.1 |
| 5 | 2.9 | 58 | 3.9 | 133 | 6.5 |
| 6 | 0.9 | 59 | 0.7 | 134 | 13.0 |
| 7 | 40.0 | 60 | 0.4 | 135 | 0.2 |
| 8 | 0.4 | 61 | 0.2 | 136 | 5.4 |
| 10 | 0.3 | 62 | 0.2 | 137 | 49.2 |
| 11 | 0.9 | 63 | 0.2 | 139 | 2.2 |
| 12 | 13.7 | 64 | 0.2 | 140 | 1.7 |
| 13 | 2.6 | 65 | 0.2 | 143 | 5.0 |
| 14 | 0.7 | 66 | 0.4 | | |
| 15 | 23.9 | 67 | 0.4 | | |
| 16 | 4.9 | 68 | 3.9 | | |
| 17 | 0.1 | 69 | 0.4 | | |
| 18 | 0.3 | 70 | 5.2 | | |
| 19 | 1.0 | 91 | 0.9 | | |
| 20 | 0.3 | 92 | 0.6 | | |
| 21 | 0.4 | 93 | 0.2 | | |
| 22 | 0.3 | 94 | 0.3 | | |
| 23 | 8.1 | 95 | 7.5 | | |
| 24 | 0.4 | 96 | 1.9 | | |
| 25 | 0.2 | 97 | 1.1 | | |
| 26 | 0.7 | 98 | 0.3 | | |
| 27 | 1.0 | 99 | 8.2 | | |
| 28 | 0.2 | 100 | 4.6 | | |
| 29 | 0.1 | 101 | 6.2 | | |
| 30 | 3.8 | 102 | 0.9 | | |
| 31 | 4.6 | 103 | 15.6 | | |
| 32 | 0.6 | 105 | 43.8 | | |
| 33 | 1.2 | 106 | 5.3 | | |
| 34 | 0.5 | 107 | 23.2 | | |
| 35 | 3.2 | 108 | 29.8 | | |

(continued)

| Example | HT-29 $EC_{50}$(nM) | Example | HT-29 $EC_{50}$(nM) | Example | HT-29 $EC_{50}$(nM) |
|---|---|---|---|---|---|
| 36 | 0.6 | 109 | 1.0 | | |
| 37 | 1.1 | 110 | 4.0 | | |
| 38 | 1.6 | 112 | 3.3 | | |
| 39 | 0.9 | 113 | 2.4 | | |
| 41 | 1.0 | 114 | 1.9 | | |
| 42 | 0.6 | 115 | 1.7 | | |
| 43 | 7.3 | 116 | 1.0 | | |
| 44 | 4.1 | 117 | 2.4 | | |
| 45 | 4.6 | 118 | 4.3 | | |
| 46 | 19.4 | 120 | 1.0 | | |
| 47 | 0.5 | 121 | 11.7 | | |
| 48 | 0.4 | 122 | 20.4 | | |
| 49 | 1.3 | 123 | 0.3 | | |
| 50 | 1.3 | 124 | 0.2 | | |
| 51 | 23.3 | 125 | 1.4 | | |
| 52 | 2.8 | 126 | 0.4 | | |
| 53 | 0.5 | 128 | 38.2 | | |

## Claims

1. A compound of Chemical Formula 1 below, an isomer thereof, a solvate thereof, a hydrate thereof, or a pharmaceutically acceptable salt thereof:

[Chemical Formula 1]

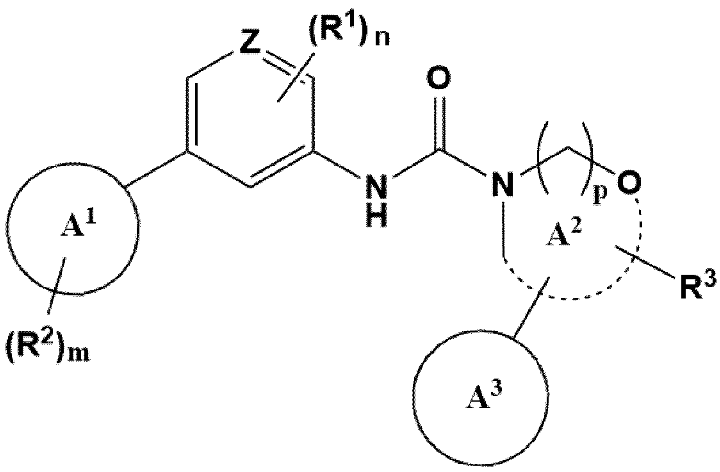

In Chemical Formula 1,

Z is N or CH,
ring $A^1$ is 5- to 12-membered heteroaryl,

ring $A^2$ is 5- to 10-membered heterocycloalkyl, 5- to 10-membered heterocycloalkylaryl bicyclic ring, or 5- to 10-membered heterocycloalkyl-heteroaryl bicyclic ring, wherein p is an integer from 0 to 2,
ring $A^3$ is 4- to 12-membered aryl or 4- to 12-membered heteroaryl, and is unsubstituted or substituted with one or more substituents $R^4$ selected from halogen, cyano, $C_{1-8}$ alkoxy, $C_{1-8}$ alkyl, $C_{1-8}$ haloalkyl, and $C_{1-8}$ alkylamino,
$R^1$ is hydrogen, halogen, $C_{1-8}$ alkyl, $C_{1-8}$ alkoxy, cyano, $C_{3-8}$ cycloalkyl, or $C_{3-8}$ heterocycloalkyl, and n is an integer from 1 to 3,
$R^2$ is halogen, $C_{1-8}$ alkyl, $C_{1-8}$ alkoxy, amine, cyano, $C_{1-8}$ haloalkyl, or $C_{2-8}$ alkynyl that is unsubstituted or substituted with a hydroxyl group,

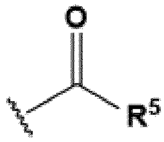, 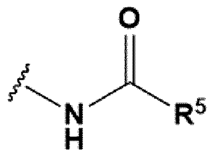,

or

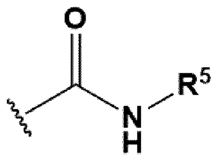,

and m is an integer from 0 to 4,
$R^5$ is $C_{1-8}$ alkyl, $-C_{1-8}$ alkyl-$C_{1-8}$ alkoxy, $-C_{1-8}$ alkyl-hydroxy, or $C_{3-8}$ cycloalkyl, where the $C_{3-8}$ cycloalkyl of $R^5$ is unsubstituted or substituted with $C_{1-8}$ alkylamino or halogen, and
$R^3$ is hydrogen or $C_{1-8}$ alkyl.

**2.** The compound of Chemical Formula 1, the isomer thereof, the solvate thereof, the hydrate thereof, or the pharmaceutically acceptable salt thereof of claim 1, wherein Z is N or CH,

ring $A^1$ is 8- to 10-membered heteroaryl that includes one or more hetero atoms selected from the group consisting of N, O, and S,
ring $A^2$ is 5- to 8-membered heterocycloalkyl or 8- to 10-membered heterocycloalkylaryl bicyclic ring, where p is an integer from 0 to 2,
ring $A^3$ is 5- to 10-membered aryl or 4- to 8-membered heteroaryl including one or more hetero atoms selected from N, O, and S, and is unsubstituted or substituted with one or more substituents $R^4$ selected from halogen, cyano, $C_{1-6}$ alkoxy, $C_{1-6}$ alkyl, $C_{1-6}$ haloalkyl, and $C_{1-6}$alkylamino,
$R^1$ is hydrogen, halogen, $C_{1-6}$ alkyl, $C_{1-6}$ alkoxy, cyano, or $C_{3-6}$ cycloalkyl, and n is an integer from 1 to 3,
$R^2$ is halogen, $C_{1-6}$ alkyl, $C_{1-6}$ alkoxy, amine, cyano, $C_{1-6}$ haloalkyl, or $C_{2-6}$ alkynyl that is unsubstituted or substituted with a hydroxyl group,

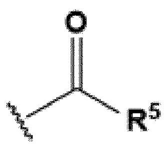, 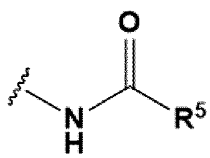,

or

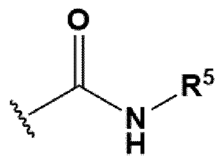

,

and m is an integer from 0 to 4,
$R^5$ is $C_{1-6}$ alkyl, $-C_{1-6}$ alkyl-$C_{1-6}$ alkoxy, $-C_{1-6}$ alkyl-hydroxy, or $C_{3-6}$ cycloalkyl, where the $C_{3-6}$ cycloalkyl of $R^5$ is unsubstituted or substituted with $C_{1-6}$ alkylamino or halogen, and
$R^3$ is hydrogen or $C_{1-6}$ alkyl.

3. The compound of Chemical Formula 1, the isomer thereof, the solvate thereof, the hydrate thereof, or the pharmaceutically acceptable salt thereof of claim 1, wherein ring $A^1$ is imidazo[1,2-b]pyridazine, benzo[d]oxazole, benzo[d][1,2,3]triazole, benzo[d]thiazole, indazole, thiazolo[5,4-b]pyridine, triazolo[1,5-a]pyridine, pyrrolo[2,1-f][1,2,4]triazine, triazolo[4,3-b]pyridazine, imidazo[1,2-a]pyridine, thieno[3,2-d]pyrimidine, pyrazolo[1,5-a]pyridine, thiazolo[4,5-c]pyridine, imidazo[4,5-c]pyridine, or imidazo[4,5-b]pyridine.

4. The compound of Chemical Formula 1, the isomer thereof, the solvate thereof, the hydrate thereof, or the pharmaceutically acceptable salt thereof of claim 1, wherein ring $A^2$ is isoxazolidine, morpholine, benzo[d]isoxazolidine, oxa-azaspiro[2.4]heptane, or oxazinane.

5. The compound of Chemical Formula 1, the isomer thereof, the solvate thereof, the hydrate thereof, or the pharmaceutically acceptable salt thereof of claim 1, wherein, when $R^2$ is

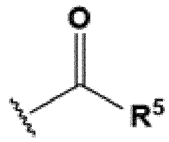

,

$R^5$ is $C_{3-8}$ cycloalkyl that is unsubstituted or substituted with halogen,

when $R^2$ is

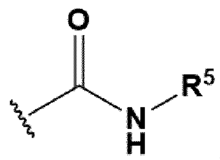

,

$R^5$ is $C_{1-8}$ alkyl or $C_{3-8}$ cycloalkyl that is unsubstituted or substituted with $C_{1-8}$ alkylamino, and
when $R^2$ is

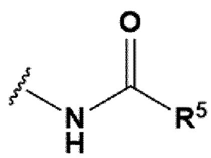

,

$R^5$ may be $C_{3-8}$ cycloalkyl that is unsubstituted or substituted with $C_{1-8}$ alkyl, $-C_{1-8}$ alkyl-$C_{1-8}$ alkoxy, $-C_{1-8}$ alkyl-hydroxy, or halogen.

6. The compound of Chemical Formula 1, the isomer thereof, the solvate thereof, the hydrate thereof, or the pharmaceutically acceptable salt thereof of claim 1, wherein the compound represented by Chemical Formula 1 is any one of compounds 1 to 158 below:

<1> (S)-N-(3-(2-acetamidoimidazo[1,2-b]pyridazin-6-yl)phenyl)-3-phenylisoxazolidine-2-carboxamide; <2> (S)-N-(3-(2-(cyclopropanecarboxamido)imidazo[1,2-b]pyridazin-6-yl)phenyl)-3-phenylisoxazolidine-2-carboxamide; <3> (S)-N-(5-(2-acetamidoimidazo[1,2-b]pyridazin-6-yl)-2-methoxypyridin-3-yl)-3-phenylisoxazolidine-2-carboxamide; <4> (S)-N-(5-(2-(cyclopropanecarboxamido)imidazo[1,2-b]pyridazin-6-yl)-2-methoxypyridin-3-yl)-3-phenylisoxazolidine-2-carboxamide; <5> (S)-N-(5-(2-(2-hydroxyacetamido)imidazo[1,2-b]pyridazin-6-yl)-2-methoxypyridin-3-yl)-3-phenylisoxazolidine-2-carboxamide; <6> (S)-N-(2-methoxy-5-(2-(2-methoxyacetamido)imidazo[1,2-b]pyridazin-6-yl)pyridin-3-yl)-3-phenylisoxazolidine-2-carboxamide; <7> (S)-N-(5-(2-aminoimidazo[1,2-b]pyridazin-6-yl)-2-methoxypyridin-3-yl)-3-phenylisoxazolidine-2-carboxamide; <8> (S)-N-(5-(2-acetamidoimidazo[1,2-b]pyridazin-6-yl)-2-methoxyphenyl)-3-phenylisoxazolidine-2-carboxamide; <9> (S)-N-(5-(2-aminoimidazo[1,2-b]pyridazin-6-yl)-2-methoxyphenyl)-3-phenylisoxazolidine-2-carboxamide;

<10> (S)-N-(5-(2-(cyclopropanecarboxamido)imidazo[1,2-b]pyridazin-6-yl)-2-methylpyridin-3-yl)-3-phenylisoxazolidine-2-carboxamide; <11> (S)-N-(5-(2-acetamidoimidazo[1,2-b]pyridazin-6-yl)-2-methylpyridin-3-yl)-3-phenylisoxazolidine-2-carboxamide; <12> (S)-N-(5-(1-isopropyl-1H-benzo[d][1,2,3]triazol-6-yl)-2-methoxypyridin-3-yl)-3-phenylisoxazolidine-2-carboxamide; <13> (S)-N-(5-(2-acetamidobenzo[d]oxazol-6-yl)-2-methoxypyridin-3-yl)-3-phenylisoxazolidine-2-carboxamide; <14> (S)-N-(5-(2-acetamidothiazolo[5,4-b]pyridin-5-yl)-2-methoxypyridin-3-yl)-3-phenylisoxazolidine-2-carboxamide; <15> (S)-N-(5-(1-isopropyl-1H-benzo[d][1,2,3]triazol-6-yl)-2-methoxyphenyl)-3-phenylisoxazolidine-2-carboxamide; <16> (S)-N-(5-(2-acetamidobenzo[d]oxazol-6-yl)-2-methoxyphenyl)-3-phenylisoxazolidine-2-carboxamide; <17> (S)-N-(3-(3-(methylcarbamoyl)-IH-indazol-6-yl)phenyl)-3-phenylisoxazolidine-2-carboxamide; <18> (S)-N-(6-methyl-5-(3-(methylcarbamoyl)-IH-indazol-6-yl)pyridin-3-yl)-3-phenylisoxazolidine-2-carboxamide;

<19> (S)-N-(5-(3-(methylcarbamoyl)-1H-indazol-6-yl)pyridin-3-yl)-3-phenylisoxazolidine-2-carboxamide; <20> (S)-N-(3-(3-((4-(dimethylamino)cyclohexyl)carbamoyl)-1H-indazol-6-yl)phenyl)-3-phenylisoxazolidine-2-carboxamide; <21> (S)-N-(2-methyl-5-(3-(methylcarbamoyl)-1H-indazol-6-yl)pyridin-3-yl)-3-phenylisoxazolidine-2-carboxamide;

<22> (S)-N-(2-methoxy-5-(3-(methylcarbamoyl)-1H-indazol-6-yl)pyridin-3-yl)-3-phenylisoxazolidine-2-carboxamide; <23> (S)-N-(6-methoxy-5-(3-(methylcarbamoyl)-1H-indazol-6-yl)pyridin-3-yl)-3-phenylisoxazolidine-2-carboxamide; <24> (S)-N-(2-methoxy-5-(3-(methylcarbamoyl)-1H-indazol-6-yl)phenyl)-3-phenylisoxazolidine-2-carboxamide; <25> (S)-N-(3-(2-(cyclopropanecarboxamido)benzo[d]thiazol-6-yl)phenyl)-3-phenylisoxazolidine-2-carboxamide; <26> (S)-N-(5-(2-acetamidobenzo[d]thiazol-6-yl)-2-methoxypyridin-3-yl)-3-phenylisoxazolidine-2-carboxamide; <27> (S)-N-(5-(2-acetamidobenzo[d]thiazol-6-yl)-2-methoxyphenyl)-3-phenylisoxazolidine-2-carboxamide; <28> (S)-N-(5-(2-acetamidobenzo[d]thiazol-6-yl)-2-methylpyridin-3-yl)-3-phenylisoxazolidine-2-carboxamide;

<29> (S)-N-(5-(2-(cyclopropanecarboxamido)benzo[d]thiazol-6-yl)-2-methylpyridin-3-yl)-3-phenylisoxazolidine-2-carboxamide; <30> (S)-N-(5-(2-amino-[1,2,4]triazolo[1,5-a]pyridin-6-yl)-2-methoxypyridin-3-yl)-3-phenylisoxazolidine-2-carboxamide; <31> (S)-N-(5-(2-amino-[1,2,4]triazolo[1,5-a]pyridin-6-yl)-2-methoxyphenyl)-3-phenylisoxazolidine-2-carboxamide;

<32> (S)-N-(5-(2-amino-[1,2,4]triazolo[1,5-a]pyridin-6-yl)-2-methylphenyl)-3 - phenylisoxazolidine-2-carboxamide; <33> (S)-N-(5-(2-amino-[1,2,4]triazolo[1,5-a]pyridin-6-yl)-2-methylpyridin-3-yl)-3-phenylisoxazolidine-2-carboxamide; <34> (S)-N-(5-(2-amino-[1,2,4]triazolo[1,5-a]pyridin-6-yl)-4-fluoro-2-methylphenyl)-3-phenylisoxazolidine-2-carboxamide; <35> (S)-N-(5-(2-acetamido-[1,2,4]triazolo[1,5-a]pyridin-6-yl)-2-methylpyridin-3-yl)-3-phenylisoxazolidine-2-carboxamide; <36> (S)-N-(5-(2-(cyclopropanecarboxamido)-[1,2,4]triazolo[1,5-a]pyridin-6-yl)-2-methylpyridin-3-yl)-3-phenylisoxazolidine-2-carboxamide; <37> (S)-N-(5-(2-amino-[1,2,4]triazolo[1,5-a]pyridin-6-yl)-2-methylpyridin-3-yl)-3-(3-fluorophenyl)isoxazolidine-2-carboxamide; <38> (S)-N-(5-(2-amino-[1,2,4]triazolo[1,5-a]pyridin-6-yl)-2-methylpyridin-3-yl)-3-(3,5-difluorophenyl)isoxazolidine-2-carboxamide; <39> (S)-N-(5-(2-amino-[1,2,4]triazolo[1,5-a]pyridin-6-yl)-2-methylpyridin-3-yl)-3-(3-chlorophenyl)isoxazolidine-2-carboxamide; <40> (S)-N-(5-(2-amino-[1,2,4]triazolo[1,5-a]pyridin-6-yl)-2-methylpyridin-3-yl)-3-(pyridin-3-yl)isoxazolidine-2-carboxamide; <41> (S)-N-(5-(2-amino-[1,2,4]triazolo[1,5-a]pyridin-6-yl)-2-methylpyridin-3-yl)-3-(3-(trifluoromethyl)phenyl)isoxazolidine-2-carboxamide; <42> (S)-N-(5-(2-amino-[1,2,4]triazolo[1,5-a]pyridin-6-yl)-2-methylpyridin-3-yl)-3-(3-chloro-4-fluorophenyl)isoxazolidine-2-carboxamide; <43> (S)-N-(5-(2-amino-[1,2,4]triazolo[1,5-a]pyridin-6-yl)-2-methylpyridin-3-yl)-3-(3-fluoro-5-(trifluoromethyl)phenyl)isoxazolidine-2-carboxamide; <44> (S)-N-(5-(2-amino-[1,2,4]triazolo[1,5-a]pyridin-6-yl)-2-methylpyridin-3-yl)-3-(4-chloro-3-(trifluoromethyl)phenyl)isoxazolidine-2-carboxamide; <45> (S)-N-(5-(2-amino-[1,2,4]triazolo[1,5-a]pyridin-6-yl)-2-methylpyridin-3-yl)-3-(3-methoxyphenyl)isoxazolidine-2-carboxamide; <46> (S)-N-(5-(2-amino-[1,2,4]triazolo[1,5-a]pyridin-6-yl)-2-methylpyridin-3-yl)-3-(3-cyanophenyl)isoxazolidine-2-carboxamide; <47> (S)-N-(5-(2-amino-[1,2,4]triazolo[1,5-a]pyridin-7-yl)-2-methoxypyridin-3-yl)-3-phenylisoxazolidine-2-carboxamide; <48> (S)-N-(5-(2-(cyclopropanecarboxamido)-[1,2,4]triazolo[1,5-a]pyridin-7-yl)-2-methylpyridin-3-yl)-3-phenylisoxazolidine-2-carboxamide; <49> (S)-N-(5-(2-acetamido-[1,2,4]triazolo[1,5-a]pyridin-7-yl)-2-methylpyridin-3-yl)-3-phenylisoxazolidine-2-carboxamide; <50> (S)-N-(5-(2-amino-[1,2,4]triazolo[1,5-a]pyridin-7-yl)-2-methylpyridin-3-yl)-3-phenylisoxazolidine-2-carboxamide; <51> (S)-N-(5-(2-amino-[1,2,4]triazolo[1,5-a]pyridin-6-yl)-2-methylpyridin-3-yl)-3-(6-methylpyridin-3-yl)isoxazolidine-2-carboxamide; <52> (S)-N-(5-(2-amino-[1,2,4]triazolo[1,5-a]pyridin-6-yl)-2-methylpyridin-3-yl)-3-(3-chioro-5-fluorophenyl)isoxazolidine-2-carboxamide; <53> (S)-N-(5-(2-amino-[1,2,4]triazolo[1,5-a]pyridin-7-yl)-2-methoxypyridin-3-yl)-3-(3-fluorophenyl)isoxazolidine-2-carboxamide;

<54> (S)-N-(5-(2-amino-[1,2,4]triazolo[1,5-a]pyridin-7-yl)-2-methoxypyridin-3-yl)-3-(3,5-difluorophenyl)isoxazolidine-2-carboxamide; <55> (S)-N-(5-(2-amino-[1,2,4]triazolo[1,5-a]pyridin-6-yl)-2-methylpyridin-3-yl)-3-(3,4-difluorophenyl)isoxazolidine-2-carboxamide;

<56> (S)-N-(5-(2-amino-[1,2,4]triazolo[1,5-a]pyridin-6-yl)-2-methylpyridin-3-yl)-3-(4-fluorophenyl)isoxazolidine-2-carboxamide; <57> (S)-N-(5-(2-amino-[1,2,4]triazolo[1,5-a]pyridin-6-yl)-2-methoxypyridin-3 -yl)-3 -(3, 5-difluorophenyl)isoxazolidine-2-carboxamide;

<58> (S)-N-(5-(2-amino-[1,2,4]triazolo[1,5-a]pyridin-6-yl)-2-methoxypyridin-3-yl)-3-(3-fluorophenyl)isoxazolidine-2-carboxamide; <59> (S)-N-(5-(2-amino-[1,2,4]triazolo[1,5-a]pyridin-6-yl)-2-methylphenyl)-3-(3,5-difluorophenyl)isoxazolidine-2-carboxamide; <60> (S)-N-(5-(2-amino-[1,2,4]triazolo[1,5-a]pyridin-6-yl)-2-methylphenyl)-3-(3-fluorophenyl)isoxazolidine-2-carboxamide; <61> (S)-N-(5-(2-amino-[1,2,4]triazolo[1,5-a]pyridin-7-yl)-2-methylphenyl)-3-phenylisoxazolidine-2-carboxamide; <62> (S)-N-(5-(2-amino-[1,2,4]triazolo[1,5-a]pyridin-7-yl)-2-methylphenyl)-3-(3-fluorophenyl)isoxazolidine-2-carboxamide; <63> (S)-N-(5-(2-amino-[1,2,4]triazolo[1,5-a]pyridin-7-yl)-2-methylphenyl)-3-(3,5-difluorophenyl)isoxazolidine-2-carboxamide; <64> (S)-N-(5-(2-amino-[1,2,4]triazolo[1,5-a]pyridin-7-yl)-2-methylphenyl)-3-(4-fluorophenyl)isoxazolidine-2-carboxamide; <65> (S)-N-(5-(2-amino-[1,2,4]triazolo[1,5-a]pyridin-7-yl)-2-methylphenyl)-3-(4-chlorophenyl)isoxazolidine-2-carboxamide; <66> (S)-N-(5-(2-amino-[1,2,4]triazolo[1,5-a]pyridin-6-yl)-2-methylphenyl)-3-(4-fluorophenyl)isoxazolidine-2-carboxamide; <67> (S)-N-(5-(2-amino-[1,2,4]triazolo[1,5-a]pyridin-6-yl)-2-methylphenyl)-3-(4-chlorophenyl)isoxazolidine-2-carboxamide; <68> (S)-N-(5-(2-amino-[1,2,4]triazolo[1,5-a]pyridin-6-yl)-2-methylpyridin-3-yl)-3-(4-cyanophenyl)isoxazolidine-2-carboxamide; <69> (S)-N-(5-(2-amino-[1,2,4]triazolo[1,5-a]pyridin-6-yl)-2-methylpyridin-3-yl)-3-(4-chlorophenyl)isoxazolidine-2-carboxamide; <70> (S)-N-(5-(2-amino-[1,2,4]triazolo[1,5-a]pyridin-7-yl)-2-methylpyridin-3-yl)-3-(4-cyanophenyl)isoxazolidine-2-carboxamide; <71> (S)-N-(5-(2-amino-[1,2,4]triazolo[1,5-a]pyridin-6-yl)-2-methylpyridin-3-yl)-3-(naphthalen-2-yl)isoxazolidine-2-carboxamide; <72> (S)-N-(5-(2-amino-[1,2,4]triazolo[1,5-a]pyridin-6-yl)-2-methylpyridin-3-yl)-3-(3-(dimethylamino)phenyl)isoxazolidine-2-carboxamide; <73> (S)-N-(5-(2-amino-[1,2,4]triazolo[1,5-a]pyridin-6-yl)-2-methylpyridin-3-yl)-3-(3-chloro-2-methylphenyl)isoxazolidine-2-carboxamide; <74> (S)-N-(5-(2-amino-[1,2,4]triazolo[1,5-a]pyridin-6-yl)-2-methylpyridin-3-yl)-3-(4-methoxyphenyl)isoxazolidine-2-carboxamide;

<75> (S)-N-(5-(2-amino-[1,2,4]triazolo[1,5-a]pyridin-6-yl)-2-methylpyridin-3-yl)-3-(4-(trifluoromethyl)thiazol-2-yl)isoxazolidine-2-carboxamide; <76> (S)-N-(5-(2-amino-[1,2,4]triazolo[1,5-a]pyridin-6-yl)-2-methylpyridin-3-yl)-3-(1-methyl-1H-pyrazol-4-yl)isoxazolidine-2-carboxamide; <77> (S)-N-(5-(2-amino-[1,2,4]triazolo[1,5-a]pyridin-6-yl)-2-methylpyridin-3-yl)-3-(furan-2-yl)isoxazolidine-2-carboxamide; <78> (S)-N-(5-(2-amino-[1,2,4]triazolo[1,5-a]pyridin-6-yl)-2-methylpyridin-3-yl)-3-(5-fluoropyridin-3-yl)isoxazolidine-2-carboxamide; <79> (R)-N-(5-(2-amino-[1,2,4]triazolo[1,5-a]pyridin-6-yl)-2-methylpyridin-3-yl)-3-(5-fluoropyridin-3-yl)isoxazolidine-2-carboxamide; <80> (S)-N-(5-(2-(cyclopropanecarboxamido)imidazo[1,2-b]pyridazin-6-yl)-2-methylphenyl)-3-phenylisoxazolidine-2-carboxamide; <81> (S)-N-(5-(2-acetamidoimidazo[1,2-b]pyridazin-6-yl)-2-methylphenyl)-3-phenylisoxazolidine-2-carboxamide; <82> (S)-N-(5-(2-(cyclopropanecarboxamido)imidazo[1,2-b]pyridazin-6-yl)-2-methylphenyl)-3-(4-fluorophenyl)isoxazolidine-2-carboxamide; <83> (S)-N-(5-(2-(cyclopropanecarboxamido)thiazolo[5,4-b]pyridin-5-yl)-2-methylphenyl)-3-(4-fluorophenyl)isoxazolidine-2-carboxamide;

<84> (S)-N-(5-(2-((1R,2R)-2-fluorocyclopropane-1-carboxamido)benzo[d]thiazol-6-yl)-2-methylphenyl)-3-phenylisoxazolidine-2-carboxamide; <85> (S)-N-(5-(2-((1S,2S)-2-fluorocyclopropane-1-carboxamido)benzo[d]thiazol-6-yl)-2-methylphenyl)-3-phenylisoxazolidine-2-carboxamide;

<86> (S)-N-(5-(2-((1R,2R)-2-fluorocyclopropane-1-carboxamido)imidazo[1,2-b]pyridazin-6-yl)-2-methylphenyl)-3-phenylisoxazolidine-2-carboxamide; <87> (S)-N-(5-(2-((1S,2S)-2-fluorocyclopropane-l-carboxamido)imidazo[1,2-b]pyridazin-6-yl)-2-methylphenyl)-3-phenylisoxazolidine-2-carboxamide; <88> (S)-N-(5-(2-((1S,2S)-2-fluorocyclopropane-1-carboxamido)thiazolo[5,4-b]pyridin-5-yl)-2-methylphenyl)-3-phenylisoxazolidine-2-carboxamide; <89> (S)-N-(5-(2-aminobenzo[d]thiazol-6-yl)-2-methylphenyl)-3-phenylisoxazolidine-2-carboxamide; <90> (S)-N-(5-(2-aminothiazolo[5,4-b]pyridin-5-yl)-2-methylphenyl)-3-(4-fluorophenyl)isoxazolidine-2-carboxamide; <91> (S)-N-(5-(2-amino-[1,2,4]triazolo[1,5-a]pyridin-6-yl)-2-methylphenyl)-3-(4-cyanophenyl)isoxazolidine-2-carboxamide; <92> (S)-N-(5-(2-amino-[1,2,4]triazolo[1,5-a]pyridin-7-yl)-2-methylphenyl)-3-(4-cyanophenyl)isoxazolidine-2-carboxamide; <93> (S)-3-(4-cyanophenyl)-N-(5-(2-(cyclopropanecarboxamido)benzo[d]thiazol-6-yl)-2-methylphenyl)isoxazolidine-2-carboxamide; <94> (S)-3-(4-cyanophenyl)-N-(5-(2-(cyclopropanecarboxamido)thiazolo[5,4-b]pyridin-5-yl)-2-methylphenyl)isoxazolidine-2-carboxamide; <95> (S)-N-(5-(2-aminoimidazo[1,2-b]pyridazin-6-yl)-2-methylphenyl)-3-phenylisoxazolidine-2-carboxamide;

<96> (S)-N-(5-(2-amino-[1,2,4]triazolo[1,5-a]pyridin-6-yl)-2-fluorophenyl)-3-phenylisoxazolidine-2-carboxamide; <97> (S)-N-(5-(2-amino-[1,2,4]triazolo[1,5-a]pyridin-6-yl)-2-chlorophenyl)-3-phenylisoxazolidine-2-carboxamide; <98> (S)-N-(5-(2-aminothiazolo[5,4-b]pyridin-5-yl)-2-methylphenyl)-3-phenylisoxazolidine-2-carboxamide;

<99> (S)-N-(5-(4-aminopyrrolo[2,1-f][1,2,4]triazin-7-yl)-2-methoxypyridin-3-yl)-3-phenylisoxazolidine-2-carboxamide; <100> (S)-N-(5-(4-aminopyrrolo[2,1-f][1,2,4]triazin-7-yl)-2-methylphenyl)-3-phenylisoxazolidine-2-carboxamide; <101> (S)-N-(5-([1,2,4]triazolo[1,5-a]pyridin-6-yl)-2-methylphenyl)-3-phenylisoxazolidine-2-carboxamide;

<102> (S)-N-(5-([1,2,4]triazolo[1,5-a]pyridin-7-yl)-2-methylphenyl)-3-phenylisoxazolidine-2-carboxamide; <103> (S)-N-(3-(2-amino-[1,2,4]triazolo[1,5-a]pyridin-7-yl)-2-fluoro-6-methoxyphenyl)-3-phenylisoxazolidine-2-carboxamide; <104> (S)-N-(3-(2-amino-[1,2,4]triazolo[1,5-a]pyridin-6-yl)-2-fluoro-6-methoxyphenyl)-3-phenylisoxazolidine-2-carboxamide; <105> (S)-N-(3-(2-amino-[1,2,4]triazolo[1,5-a]pyridin-6-yl)-6-chloro-2-fluorophenyl)-3-phenylisoxazolidine-2-carboxamide; <106> (S)-N-(5-([1,2,4]triazolo[4,3-b]pyridazin-6-yl)-2-methylphenyl)-3-phenylisoxazolidine-2-carboxamide; <107> (S)-N-(2-methyl-5-(3-methyl-[1,2,4]triazolo[4,3-b]pyridazin-6-yl)phenyl)-3-phenylisoxazolidine-2-carboxamide; <108> (S)-N-(2-methyl-5-(3-(trifluoromethyl)-[1,2,4]triazolo[4,3-b]pyridazin-6-yl)phenyl)-3-phenylisoxazolidine-2-carboxamide; <109> (S)-N-(5-(2-aminobenzo[d]oxazol-6-yl)-2-methylphenyl)-3-phenylisoxazolidine-2-carboxamide; <110> (S)-N-(5-(2-aminobenzo[d]oxazol-6-yl)-2-methoxypyridin-3-yl)-3-phenylisoxazolidine-2-carboxamide; <111> (S)-N-(5-(2-aminoimidazo[1,2-a]pyridin-6-yl)-2-methoxypyridin-3-yl)-3-phenylisoxazolidine-2-carboxamide; <112> (S)-N-(5-(imidazo[1,2-b]pyridazin-6-yl)-2-methylphenyl)-3-phenylisoxazolidine-2-carboxamide; <113> (S)-N-(5-(imidazo[1,2-a]pyridin-6-yl)-2-methylphenyl)-3-phenylisoxazolidine-2-carboxamide; <114> (S)-N-(5-(3-cyanoimidazo[1,2-a]pyridin-6-yl)-2-methylphenyl)-3-phenylisoxazolidine-2-carboxamide;

<115> (S)-N-(5-(3-(3-hydroxy-3-methylbut-1-yn-1-yl)imidazo[1,2-a]pyridin-6-yl)-2-methylphenyl)-3-phenylisoxazolidine-2-carboxamide; <116> (S)-N-(5-(3-ethynylimidazo[1,2-a]pyridin-6-yl)-2-methylphenyl)-3-phenylisoxazolidine-2-carboxamide;

<117> (S)-N-(5-(3-(3-hydroxy-3-methylbut-1-yn-1-yl)imidazo[1,2-b]pyridazin-6-yl)-2-methylphenyl)-3-phenylisoxazolidine-2-carboxamide; <118> (S)-N-(5-(3-cyanoimidazo[1,2-b]pyridazin-6-yl)-2-methylphenyl)-3-phenylisoxazolidine-2-carboxamide; <119> (S)-N-(3-(4-aminopyrrolo[2,1-f][1,2,4]triazin-7-yl)phenyl)-3-phenylisoxazolidine-2-carboxamide; <120> (S)-N-(5-(4-aminopyrrolo[2,1-f][1,2,4]triazin-7-yl)-2-methylpyridin-3-yl)-3-phenylisoxazolidine-2-carboxamide; <121> (S)-N-(5-(4-aminothieno[3,2-d]pyrimidin-7-yl)-2-methylpyridin-3-yl)-3-phenylisoxazolidine-2-carboxamide; <122> (S)-N-(5-(4-aminothieno[3,2-d]pyrimidin-7-yl)-2-methylphenyl)-3-phenylisoxazolidine-2-carboxamide;

<123> (S)-N-(5-(2-amino-[1,2,4]triazolo[1,5-a]pyridin-7-yl)-2-fluorophenyl)-3-phenylisoxazolidine-2-carboxamide; <124> (S)-N-(5-(2-amino-[1,2,4]triazolo[1,5-a]pyridin-7-yl)-2-chlorophenyl)-3-phenylisoxazolidine-2-carboxamide; <125> (S)-N-(5-(3-ethynylimidazo[1,2-b]pyridazin-6-yl)-2-methylphenyl)-3-phenylisoxazolidine-2-carboxamide;

<126> (S)-N-(5-(2-aminopyrazolo[1,5-a]pyridin-5-yl)-2-methylphenyl)-3-phenylisoxazolidine-2-carboxamide; <127> (S)-N-(3-(4-aminopyrrolo[2,1-f][1,2,4]triazin-7-yl)-4-methylphenyl)-3-phenylisoxazolidine-2-carboxamide; <128> (S)-N-(5-(4-aminothieno[3,2-d]pyrimidin-7-yl)-2-methoxypyridin-3-yl)-3-phenylisoxazolidine-2-carboxamide; <129> (S)-N-(5-(4-amino-5-(trifluoromethyl)pyrrolo[2,1-f][1,2,4]triazin-7-yl)-2-methoxypyridin-3-yl)-3-phenylisoxazolidine-2-carboxamide; <130> (S)-N-(2-methyl-5-(thiazolo[4,5-c]pyridin-6-yl)phenyl)-3-phenylisoxazolidine-2-carboxamide; <131> (S)-N-(2-methyl-5-(thiazolo[5,4-b]pyridin-5-yl)phenyl)-3-phenylisoxazolidine-2-carboxamide; <132> (S)-N-(3-(2-aminobenzo[d]thiazol-6-yl)-2,6-difluorophenyl)-3-phenylisoxazolidine-2-carboxamide; <133> (S)-N-(2-methyl-5-(1-methyl-1H-imidazo[4,5-c]pyridin-6-yl)phenyl)-3-phenylisoxazolidine-2-carboxamide; <134> (S)-N-(3-(2-amino-[1,2,4]triazolo[1,5-a]pyridin-7-yl)-2,6-difluorophenyl)-3-phenylisoxazolidine-2-carboxamide; <135> (S)-N-(5-(2-amino-3-((1S,2S)-2-fluorocyclopropane-1-carbonyl)imidazo[1,2-a]pyridin-6-yl)-2-methylphenyl)-3-phenylisoxazolidine-2-carboxamide; <136> (R)-N-(5-(2-aminopyrazolo[1,5-a]pyridin-5-yl)-2-methylphenyl)-3-phenylmorpholine-4-carboxamide; <137> (S)-N-(5-(2-aminopyrazolo[1,5-a]pyridin-5-yl)-2-methylphenyl)-2-phenylmorpholine-4-carboxamide; <138> (S)-N-(5-(2-aminopyrazolo[1,5-a]pyridin-5-yl)-2-methylphenyl)-3-phenylbenzo[d]isooxazole-2(3H)-carboxamide; <139> (R)-N-(5-(2-aminopyrazolo[1,5-a]pyridin-5-yl)-2-methylphenyl)-3-phenylbenzo[d]isoxazole-2(3H)-carboxamide; <140> (R)-N-(5-(2-aminopyrazolo[1,5-a]pyridin-5-yl)-2-methylphenyl)-7-phenyl-5-oxa-6-azaspiro[2.4]heptane-6-carboxamide;

<141> (S)-N-(5-(2-aminopyrazolo[1,5-a]pyridin-5-yl)-2-methylphenyl)-3-methyl-3-phenylisoxazolidine-2-carboxamide; <142> (R)-N-(5-(2-aminopyrazolo[1,5-a]pyridin-5-yl)-2-methylphenyl)-3-methyl-3-phenylisoxazolidine-2-carboxamide; <143> (S)-N-(2-methyl-5-(3-methyl-3H-imidazo[4,5-b]pyridin-5-yl)phenyl)-3-phenylisoxazolidine-2-carboxamide;

<144> (S)-N-(5-(2-amino-[1,2,4]triazolo[1,5-a]pyridin-6-yl)-2-cyanophenyl)-3-phenylisoxazolidine-2-carboxamide; <145> (S)-N-(5-(2-amino-[1,2,4]triazolo[1,5-a]pyridin-6-yl)-2-methylphenyl)-3-phenyl-1,2-oxazinane-2-carboxamide; <146> (S)-N-(5-(2-amino-[1,2,4]triazolo[1,5-a]pyridin-6-yl)-2-methylphenyl)-3-(4-fluorophenyl)-1,2-oxazinane-2-carboxamide; <147> (S)-N-(5-(2-amino-[1,2,4]triazolo[1,5-a]pyridin-6-yl)-2-methylphenyl)-3-(3,5-difluorophenyl)-1,2-oxazinane-2-carboxamide; <148> (S)-N-(5-(2-amino-[1,2,4]triazolo[1,5-a]pyridin-7-yl)-2-methylphenyl)-3-phenyl-1,2-oxazinane-2-carboxamide;
<149> (S)-N-(3-(2-amino-[1,2,4]triazolo[1,5-a]pyridin-6-yl)-2-fluoro-6-methylphenyl)-3-phenylisoxazolidine-2-carboxamide; <150> (S)-N-(3-(2-amino-[1,2,4]triazolo[1,5-a]pyridin-6-yl)-6-ethyl-2-fluorophenyl)-3-phenylisoxazolidine-2-carboxamide; <151> (S)-N-(3-(2-amino-[1,2,4]triazolo[1,5-a]pyridin-6-yl)-6-cyclopropyl-2-fluorophenyl)-3-phenylisoxazolidine-2-carboxamide; <152> (S)-N-(5-(2-amino-[1,2,4]triazolo[1,5-a]pyridin-6-yl)-2-ethylphenyl)-3-phenylisoxazolidine-2-carboxamide; <153> (S)-N-(5-(2-amino-[1,2,4]triazolo[1,5-a]pyridin-6-yl)-2-cyclopropylphenyl)-3-phenylisoxazolidine-2-carboxamide; <154> (S)-N-(3-(2-amino-[1,2,4]triazolo[1,5-a]pyridin-7-yl)-2-fluoro-6-methylphenyl)-3-phenylisoxazolidine-2-carboxamide; <155> (S)-N-(3-(2-amino-[1,2,4]triazolo[1,5-a]pyridin-7-yl)-6-ethyl-2-fluorophenyl)-3-phenylisoxazolidine-2-carboxamide; <156> (S)-N-(3-(2-amino-[1,2,4]triazolo[1,5-a]pyridin-7-yl)-6-cyclopropyl-2-fluorophenyl)-3-phenylisoxazolidine-2-carboxamide;
<157> (S)-N-(5-(2-amino-[1,2,4]triazolo[1,5-a]pyridin-7-yl)-2-ethylphenyl)-3-phenylisoxazolidine-2-carboxamide; and <158> (S)-N-(5-(2-amino-[1,2,4]triazolo[1,5-a]pyridin-7-yl)-2-cyclopropylphenyl)-3-phenylisoxazolidine-2-carboxamide.

**7.** A method of preparing a compound of Chemical Formula 1, comprising:

preparing a compound of Chemical Formula 3 from a compound of Chemical Formula 2 (Step 1); and
preparing a compound of Chemical Formula 1 from the compound of Chemical Formula 3 (Step 2),

[Chemical Formula 2]

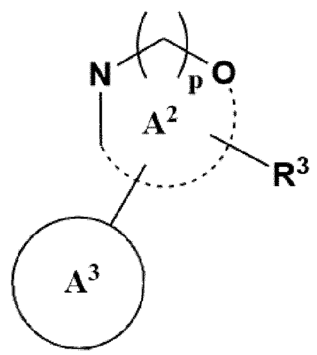

[Chemical Formula 3]

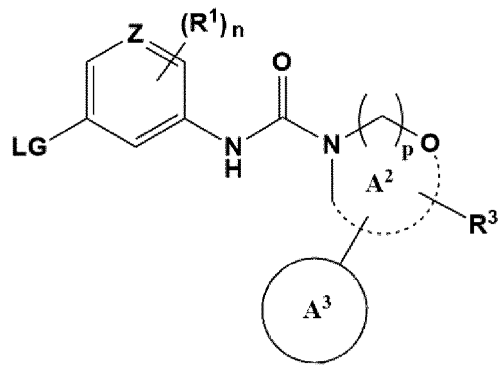

[Chemical Formula 1]

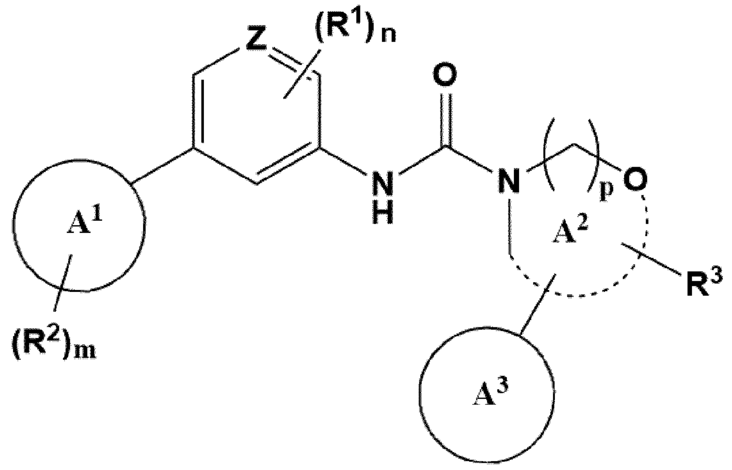

In the above formulas, LG is a leaving group, and Z, rings $A^1$ to $A^3$, $R^1$ to $R^3$, m, n, and p are the same as defined in claim 1.

**8.** A pharmaceutical composition comprising the compound of Chemical Formula 1 according to any one of claims 1 to 6, an isomer thereof, a solvate thereof, a hydrate thereof, or a pharmaceutically acceptable salt thereof as an active ingredient, for preventing or treating a disease selected from the group consisting of inflammatory bowel disease, Crohn's disease, ulcerative colitis, psoriasis, retinal detachment, retinitis pigmentosa, macular degeneration, pancreatitis, atopic dermatitis, rheumatoid arthritis, spondyloarthritis, gout, Sjogren's syndrome, systemic scleroderma, anti-phospholipid syndrome, vasculitis, osteoarthritis, non-alcoholic steatohepatitis, alcoholic steatohepatitis, primary sclerosing cholangitis, nephritis, celiac disease, transplant rejection, sepsis, systemic inflammatory response syndrome, myocardial infarction, Huntington's disease, Alzheimer's disease, Parkinson's disease, allergic disease, asthma, atopic dermatitis, multiple sclerosis, type I diabetes, Wegener's granulomatosis, pulmonary sarcoidosis, Behcet's disease, motor neurone disease, chronic obstructive pulmonary disease, and periodontitis.

**9.** The pharmaceutical composition of claim 8, which inhibits the expression or activity of RIPK1 kinase.

**10.** A method of preventing or treating an RIPK1 kinase-related disease, comprising:
administering the compound of Chemical Formula 1 according to any one of claims 1 to 6, an isomer thereof, a solvate thereof, a hydrate thereof, or a pharmaceutically acceptable salt thereof to a subject in need thereof at a pharmaceutically effective amount.

**11.** The method of claim 10, wherein the RIPK1 kinase-related disease is selected from the group consisting of inflammatory bowel disease, Crohn's disease, ulcerative colitis, psoriasis, retinal detachment, retinitis pigmentosa, macular degeneration, pancreatitis, atopic dermatitis, rheumatoid arthritis, spondyloarthritis, gout, Sjogren's syndrome, systemic scleroderma, anti-phospholipid syndrome, vasculitis, osteoarthritis, non-alcoholic steatohepatitis, alcoholic steatohepatitis, primary sclerosing cholangitis, nephritis, celiac disease, transplant rejection, sepsis, systemic inflammatory response syndrome, myocardial infarction, Huntington's disease, Alzheimer's disease, Parkinson's disease, allergic disease, asthma, atopic dermatitis, multiple sclerosis, type I diabetes, Wegener's granulomatosis, pulmonary sarcoidosis, Behcet's disease, motor neurone disease, chronic obstructive pulmonary disease, and periodontitis.

**12.** A use of the compound of Chemical Formula 1 according to any one of claims 1 to 6, an isomer thereof, a solvate thereof, a hydrate thereof, or a pharmaceutically acceptable salt thereof for preparing a medicament used in the prevention or treatment of an RIPK1 kinase-related disease.

**13.** The use of claim 12, wherein the RIPK1 kinase-related disease is selected from the group consisting of inflammatory bowel disease, Crohn's disease, ulcerative colitis, psoriasis, retinal detachment, retinitis pigmentosa, macular degeneration, pancreatitis, atopic dermatitis, rheumatoid arthritis, spondyloarthritis, gout, Sjogren's syndrome, systemic scleroderma, anti-phospholipid syndrome, vasculitis, osteoarthritis, non-alcoholic steatohepatitis, alcoholic steatohepatitis, primary sclerosing cholangitis, nephritis, celiac disease, transplant rejection, sepsis, systemic inflammatory response syndrome, myocardial infarction, Huntington's disease, Alzheimer's disease, Parkinson's disease, allergic disease, asthma, atopic dermatitis, multiple sclerosis, type I diabetes, Wegener's granulomatosis, pulmonary sarcoidosis, Behcet's disease, motor neurone disease, chronic obstructive pulmonary disease, and periodontitis.

## INTERNATIONAL SEARCH REPORT

International application No.
**PCT/KR2022/016188**

### A. CLASSIFICATION OF SUBJECT MATTER

**C07D 487/04**(2006.01)i; **A61K 31/5025**(2006.01)i; **A61K 31/4439**(2006.01)i; **A61K 31/422**(2006.01)i; **C07D 413/14**(2006.01)i; **C07D 513/04**(2006.01)i; **C07D 413/12**(2006.01)i; **C07D 417/12**(2006.01)i; **C07D 417/14**(2006.01)i; **C07D 471/04**(2006.01)i

According to International Patent Classification (IPC) or to both national classification and IPC

### B. FIELDS SEARCHED

Minimum documentation searched (classification system followed by classification symbols)

C07D 487/04(2006.01); A23L 33/10(2016.01); A61K 31/422(2006.01); A61K 31/4439(2006.01); C07D 401/04(2006.01); C07D 401/14(2006.01); C07D 403/12(2006.01); C07D 413/14(2006.01); C07D 495/04(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: 수용체-상호작용 단백질-1 키나아제(receptor-interacting serine/threonine-protein kinase 1, RIPK1), 억제제(inhibitor), 아이소옥사졸리딘-카르복사아마이드(isoxazoline-carboxamide)

### C. DOCUMENTS CONSIDERED TO BE RELEVANT

| Category* | Citation of document, with indication, where appropriate, of the relevant passages | Relevant to claim No. |
|---|---|---|
| X | US 2014-0228367 A1 (FLYNN, G. A.) 14 August 2014 (2014-08-14) See claims 1-3, 6, 7, 11, 12, 24, 25 and 29. | 1-6,8,9,12,13 |
| Y | | 7 |
| Y | KR 10-2020-0089219 A (VORONOI INC. et al.) 24 July 2020 (2020-07-24) See paragraphs [0303]-[0313]. | 7 |
| X | US 2018-0353480 A1 (DENALI THERAPEUTICS INC.) 13 December 2018 (2018-12-13) See claims 1, 2, 4 and 28-30; and paragraph [0388], compound 4. | 1-6,8,9,12,13 |
| A | WO 2018-183122 A1 (SIDECAR THERAPEUTICS, INC.) 04 October 2018 (2018-10-04) See entire document. | 1-9,12,13 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 February 2023** | **13 February 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

INTERNATIONAL SEARCH REPORT

International application No.
**PCT/KR2022/016188**

**C. DOCUMENTS CONSIDERED TO BE RELEVANT**

| Category* | Citation of document, with indication, where appropriate, of the relevant passages | Relevant to claim No. |
|---|---|---|
| A | WO 2014-125444 A1 (GLAXOSMITHKLINE INTELLECTUAL PROPERTY DEVELOPMENT LIMITED) 21 August 2014 (2014-08-21) See entire document. | 1-9,12,13 |

Form PCT/ISA/210 (second sheet) (July 2022)

**INTERNATIONAL SEARCH REPORT**

International application No.
**PCT/KR2022/016188**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **10,11**
because they relate to subject matter not required to be searched by this Authority, namely:

Claims 10 and 11 pertain to a method for treatment of the human body by surgery or therapy (PCT Article 17(2)(a)(i) and PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/KR2022/016188**

| Patent document cited in search report | Publication date (day/month/year) | Patent family member(s) | Publication date (day/month/year) |
|---|---|---|---|
| US 2014-0228367 A1 | 14 August 2014 | EP 2739143 A1 | 11 June 2014 |
| | | EP 2739143 A4 | 07 January 2015 |
| | | EP 2739143 B1 | 11 July 2018 |
| | | US 2016-0303128 A1 | 20 October 2016 |
| | | US 2018-0125848 A1 | 10 May 2018 |
| | | US 9221805 B2 | 29 December 2015 |
| | | US 9833455 B2 | 05 December 2017 |
| | | WO 2013-022766 A1 | 14 February 2013 |
| KR 10-2020-0089219 A | 24 July 2020 | KR 10-2020-0006495 A | 20 January 2020 |
| | | US 2021-0284647 A1 | 16 September 2021 |
| | | WO 2020-013531 A1 | 16 January 2020 |
| | | WO 2020-149553 A1 | 23 July 2020 |
| US 2018-0353480 A1 | 13 December 2018 | CN 108602809 A | 28 September 2018 |
| | | CN 108602809 B | 30 September 2022 |
| | | US 10709692 B2 | 14 July 2020 |
| | | WO 2017-096301 A1 | 08 June 2017 |
| WO 2018-183122 A1 | 04 October 2018 | EP 3601250 A1 | 05 February 2020 |
| | | EP 3601250 A4 | 25 November 2020 |
| | | JP 2020-512976 A | 30 April 2020 |
| | | US 11345676 B2 | 31 May 2022 |
| | | US 2020-0048218 A1 | 13 February 2020 |
| WO 2014-125444 A1 | 21 August 2014 | AR 094773 A1 | 26 August 2015 |
| | | AR 118165 A2 | 22 September 2021 |
| | | AU 2014-217453 A1 | 13 August 2015 |
| | | AU 2014-217453 B2 | 14 April 2016 |
| | | BR 112015019627 A2 | 18 July 2017 |
| | | BR 122017002946 A2 | 10 September 2019 |
| | | BR 122017002949 A2 | 10 September 2019 |
| | | BR 122017002951 A2 | 28 January 2020 |
| | | CA 2900695 A1 | 21 August 2014 |
| | | CA 2900695 C | 12 October 2021 |
| | | CL 2015002279 A1 | 08 July 2016 |
| | | CN 105121432 A | 02 December 2015 |
| | | CN 105121432 B | 03 August 2018 |
| | | CN 106928203 A | 07 July 2017 |
| | | CN 106928203 B | 07 February 2020 |
| | | CN 108774214 A | 09 November 2018 |
| | | CN 108774214 B | 09 March 2021 |
| | | CR 20150420 A | 19 October 2015 |
| | | CR 20190511 A | 08 January 2020 |
| | | CR 20190512 A | 08 January 2020 |
| | | CY 1120320 T1 | 10 July 2019 |
| | | DK 2956452 T3 | 06 June 2018 |
| | | DK 3342771 T3 | 08 February 2021 |
| | | DO P2015000196 A | 15 May 2016 |
| | | DO P2018000088 A | 31 October 2018 |
| | | DO P2018000089 A | 30 June 2018 |
| | | EA 028991 B1 | 31 January 2018 |
| | | EA 031971 B1 | 29 March 2019 |
| | | EA 201591506 A1 | 29 January 2016 |

Form PCT/ISA/210 (patent family annex) (July 2022)

**INTERNATIONAL SEARCH REPORT**
**Information on patent family members**

International application No.
**PCT/KR2022/016188**

| Patent document cited in search report | Publication date (day/month/year) | Patent family member(s) | Publication date (day/month/year) |
|---|---|---|---|
| | | EA 201792039 A1 | 31 January 2018 |
| | | EP 2956452 A1 | 23 December 2015 |
| | | EP 2956452 B1 | 21 March 2018 |
| | | EP 3342771 A1 | 04 July 2018 |
| | | EP 3342771 B1 | 25 November 2020 |
| | | EP 3375780 A1 | 19 September 2018 |
| | | EP 3375780 B1 | 03 June 2020 |
| | | EP 3508484 A1 | 10 July 2019 |
| | | EP 3508484 B1 | 07 April 2021 |
| | | ES 2672530 T3 | 14 June 2018 |
| | | ES 2814556 T3 | 29 March 2021 |
| | | ES 2855135 T3 | 23 September 2021 |
| | | HK 1213545 A1 | 08 July 2016 |
| | | HK 1249100 A1 | 26 October 2018 |
| | | HK 1253099 A1 | 06 June 2019 |
| | | HR P20180900 T1 | 13 July 2018 |
| | | HR P20210252 T1 | 02 April 2021 |
| | | HU E037763 T2 | 28 September 2018 |
| | | HU E053215 T2 | 28 June 2021 |
| | | IL 240280 A | 24 September 2015 |
| | | IL 240280 B | 30 April 2018 |
| | | IL 256500 A | 28 February 2018 |
| | | IL 256500 B | 29 November 2018 |
| | | IL 256502 A | 28 February 2018 |
| | | IL 256502 B | 29 November 2018 |
| | | JP 2016-512488 A | 28 April 2016 |
| | | JP 2018-111712 A | 19 July 2018 |
| | | JP 2018-150372 A | 27 September 2018 |
| | | JP 6321045 B2 | 09 May 2018 |
| | | JP 6352571 B1 | 04 July 2018 |
| | | KR 10-2015-0119174 A | 23 October 2015 |
| | | KR 10-2019-0018558 A | 22 February 2019 |
| | | KR 10-2228764 B1 | 18 March 2021 |
| | | KR 10-2267977 B1 | 21 June 2021 |
| | | LT 2956452 T | 11 June 2018 |
| | | LT 3342771 T | 10 March 2021 |
| | | MA 38326 A1 | 30 November 2017 |
| | | MA 38326 B1 | 30 April 2018 |
| | | MA 44820 A1 | 30 September 2019 |
| | | MA 44820 B1 | 30 June 2020 |
| | | MX 2015010572 A | 16 November 2015 |
| | | MX 360715 B | 13 November 2018 |
| | | MY 178552 A | 16 October 2020 |
| | | NZ 628447 A | 26 August 2016 |
| | | PE 20151752 A1 | 25 November 2015 |
| | | PH 12015501675 A1 | 19 October 2015 |
| | | PH 12018500341 A1 | 10 September 2018 |
| | | PH 12018500342 A1 | 10 September 2018 |
| | | PH 12018500343 A1 | 10 September 2018 |
| | | PL 2956452 T3 | 31 August 2018 |

Form PCT/ISA/210 (patent family annex) (July 2022)

**INTERNATIONAL SEARCH REPORT**
**Information on patent family members**

International application No.
**PCT/KR2022/016188**

| Patent document cited in search report | Publication date (day/month/year) | Patent family member(s) | Publication date (day/month/year) |
|---|---|---|---|
| | | PL 3342771 T3 | 14 June 2021 |
| | | PT 2956452 T | 01 June 2018 |
| | | PT 3342771 T | 11 February 2021 |
| | | RS 57332 B1 | 31 August 2018 |
| | | RS 61439 B1 | 31 March 2021 |
| | | SG 11201505796 A | 28 August 2015 |
| | | SI 2956452 T1 | 29 June 2018 |
| | | SI 3342771 T1 | 30 April 2021 |
| | | TR 201808112 T4 | 23 July 2018 |
| | | TW 201443042 A | 16 November 2014 |
| | | TW 201819368 A | 01 June 2018 |
| | | TW 201819369 A | 01 June 2018 |
| | | TW 201819370 A | 01 June 2018 |
| | | TW I637951 B | 11 October 2018 |
| | | TW I638815 B | 21 October 2018 |
| | | TW I648273 B | 21 January 2019 |
| | | TW I648274 B | 21 January 2019 |
| | | UA 118259 C2 | 26 December 2018 |
| | | US 10292987 B2 | 21 May 2019 |
| | | US 10933070 B2 | 02 March 2021 |
| | | US 10940154 B2 | 09 March 2021 |
| | | US 2015-0353533 A1 | 10 December 2015 |
| | | US 2017-0008878 A1 | 12 January 2017 |
| | | US 2017-0183332 A1 | 29 June 2017 |
| | | US 2018-0098998 A1 | 12 April 2018 |
| | | US 2019-0201413 A1 | 04 July 2019 |
| | | US 2019-0262356 A1 | 29 August 2019 |
| | | US 9556152 B2 | 31 January 2017 |
| | | US 9624202 B2 | 18 April 2017 |
| | | UY 35330 A | 29 August 2014 |
| | | ZA 201505267 B | 30 August 2017 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **THOMAS A. HAMILTON.** Encyclopedia of Immunology. 1998, 2028-2033
- **HOLLER et al.** *Nat Immunol,* 2000, vol. 1, 489-495
- **DEGTEREV et al.** *Nat Chem Biol,* 2008, vol. 4, 313-321
- *Nature,* 2011, vol. 477, 330-334
- *Immunity,* 2011, vol. 35, 572-582
- *PNAS,* 2010, vol. 107, 21695-21700
- *Proc. Natl. Acad. Sci.,* 2012, vol. 109 (36), 14598-14603
- *Cell,* 2009, vol. 137, 1100-1111
- *Immunity,* 2011, vol. 35, 908-918
- *PNAS,* 10 October 2017, vol. 114 (41), E8788-E8797
- *Cell Death and Disease,* 2012, vol. 3, e320
- *Cancer Cell,* 12 November 2018, vol. 34, 757-774